# EUROPEAN PATENT APPLICATION

(11) **EP 4 635 975 A1**
(43) Date of publication of application: **22.10.2025**
(21) Application number: 24171459.1
(22) Date of filing: 19.04.2024
(51) Int. Cl.: C07K 14/245, C12N 1/08, C12N 15/70, C12N 1/28, C12R 1/19

(54) **MODIFIED BACTERIAL STRAINS**

(71) Applicant: Institute of Science and Technology Austria, 3400 Klosterneuburg (AT)
(72) Inventor: GUET, Calin, Klosterneuburg (AT); RÖMHILD, Roderich, Klosterneuburg (AT)
(74) Representative: Script Intellectual Property LLP

(57) **Abstract**

Described herein is a modified bacterial strain lacking an insertion sequence (IS), wherein the modified bacterial strain (i) includes a specific deletion of an IS present in an unmodified bacterial strain, and (ii) retains non-IS sequences flanking the IS in the unmodified bacterial strain. There is also provided a method for producing said modified bacterial strains, the method comprising: (a) providing a bacterial strain comprising an IS flanked by non-IS sequences; and (b) specifically deleting the IS in the bacterial strain without deleting non-IS sequences flanking the IS.

## Description

### FIELD OF THE INVENTION

The invention relates to modified bacterial strains. In particular, the present invention relates to modified bacterial strains lacking an insertion sequence (IS), including modified bacterial strains lacking all IS. In said strains, non-IS sequences flanking the IS have not been deleted, such that more than 98% of the bacterial genome sequence is retained. Also provided are methods for producing said bacterial strains.

### BACKGROUND OF THE INVENTION

Bacterial strains are used extensively in biotechnology to produce a variety of commercial products. Indeed, bacteria are particularly useful for the production of polypeptides and polynucleotides, for example, for use in the pharmaceutical and food industries. For example, bacteria may be used to produce therapeutic DNA for use in gene therapy, DNA vaccines and RNA interference, and may be used to produce various enzymes and proteins. However, a substantial challenge in employing bacteria for biotechnological processes lies in the stability of the bacterial genome. Genetic alterations within bacterial genomes can profoundly influence bacterial phenotype, thereby affecting process efficiency, yield, and the reliability of experimental outcomes.

One significant contributor to genetic instability in bacteria is the presence of small mobile genetic elements known as insertion sequences (IS, Siguier et al., 2015, Everyman's guide to bacterial insertion sequences. Microbiology Spectrum, 3(2): MDNA3-0030-2014). IS elements, typically measuring between 700 bp and 2500bp, possess the capability to translocate and duplicate within the bacterial genome (Siguier et al., 2015). Consequently, IS elements have the potential to disrupt the function of coding sequences, leading to a myriad of phenotypic changes, such as changes to bacterial growth characteristics, metabolic activity, and loss of desired traits. Moreover, the presence of multiple IS elements in a bacterial genome can promote homologous recombination (Siguier et al., 2015), resulting in genomic rearrangements which impact the phenotype of the bacteria being used.

Another concern of IS elements is their ability to transfer to plasmids, which are commonly used as expression vectors in bacterial hosts for the production of polynucleotides and polypeptides (Siguier et al., 2015). The transposition of IS elements to plasmids introduces genetic variability in the system, thereby compromising the consistency of the production process. Variations in product quality or yield may not only affect the efficiency of the production process but may also pose challenges in meeting regulatory standards.

To overcome this issue, EP2543726 describes *E. coli* lacking IS. However, the strains described in EP2543726 are reduced genome bacteria, which have significant portions of non-IS genome sequence deleted. In addition, it was later found that these strains did not successfully delete all IS (as described in WO 2007/118162). WO 2007/118162 describes insertion sequence-free bacteria. However, the IS-free bacterial strains of WO 2007/118162 also had large deletions of non-IS genomic sequence (around 14 to 15% of the genome deleted). Even in strains lacking only some IS in EP2543726 and WO 2007/118162, deletion of IS either left residual IS (and associated TSD where present), resulted in insertion of additional sequence, and/or deleted non-IS sequences flanking the IS.

### SUMMARY OF THE INVENTION

The present invention addresses the problems discussed above. In particular, according to one aspect of the invention, there is provided a modified bacterial strain lacking an insertion sequence (IS), wherein the modified bacterial strain (i) includes a specific deletion of an IS present in an unmodified bacterial strain, and (ii) retains non-IS sequences flanking the IS in the unmodified bacterial strain. Complete deletion of an IS is associated with the benefit of not leaving residual IS sequence in the modified bacterial genome, for example, where residual IS sequence may interfere with the coding sequence. In addition, deletion of only the IS sequence without deletion of genomic sequences flanking the IS means that the original genome sequence, i.e., before the IS was inserted, is restored. Therefore, in embodiments where the IS is located in a coding sequence in the unmodified bacterial strain, deletion of the IS in the modified bacterial strain results in restored expression of a native coding sequence. Therefore, in such embodiments, normal function of one or more genes is restored, i.e., as compared to the unmodified bacterial strain.

In embodiments, the bacterial strain is an *Escherichia coli* (*E. coli*) strain. In embodiments, the *E*. *coli* is strain B, BL21, REL606, C, Nissle 1917, W, K-12 MG1655, K-12 EMG2, K-12 WG1, K-12 W2637, K-12 W3110, K-12 DH5alpha, or HMS173, or a hybrid or derivative strain thereof, preferably wherein the *E. coli* is strain MG1655. These strains are commonly used in biotechnological processes, and therefore have the benefit of being possible to swap the bacterial strain of the current invention into existing processes. In embodiments, the bacterial strain is a synthetic bacterial strain. Synthetic bacterial strains have the benefit of avoiding or reducing unintended spontaneous mutations and/or transposition of IS compared to removal of IS in naturally occurring bacterial strains.

In embodiments, the modified bacterial strain includes specific deletions of at least 2, 3, 5, 10, 15, 20, 30, 40 or 45 IS present in the unmodified bacterial strain, preferably wherein the modified bacterial strain includes a specific deletion of all IS present in the unmodified bacterial strain, more preferably wherein the modified bacterial strain includes a specific deletion of all IS present in the unmodified bacterial strain and retains a genome size greater than 98% as compared to the genome size of the unmodified bacterial strain. Deletion of more IS elements improves genetic stability, with bacterial strains lacking all IS elements being the most genetically stable. In embodiments, IS families or subfamilies that have the highest insertion or recombination activity are deleted. In strains lacking all IS elements, transposition of IS within the bacterial genome is eliminated. In addition, in strains lacking all IS elements, mutation rates are reduced. In addition, deletion of all IS elements avoids transposition of any IS into plasmids of interest. In addition, deletion of all IS elements according to the present invention resulted in improved biomass, growth rate and bacterial yield compared to the unmodified strain, and similar protein yields compared to the unmodified strain. In addition, strains lacking all IS according to the present invention showed greatly improved biomass, protein yields, growth rate and competition rate compared to the MDS42 strain of EP2543726 and WO 2007/118162. Having a genome size greater than 98% compared to the unmodified bacterial strain has the benefit that most, or all of, the non-IS genome sequence is retained in the modified bacterial strain. In comparison, MDS42 has a genome size of only 85.7%. This means that the present modified bacterial strain has the benefit of retaining all polynucleotides or polypeptides of the native genome. In particular, the metabolic activity of the modified bacterial strain is retained or improved compared to the unmodified bacterial strain.

In some embodiments, the modified bacterial strain includes a specific deletion of all nucleotide residues between and including two inverted repeats each defining an end of the IS. This allows complete deletion of the IS.

However, in some embodiments, the modified bacterial strain includes a specific deletion of only IS nucleotide residues between and including two inverted repeats (IR) each defining an end of the IS. In embodiments, the modified bacterial strain includes a specific deletion of only IS nucleotide residues between and including two inverted repeats each defining an end of the IS, without deletion of non-IS nucleotide residues located between the two inverted repeats. In embodiments, the modified bacterial strain includes a specific deletion of IS nucleotide residues between and including two inverted repeats each defining an end of the IS, without deletion of non-IS coding sequence. In embodiments, the IS nucleotide residues do not include non-IS nucleotide residues (i.e., do not include non-IS bacterial genome sequence). Where an IS overlaps with bacterial genome sequence, deletion of the complete IS (i.e., all nucleotides between and including the two IR) would also result in deletions of non-IS bacterial genome sequence. In these examples, where the IS is located in a coding sequence, deletion of the complete IS could impact the resulting mRNA and protein, and therefore impact the phenotype of the bacterial strain.

In embodiments, the modified bacterial strain further includes a specific deletion of a target site duplication sequence (direct repeat) present in the unmodified bacterial strain adjacent to the IS. Insertion of an IS into a bacterial genome can result in duplication of part of the bacterial genome, known as target site duplication (also referred to as direct repeats (DR). These TSDs are positioned immediately adjacent to the IR flanking the IS. Therefore, in some embodiments where the deleted IS is associated with a TSD, the TSD is also deleted. This has the benefit that no IS-associated sequence is retained in the modified bacterial strain. This has the benefit of restoring the genomic sequence. In embodiments where the IS and TSD are located in a coding sequence in the unmodified bacterial strain, deletion of the IS and TSD in the modified bacterial strain results in restored expression of a native coding sequence. Therefore, in such embodiments, normal function of one or more genes is restored.

In embodiments, the modified bacterial strain retains at least 3, 10, 20, 30, 50, 100 or 500 non-IS nucleotide residues that immediately flank either end of the IS in the unmodified bacterial strain, preferably wherein the modified bacterial strain retains all non-IS sequences present in the unmodified bacterial strain. In some embodiments, the retained nucleotide residues (i.e., the nucleotide residues that flank the deleted IS) have at least 90, 91, 92, 93, 94, 95, 96, 97, 98, or 99%, or 100%, sequence identity to the nucleotide residues that immediately flank either end of the IS in the unmodified bacterial strain. Where most nucleotide residues flanking the IS are retained, the original genome sequence is maintained. Where all nucleotide residues flanking the IS are retained, the original genome sequence, i.e., before the IS was inserted, is restored. Therefore, in embodiments where the IS is located in a coding sequence in the unmodified bacterial strain, deletion of the IS in the modified bacterial strain results in restored expression of a native coding sequence. Therefore, in such embodiments, normal function of one or more genes is restored, i.e., as compared to the unmodified bacterial strain.

In embodiments, the modified bacterial strain lacks additional exogenous sequence elements compared to the unmodified bacterial strain. Therefore, in embodiments, deletion of the IS does not result in any additional alterations to the genome sequence in the modified bacterial strain (e.g., no further changes except removal of the IS). Therefore, in embodiments, deletion of the IS does not impact expression of the coding sequence of the bacterial genome. As such, deletion of the IS may restore function of one or more genes.

In embodiments, the specific deletion in the modified bacterial strain restores expression of a native coding sequence interrupted by the IS in the unmodified bacterial strain.

In embodiments, the modified bacterial strain comprises a stop codon and/or a frame-shift mutation between the retained non-IS flanking sequences. This has the benefit of preventing gene or pseudogene expression in the modified bacterial strain.

In embodiments, the stop codon and/or frame-shift mutation is in a *wbbL* gene, preferably wherein the stop codon and/or a frame-shift mutation is in a *wbbL* gene at a position corresponding to an IS present in the unmodified bacterial strain, more preferably wherein the stop codon and/or frameshift mutation is in a *wbbL* gene at a position corresponding to IS5I present in the unmodified bacterial strain. Replacement of an IS in the *wbbL* gene, particularly IS5I in MG1655 bacteria, with a stop codon and/or frame-shift mutation has the benefit of retaining phage susceptibility so that routine bacterial transduction techniques can continue to be used on the bacteria, i.e., to make further modifications of interest to the bacterial genome.

In embodiments, the IS is a member of one of the following families or subfamilies: insL-2, IS1, IS2, IS3, IS4, IS5, IS6, IS629, IS50, IS21, IS200, IS256, IS621, IS630, IS257, IS15, IS26, IS10, IS30, IS150, IS186, IS600, IS609, IS911, ISX and/or ISZ, and/or a homologue thereof. In embodiments, the IS has at least 95% identity to: IS1A (SEQ ID NO: 2), IS1B (SEQ ID NO: 3), IS1C (SEQ ID NO:4), IS1D (SEQ ID NOs: 5 or 47), IS1E (SEQ ID NOs: 6 or 48), IS1F (SEQ ID NO: 7), IS1H (SEQ ID NOs: 8 or 49), IS5A (SEQ ID NO: 9), IS5B (SEQ ID NOs: 10 or 50), IS5D (SEQ ID NOs: 11 or 51), IS5F (SEQ ID NOs: 12 or 52), IS5H (SEQ ID NOs: 13 or 53), IS5I (SEQ ID NOs: 14 or 54), IS5K (SEQ ID NOs: 15 or 55), IS5LO (SEQ ID NO: 16), IS5R (SEQ ID NOs: 17 or 56), IS5T (SEQ ID NOs: 18 or 57), IS5Y (SEQ ID NOs: 19 or 58), IS4 (SEQ ID NOs: 20 or 62), IS2A (SEQ ID NOs: 21 or 65), IS2D (SEQ ID NOs: 22 or 67), IS2E (SEQ ID NO: 23), IS2F (SEQ ID NOs: 24 or 69), IS2H (SEQ ID NO: 25), IS2I (SEQ ID NOs: 26 or 70), IS2K (SEQ ID NOs: 27 or 71), IS3A (SEQ ID NO: 28), IS3B (SEQ ID NOs: 29 or 66), IS3C (SEQ ID NO: 30), IS3D (SEQ ID NO: 31), IS3E (SEQ ID NOs: 32 or 68), IS30A (SEQ ID NO: 33), IS30B (SEQ ID NO: 34), IS30C (SEQ ID NOs: 35 or 63), IS30D (SEQ ID NO: 36), IS150 (SEQ ID NO: 37), IS186A (SEQ ID NOs: 38 or 59), IS186B (SEQ ID NOs: 39 or 60), IS186C (SEQ ID NOs: 40 or61), IS600 (SEQ ID NO: 41), IS609 (SEQ ID NOs: 42 or64), IS911A (SEQ ID NO: 43), IS911B (SEQ ID NO: 44), ISX (SEQ ID NO: 45), ISZ (SEQ ID NO: 46), IS629 (SEQ ID NO: 161), IS10 (SEQ ID NO: 162), IS50 (SEQ ID NO: 163), IS257 (SEQ ID NO: 164), IS15 (SEQ ID NO: 165), IS26 (SEQ ID NO: 166), IS21 (SEQ ID NO: 202), IS200 (SEQ ID NO: 203), IS256 (SEQ ID NO: 204), IS621 (SEQ ID NO: 205), IS630 (SEQ ID NO: 206), and/or insL-2 (SEQ ID NOs: 1 or 218), and/or a homologue thereof. These IS families/subfamilies may be found in bacterial strains such as *E. coli .*

In embodiments, the modified bacterial strain lacks IS comprising or consisting of:
(a) IS1A (SEQ ID NO: 2), IS1B (SEQ ID NO: 3), IS1C (SEQ ID NO:4), IS1D (SEQ ID NOs: 5 or 47), IS1E (SEQ ID NOs: 6 or 48), IS1F (SEQ ID NO: 7), and IS1H (SEQ ID NOs: 8 or 49), or homologues thereof;
(b) IS1A (SEQ ID NO: 2), IS1B (SEQ ID NO: 3), IS1C (SEQ ID NO:4), IS1D (SEQ ID NOs: 5 or 47), IS1E (SEQ ID NOs: 6 or 48), IS1F (SEQ ID NO: 7), IS1H (SEQ ID NOs: 8 or 49), IS5A (SEQ ID NO: 9), IS5B (SEQ ID NOs: 10 or 50), IS5D (SEQ ID NOs: 11 or 51), IS5F (SEQ ID NOs: 12 or 52), IS5H (SEQ ID NOs: 13 or 53), IS5I (SEQ ID NOs: 14 or 54), IS5K (SEQ ID NOs: 15 or 55), IS5LO (SEQ ID NO: 16), IS5R (SEQ ID NOs: 17 or 56), IS5T (SEQ ID NOs: 18 or 57), and IS5Y (SEQ ID NOs: 19 or 58), or homologues thereof;
(c) IS1A (SEQ ID NO: 2), IS1B (SEQ ID NO: 3), IS1C (SEQ ID NO:4), IS1D (SEQ ID NOs: 5 or 47), IS1E (SEQ ID NOs: 6 or 48), IS1F (SEQ ID NO: 7), IS1H (SEQ ID NOs: 8 or 49), IS5A (SEQ ID NO: 9), IS5B (SEQ ID NOs: 10 or 50), IS5D (SEQ ID NOs: 11 or 51), IS5F (SEQ ID NOs: 12 or 52), IS5H (SEQ ID NOs: 13 or 53), IS5I (SEQ ID NOs: 14 or 54), IS5K (SEQ ID NOs: 15 or 55), IS5LO (SEQ ID NO: 16), IS5R (SEQ ID NOs: 17 or 56), IS5T (SEQ ID NOs: 18 or 57), IS5Y (SEQ ID NOs: 19 or 58); IS4 (SEQ ID NOs: 20 or 62), IS186A (SEQ ID NOs: 38 or 59), IS186B (SEQ ID NOs: 39 or 60), and IS186C (SEQ ID NOs: 40 or 61), or homologues thereof;
(d) IS1A (SEQ ID NO: 2), IS1B (SEQ ID NO: 3), IS1C (SEQ ID NO:4), IS1D (SEQ ID NOs: 5 or 47), IS1E (SEQ ID NOs: 6 or 48), IS1F (SEQ ID NO: 7), IS1H (SEQ ID NOs: 8 or 49), IS5A (SEQ ID NO: 9), IS5B (SEQ ID NOs: 10 or 50), IS5D (SEQ ID NOs: 11 or 51), IS5F (SEQ ID NOs: 12 or 52), IS5H (SEQ ID NOs: 13 or 53), IS5I (SEQ ID NOs: 14 or 54), IS5K (SEQ ID NOs: 15 or 55), IS5LO (SEQ ID NO: 16), IS5R (SEQ ID NOs: 17 or 56), IS5T (SEQ ID NOs: 18 or 57), IS5Y (SEQ ID NOs: 19 or 58); IS4 (SEQ ID NOs: 20 or 62), IS30A (SEQ ID NO: 33), IS30B (SEQ ID NO: 34), IS30C (SEQ ID NOs: 35 or 63), IS30D (SEQ ID NO: 36), IS150 (SEQ ID NO: 37), IS186A (SEQ ID NOs: 38 or 59), IS186B (SEQ ID NOs: 39 or 60), IS186C (SEQ ID NOs: 40 or 61); IS600 (SEQ ID NO: 41), IS609 (SEQ ID NOs: 42 or 64), IS911A (SEQ ID NO: 43), IS911B (SEQ ID NO: 44), ISX (SEQ ID NO: 45), and ISZ (SEQ ID NO: 46), or homologues thereof;
(e) IS1A (SEQ ID NO: 2), IS1B (SEQ ID NO: 3), IS1C (SEQ ID NO:4), IS1D (SEQ ID NOs: 5 or 47), IS1E (SEQ ID NOs: 6 or 48), IS1F (SEQ ID NO: 7), IS1H (SEQ ID NOs: 8 or 49), IS5A (SEQ ID NO: 9), IS5B (SEQ ID NOs: 10 or 50), IS5D (SEQ ID NOs: 11 or 51), IS5F (SEQ ID NOs: 12 or 52), IS5H (SEQ ID NOs: 13 or 53), IS5I (SEQ ID NOs: 14 or 54), IS5K (SEQ ID NOs: 15) or 55, IS5LO (SEQ ID NO: 16), IS5R (SEQ ID NOs: 17 or 56), IS5T (SEQ ID NOs: 18 or 57), IS5Y (SEQ ID NOs: 19 or 58); IS4 (SEQ ID NOs: 20 or 62), IS2A (SEQ ID NOs: 21 or 65), IS2D (SEQ ID NOs: 22 or 67), IS2E (SEQ ID NO: 23), IS2F (SEQ ID NOs: 24 or 69), IS2H (SEQ ID NO: 25), IS2I (SEQ ID NOs: 26 or 70), IS2K (SEQ ID NOs: 27 or 71), IS30A (SEQ ID NO: 33), IS30B (SEQ ID NO: 34), IS30C (SEQ ID NOs: 35 or 63), IS30D (SEQ ID NO: 36), IS150 (SEQ ID NO: 37), IS186A (SEQ ID NOs: 38 or 59), IS186B (SEQ ID NOs: 39 or 60), IS186C (SEQ ID NOs: 40 or 61); IS600 (SEQ ID NO: 41), IS609 (SEQ ID NOs: 42 or 64), IS911A (SEQ ID NO: 43), IS911B (SEQ ID NO: 44), ISX (SEQ ID NO: 45), and ISZ (SEQ ID NO: 46), or homologues thereof;
(f) IS5I (SEQ ID NOs: 14 or 54), or a homologue thereof;
(g) IS1A (SEQ ID NO: 2), IS1B (SEQ ID NO: 3), IS1C (SEQ ID NO:4), IS1D (SEQ ID NOs: 5 or 47), IS1E (SEQ ID NOs: 6 or 48), IS1F (SEQ ID NO: 7), IS1H (SEQ ID NOs: 8 or 49), IS5A (SEQ ID NO: 9), IS5B (SEQ ID NOs: 10 or 50), IS5D (SEQ ID NOs: 11 or 51), IS5F (SEQ ID NOs: 12 or 52), IS5H (SEQ ID NOs: 13 or 53), IS5I (SEQ ID NOs: 14 or 54), IS5K (SEQ ID NOs: 15 or 55), IS5LO (SEQ ID NO: 16), IS5R (SEQ ID NOs: 17 or 56), IS5T (SEQ ID NOs: 18 or 57), IS5Y (SEQ ID NOs: 19 or 58), IS4 (SEQ ID NOs: 20 or 62), IS2A (SEQ ID NOs: 21 or 65), IS2D (SEQ ID NOs: 22 or 67), IS2E (SEQ ID NO: 23), IS2F (SEQ ID NOs: 24 or 69), IS2H (SEQ ID NO: 25), IS2I (SEQ ID NOs: 26 or 70), IS2K (SEQ ID NOs: 27 or 71), IS3A (SEQ ID NO: 28), IS3B (SEQ ID NOs: 29 or 66), IS3C (SEQ ID NO: 30), IS3D (SEQ ID NO: 31), IS3E (SEQ ID NOs: 32 or 68), IS30A (SEQ ID NO: 33), IS30B (SEQ ID NO: 34), IS30C (SEQ ID NOs: 35 or 63), IS30D (SEQ ID NO: 36), IS150 (SEQ ID NO: 37), IS186A (SEQ ID NOs: 38 or 59), IS186B (SEQ ID NOs: 39 or 60), IS186C (SEQ ID NOs: 40 or 61), IS600 (SEQ ID NO: 41), IS609 (SEQ ID NOs: 42 or 64), IS911A (SEQ ID NO: 43), IS911B (SEQ ID NO: 44), ISX (SEQ ID NO: 45), and/or ISZ (SEQ ID NO: 46), or homologues thereof; or
(h) insL-2 (SEQ ID NOs: 1 or 218) or a homologue thereof.

In embodiments, the bacterial strain further comprises a modification that prevents or reduces expression or activity of one or more protease-encoding polynucleotides or polypeptides, respectively, preferably wherein the protease is Lon or OmpT, more preferably wherein a *Ion* or *ompT* encoding polynucleotide is deleted. Reduced expression or activity of a polynucleotide or polypeptide encoding Lon protease increased the mutation rate in the bacterial strain, unless IS were absent. Therefore, reduced expression or activity of a polynucleotide or polypeptide encoding Lon protease is particularly beneficial where one or more IS elements remain in the modified bacterial strain, as Lon and IS interact to increase mutation rates in bacteria. Reduced expression or activity of one or more polynucleotides or polypeptides encoding a protease has the benefit of improving protein production in the modified bacterial strain compared to the unmodified bacterial strain.

In embodiments, the IS is present in the unmodified bacterial strain in a *wbbL* gene, preferably IS5I. Deletion of an IS in the *wbbL* gene, particularly IS5I in MG1655 bacterial strains, confers phage-resistance to the bacteria. Phages can destroy bacteria and contaminate biotechnological processes that rely on bacteria, thereby impacting the quality and yield of desired products.

In embodiments, the bacterial strain further comprises a modification in one or more of the following genes: *Ion, ompT, hsdSB, hsdRMS, dcm, trxB, gor, recA, lacR, endA, luxS, acrAB,* or *acrR.* Modifications in one or more of these genes can further alter the phenotype of the modified bacterial strain.

In embodiments, there is provided a BL21 *E. coli* lacking *insL-2.* This allows provision of a BL21 strain with reduced mutation rates.

Another aspect of the invention relates to a method for producing a modified bacterial strain lacking an insertion sequence (IS) as described herein, the method comprising: (a) providing a bacterial strain comprising an IS flanked by non-IS sequences; and (b) specifically deleting the IS in the bacterial strain without deleting non-IS sequences flanking the IS. Specific deletion of an IS is associated with the benefit of not leaving residual IS sequence in the modified bacterial strain (b) genome, for example, where residual IS sequence may interfere with the coding sequence. In addition, deletion of only the IS sequence without deletion of genomic sequences flanking the IS means that the original genome sequence, i.e., before the IS was inserted, is restored. Therefore, in embodiments where the IS is located in a coding sequence in the unmodified bacterial strain (a), deletion of the IS in the modified bacterial strain (b) results in restored expression of a native coding sequence. Therefore, in such embodiments, normal function of one or more genes is restored, i.e., as compared to the bacterial strain (a)

In embodiments, bacterial strain (a) is an unmodified bacterial strain and bacterial strain (b) is a modified bacterial strain.

In embodiments, the non-IS sequences flanking the IS comprise or consist of least 3, 10, 20, 30, 50, 100 or 500 non-IS nucleotide residues that immediately flank either end of the IS in the bacterial strain (a).

In embodiments, (b) is repeated to delete more than one IS, preferably wherein (b) is repeated to delete at least 2, 3, 5, 10, 15, 20, 30, 40 or 45 IS present in the bacterial strain (a), more preferably wherein (b) is repeated to delete all IS present in the bacterial strain (a). Deletion of more IS elements improves genetic stability, with bacterial strains lacking all IS elements being the most genetically stable. In embodiments, IS families or subfamilies that have the highest insertion or recombination activity are deleted. In strains lacking all IS elements, transposition of IS within the bacterial genome is eliminated. In addition, in strains lacking all IS elements, mutation rates are reduced. In addition, deletion of all IS elements avoids transposition of any IS into plasmids of interest. In addition, deletion of all IS elements according to the present invention resulted in improved biomass, growth rate and bacterial yield compared to unmodified bacterial strain (a), and similar protein yields compared to unmodified bacterial strain (a). In addition, strains lacking all IS according to the present invention showed greatly improved biomass, protein yields, growth rate and competition rate compared to the MDS42 strain of EP2543726 and WO 2007/118162. Having a genome size greater than 98% compared to unmodified bacterial strain (a) has the benefit that most, or all of, the non-IS genome sequence is retained in the modified bacterial strain (b). In particular, the metabolic activity of the modified bacterial strain is retained or improved compared to the unmodified bacterial strain.

In embodiments, (b) further includes a specific deletion of a target site duplication sequence adjacent to the IS. Insertion of an IS into a bacterial genome can result in duplication of part of the bacterial genome, known as target site duplication (also referred to as direct repeats (DR)). These TSDs are positioned immediately adjacent to the IR flanking the IS. Therefore, in some embodiments where the deleted IS is associated with a TSD, the TSD is also deleted. This has the benefit that no IS-associated sequence is retained in the modified bacterial strain. This has the benefit of restoring the genomic sequence i.e., as though the IS had never been inserted. In embodiments where the IS and associated TSD are located in a coding sequence in the unmodified bacterial strain, deletion of the IS and associated TSD in the modified bacterial strain results in restored expression of a native coding sequence. Therefore, in such embodiments, normal function of one or more genes is restored.

In embodiments, the deletion of the IS in (b) comprises transforming the bacterial strain with a first and second insertion cassette, wherein the first insertion cassette comprises:
i. A polynucleotide sequence having at least 25 nucleotide residues (H1-a), wherein the polynucleotide sequence is identical to the flanking non-IS sequences 5' to the IS in the bacterial strain;
ii. A polynucleotide sequence having 5-35 nucleotide residues (H2-b), wherein the polynucleotide sequence is identical to the flanking non-IS sequences 3' to the IS in the bacterial strain; and
iii. A truncated polynucleotide sequence encoding part of a selection marker; and
the second insertion cassette comprises:
iv. A truncated polynucleotide sequence encoding a part of the same selection marker (iii);
v. A polynucleotide sequence having 5-35 nucleotide residues (H1-b), wherein the second nucleotide sequence is identical to the flanking non-IS sequences positioned 5' to the IS in the bacterial strain;
vi. A polynucleotide sequence having at least 25 nucleotide residues (H2-a), wherein the polynucleotide sequence is identical to the flanking non-IS sequences 3' to the IS in the bacterial strain; and

wherein the truncated polynucleotide sequences (iii) and (vi) each comprise at least 50 identical consecutive nucleotide residues, and
wherein the truncated polynucleotide sequences (iii) and (vi) are configured such that, when the first and second insertion cassette undergo recombination, the selection marker is expressed. This allows specific (and complete) deletion of the IS (and associated TSD in some embodiments), without deleting non-IS sequences flanking the IS.

In embodiments, three recombination events occur between: the polynucleotide sequence (i) and the flanking non-IS sequence 5' to the IS in the bacterial strain; the polynucleotide sequence (vi) and the flanking non-IS sequence 3' to the IS in the bacterial strain; and the truncated polynucleotide sequence encoding the selection gene (iii) and (iv); such that an intermediate bacterial strain is generated where the first and second insertion cassettes replace the IS. This allows deletion of the IS, but exogenous sequence (in the form of the insertion cassette) is still present in the bacterial genome.

In embodiments, a further, single, recombination event occurs between: (a) a polynucleotide comprising or consisting of (i) and (ii), and (b) a polynucleotide comprising or consisting of (v) and (vi); such that the nucleotide residues of the first and second expression cassette are deleted from the bacterial strain. In other words, there is a single recombination event between joined polynucleotides (i) and (ii) with joined polynucleotides (v) and (vi). This allows specific (and complete) deletion of the IS (and associated TSD in some embodiments), without deleting non-IS sequences flanking the IS.

In embodiments, the recombination event is homologous recombination, preferably spontaneous homologous recombination, or catalysed by lambda Red. Catalysing recombination improves the efficiency and speed of recombination events. This can be beneficial where there are multiple recombination events required to produce the desired effect, as the probability of each and every recombination event happening is lower.

In embodiments, the selection gene confers antibiotic resistance, preferably wherein the selection gene confers resistance to kanamycin or chloramphenicol. The selection gene may be altered depending on the user's preference.

In embodiments, the first and/or second cassette are generated by polymerase chain reaction (PCR), wherein the PCR uses an oligomer having 95% sequence identity to any of SEQ ID NOs: 75-156 or 171-200. These oligomers were used in the present examples.

In embodiments, the specific deletion of the IS in the bacterial strain (b) does not add any additional exogenous sequence elements compared to the unmodified bacterial strain. Therefore, in embodiments, deletion of the IS does not result in any additional alterations to the genome sequence in the modified bacterial strain (e.g., no further changes except removal of the IS). As such, the native genome sequence is restored. In embodiments, deletion of the IS does not impact expression of the coding sequence of the bacterial genome. in embodiments, deletion of the IS may restore function of one or more genes.

In embodiments, the first and/or second cassette further comprise a stop codon and/or frame-shift mutation, such that the deleted IS is replaced by a stop codon and/or frame-shift mutation, preferably wherein the first and/or second cassette further comprise a polynucleotide sequence encoding two stop codons and a frame shift mutation, more preferably wherein the first and/or second cassette further comprise SEQ ID NOs: 72 (CCTATTA) or 73 (TAATAGG). This has the benefit of preventing gene or pseudogene expression in the modified bacterial strain.

In embodiments, the specific deletion of the IS in the modified bacterial strain restores expression of a native coding sequence interrupted by the IS in the unmodified bacterial strain (a).

In embodiments, one or more IS are specifically deleted such that expression of a native coding sequence interrupted by the IS in the unmodified bacterial strain (a) is restored, and one or more IS are replaced by a stop codon and/or frame-shift mutation. In this way, different embodiments can be combined in a single bacterial strain such that one or more IS may be fully deleted, and a different one or more IS may be replaced by a stop codon and/or frameshift mutation.

In embodiments, the stop codon and/or frame-shift mutation replaces an IS positioned in a *wbbL* gene, preferably wherein the IS is IS5I (SEQ ID NO: 14) or a homologue thereof. Replacement of an IS in the *wbbL* gene, particularly IS5I in MG1655 bacteria, with a stop codon and/or frame-shift mutation has the benefit of retaining phage susceptibility so that routine bacterial transduction techniques can continue to be used on the bacteria, i.e., to make further modifications of interest to the bacterial genome.

In embodiments, the method further comprises modifying one or more genes of interest in the bacterial strain. Such modifications may include reverting any spontaneous mutations that arose during the method described herein or during normal culture or increasing or decreasing one or more further genes of interest.

In embodiments, the method further comprises deleting the stop codon and/or frame-shift mutation. This allows the user to prevent expression of a gene or pseudogene for a period of time before removing the stop codon and/or frameshift mutation to allow expression of said gene or pseudogene when desired. This may be particularly beneficial where expression of a gene or pseudogene is of experimental interest, but where said expression has detrimental effects on metabolic pathways, i.e., where expression of the gene or pseudogene renders the modified bacterial strain difficult to culture.

In embodiments, the method further comprises: (c) modifying one or more genes of interest in the bacterial strain; and (d) deleting the stop codon and/or frame-shift mutation. This is particularly beneficial where deletion of an IS restores a coding sequence that negatively impacts the ability to further culture or modify the bacterial strain, but where deletion of the IS is ultimately desired. For example, where deletion of IS5I in MG1655 strain confers phage resistance, this prevents the use of routine bacterial transduction techniques. The order of IS deletions and gene modifications can be altered. However, in this embodiment, a template bacterial strain can be produced where one or more IS are replaced by a stop codon and/or frameshift mutation, such that a gene of interest can still be modified in the template bacterial strain, and then the stop codon and/or frameshift mutation then deleted.

In embodiments, deletion of the stop codon and/or frame-shift mutation (d) uses the method described herein. This is beneficial in that the deletion of the stop codon and/or frameshift mutation does not result in deletion of non-IS sequences flanking the stop codon and/or frameshift mutation.

In addition, the complete stop codon and/or frameshift sequence can be deleted without leaving any residual additional exogenous sequence. Therefore, in embodiments, deletion of the IS does not result in any additional alterations to the genome sequence in the modified bacterial strain (e.g., no further changes except removal of the IS). Therefore, in embodiments the native genome sequence is restored. In embodiments, deletion of the IS does not impact expression of the coding sequence of the bacterial genome. In embodiments, deletion of the IS may restore function of one or more genes.

In embodiments, the bacterial strain is an *Escherichia coli* (*E. coli*) strain. In embodiments, the *E*. *coli* is strain B, BL21, REL606, C, Nissle 1917, W, K-12 MG1655, K-12 EMG2, K-12 WG1, K-12 W2637, K-12 W3110, K-12 DH5alpha, or HMS173, or a hybrid or derivative strain thereof, preferably wherein the *E. coli* is strain MG1655. These strains are commonly used in biotechnological processes, and therefore have the benefit of being possible to swap the bacterial strain of the current invention into existing processes. In embodiments, the bacterial strain is a synthetic bacterial strain. Synthetic bacterial strains have the benefit of avoiding or reducing unintended spontaneous mutations and/or transposition of IS compared to removal of IS in naturally occurring bacterial strains.

In some embodiments, the IS comprises, consists or consists essentially of all nucleotide residues between and including two inverted repeats each defining an end of the IS.

However, in some embodiments, the IS may overlap with bacterial genome sequence. In this case, deletion of all nucleotides between and including the two IR (the complete IS) would result in deletions of non-IS bacterial genome sequence. Where the IS is located in a coding sequence, deletion of the complete IS can impact the resulting mRNA and protein, and therefore impact the phenotype of the bacterial strain. Therefore, in some embodiments the IS comprises, consists or consists essentially of IS nucleotide residues between and including two inverted repeats each defining an end of the IS, where the IS nucleotide residues do not overlap with bacterial genome sequence. In embodiments, IS nucleotide residues between and including two inverted repeats each defining an end of the IS are deleted without deletion of non-IS nucleotide residues located between the two inverted repeats. In embodiments, IS nucleotide residues between and including two inverted repeats each defining an end of the IS are deleted without deletion of non-IS coding sequence.

In embodiments, the IS is a member of one of the following IS families or subfamilies: insL-2, IS1, IS2, IS3, IS4, IS5, IS6, IS629, IS50, IS21, IS200, IS256, IS621, IS630, IS257, IS15, IS26, IS10, IS30, IS150, IS186, IS600, IS609, IS911, ISX and/or ISZ, and/or a homologue thereof. In embodiments, the IS has at least 95% identity to: IS1A (SEQ ID NO: 2), IS1B (SEQ ID NO: 3), IS1C (SEQ ID NO:4), IS1D (SEQ ID NOs: 5 or 47), IS1E (SEQ ID NOs: 6 or 48), IS1F (SEQ ID NO: 7), IS1H (SEQ ID NOs: 8 or 49), IS5A (SEQ ID NO: 9), IS5B (SEQ ID NOs: 10 or 50), IS5D (SEQ ID NOs: 11 or 51), IS5F (SEQ ID NOs: 12 or 52), IS5H (SEQ ID NOs: 13 or 53), IS5I (SEQ ID NOs: 14 or 54), IS5K (SEQ ID NOs: 15 or 55), IS5LO (SEQ ID NO: 16), IS5R (SEQ ID NOs: 17 or 56), IS5T (SEQ ID NOs: 18 or 57), IS5Y (SEQ ID NOs: 19 or 58), IS4 (SEQ ID NOs: 20 or 62), IS2A (SEQ ID NOs: 21 or 65), IS2D (SEQ ID NOs: 22 or 67), IS2E (SEQ ID NO: 23), IS2F (SEQ ID NOs: 24 or 69), IS2H (SEQ ID NO: 25), IS2I (SEQ ID NOs: 26 or 70), IS2K (SEQ ID NOs: 27 or 71), IS3A (SEQ ID NO: 28), IS3B (SEQ ID NOs: 29 or 66), IS3C (SEQ ID NO: 30), IS3D (SEQ ID NO: 31), IS3E (SEQ ID NOs: 32 or 68), IS30A (SEQ ID NO: 33), IS30B (SEQ ID NO: 34), IS30C (SEQ ID NOs: 35 or 63), IS30D (SEQ ID NO: 36), IS150 (SEQ ID NO: 37), IS186A (SEQ ID NOs: 38 or 59), IS186B (SEQ ID NOs: 39 or 60), IS186C (SEQ ID NOs: 40 or 61), IS600 (SEQ ID NO: 41), IS609 (SEQ ID NOs: 42 or64), IS911A (SEQ ID NO: 43), IS911B (SEQ ID NO: 44), ISX (SEQ ID NO: 45), ISZ (SEQ ID NO: 46), IS629 (SEQ ID NO: 161), IS10 (SEQ ID NO: 162), IS50 (SEQ ID NO: 163), IS257 (SEQ ID NO: 164), IS15 (SEQ ID NO: 165), IS26 (SEQ ID NO: 166), IS21 (SEQ ID NO: 202), IS200 (SEQ ID NO: 203), IS256 (SEQ ID NO: 204), IS621 (SEQ ID NO: 205), IS630 (SEQ ID NO: 206), and/or insL-2 (SEQ ID NOs: 1 or 218), and/or a homologue thereof. These IS families/subfamilies may be found in bacterial strains such as *E. coli.*

In embodiments, the method further comprises modifying the bacterial strain further to prevent or reduce expression or activity of a protease-encoding polynucleotide or polypeptide, respectively, preferably wherein the protease is Lon or OmpT. In embodiments, a *Ion* or *ompT* encoding polynucleotide is deleted. Reduced expression or activity of a polynucleotide or polypeptide encoding Lon protease increased the mutation rate in the modified bacterial strain, unless IS were absent. Therefore, reduced expression or activity of a polynucleotide or polypeptide encoding Lon protease is particularly beneficial where one or more IS elements remain in the modified bacterial strain, as Lon and IS interact to increase mutation rates in bacteria. Reduced expression or activity of a polynucleotide or polypeptide encoding a protease has the benefit of improving protein production in the bacterial strain (b) compared to the bacterial strain (a).

In embodiments, the IS is in a *wbbL* gene, preferably IS5I. Deletion of an IS in the *wbbL* gene, particularly IS5I in MG1655 bacterial strains, confers phage-resistance to the bacteria. Phages can destroy bacteria and contaminate biotechnological processes that rely on bacteria, thereby impacting the quality and yield of desired products.

In embodiments, the IS to be deleted comprises or consists of:
(a) IS1A (SEQ ID NO: 2), IS1B (SEQ ID NO: 3), IS1C (SEQ ID NO:4), IS1D (SEQ ID NOs: 5 or 47), IS1E (SEQ ID NOs: 6 or 48), IS1F (SEQ ID NO: 7), and IS1H (SEQ ID NOs: 8 or 49), or homologues thereof;
(b) IS1A (SEQ ID NO: 2), IS1B (SEQ ID NO: 3), IS1C (SEQ ID NO:4), IS1D (SEQ ID NOs: 5 or 47), IS1E (SEQ ID NOs: 6 or 48), IS1F (SEQ ID NO: 7), IS1H (SEQ ID NOs: 8 or 49), IS5A (SEQ ID NO: 9), IS5B (SEQ ID NOs: 10 or 50), IS5D (SEQ ID NOs: 11 or 51), IS5F (SEQ ID NOs: 12 or 52), IS5H (SEQ ID NOs: 13 or 53), IS5I (SEQ ID NOs: 14 or 54), IS5K (SEQ ID NOs: 15 or 55), IS5LO (SEQ ID NO: 16), IS5R (SEQ ID NOs: 17 or 56), IS5T (SEQ ID NOs: 18 or 57), and IS5Y (SEQ ID NOs: 19 or 58), or homologues thereof;
(c) IS1A (SEQ ID NO: 2), IS1B (SEQ ID NO: 3), IS1C (SEQ ID NO:4), IS1D (SEQ ID NOs: 5 or 47), IS1E (SEQ ID NOs: 6 or 48), IS1F (SEQ ID NO: 7), IS1H (SEQ ID NOs: 8 or 49), IS5A (SEQ ID NO: 9), IS5B (SEQ ID NOs: 10 or 50), IS5D (SEQ ID NOs: 11 or 51), IS5F (SEQ ID NOs: 12 or 52), IS5H (SEQ ID NOs: 13 or 53), IS5I (SEQ ID NOs: 14 or 54), IS5K (SEQ ID NOs: 15 or 55), IS5LO (SEQ ID NO: 16), IS5R (SEQ ID NOs: 17 or 56), IS5T (SEQ ID NOs: 18 or 57), IS5Y (SEQ ID NOs: 19 or 58); IS4 (SEQ ID NOs: 20 or 62), IS186A (SEQ ID NOs: 38 or 59), IS186B (SEQ ID NOs: 39 or 60), and IS186C (SEQ ID NOs: 40 or 61), or homologues thereof;
(d) IS1A (SEQ ID NO: 2), IS1B (SEQ ID NO: 3), IS1C (SEQ ID NO:4), IS1D (SEQ ID NOs: 5 or 47), IS1E (SEQ ID NOs: 6 or 48), IS1F (SEQ ID NO: 7), IS1H (SEQ ID NOs: 8 or 49), IS5A (SEQ ID NO: 9), IS5B (SEQ ID NOs: 10 or 50), IS5D (SEQ ID NOs: 11 or 51), IS5F (SEQ ID NOs: 12 or 52), IS5H (SEQ ID NOs: 13 or 53), IS5I (SEQ ID NOs: 14 or 54), IS5K (SEQ ID NOs: 15 or 55), IS5LO (SEQ ID NO: 16), IS5R (SEQ ID NOs: 17 or 56), IS5T (SEQ ID NOs: 18 or 57), IS5Y (SEQ ID NOs: 19 or 58); IS4 (SEQ ID NOs: 20 or 62), IS30A (SEQ ID NO: 33), IS30B (SEQ ID NO: 34), IS30C (SEQ ID NOs: 35 or 63), IS30D (SEQ ID NO: 36), IS150 (SEQ ID NO: 37), IS186A (SEQ ID NOs: 38 or 59), IS186B (SEQ ID NOs: 39 or 60), IS186C (SEQ ID NOs: 40 or 61); IS600 (SEQ ID NO: 41), IS609 (SEQ ID NOs: 42 or 64), IS911A (SEQ ID NO: 43), IS911B (SEQ ID NO: 44), ISX (SEQ ID NO: 45), and ISZ (SEQ ID NO: 46), or homologues thereof;
(e) IS1A (SEQ ID NO: 2), IS1B (SEQ ID NO: 3), IS1C (SEQ ID NO:4), IS1D (SEQ ID NOs: 5 or 47), IS1E (SEQ ID NOs: 6 or 48), IS1F (SEQ ID NO: 7), IS1H (SEQ ID NOs: 8 or 49), IS5A (SEQ ID NO: 9), IS5B (SEQ ID NOs: 10 or 50), IS5D (SEQ ID NOs: 11 or 51), IS5F (SEQ ID NOs: 12 or 52), IS5H (SEQ ID NOs: 13 or 53), IS5I (SEQ ID NOs: 14 or 54), IS5K (SEQ ID NOs: 15) or 55, IS5LO (SEQ ID NO: 16), IS5R (SEQ ID NOs: 17 or 56), IS5T (SEQ ID NOs: 18 or 57), IS5Y (SEQ ID NOs: 19 or 58); IS4 (SEQ ID NOs: 20 or 62), IS2A (SEQ ID NOs: 21 or 65), IS2D (SEQ ID NOs: 22 or 67), IS2E (SEQ ID NO: 23), IS2F (SEQ ID NOs: 24 or 69), IS2H (SEQ ID NO: 25), IS2I (SEQ ID NOs: 26 or 70), IS2K (SEQ ID NOs: 27 or 71), IS30A (SEQ ID NO: 33), IS30B (SEQ ID NO: 34), IS30C (SEQ ID NOs: 35 or 63), IS30D (SEQ ID NO: 36), IS150 (SEQ ID NO: 37), IS186A (SEQ ID NOs: 38 or 59), IS186B (SEQ ID NOs: 39 or 60), IS186C (SEQ ID NOs: 40 or 61); IS600 (SEQ ID NO: 41), IS609 (SEQ ID NOs: 42 or 64), IS911A (SEQ ID NO: 43), IS911B (SEQ ID NO: 44), ISX (SEQ ID NO: 45), and ISZ (SEQ ID NO: 46), or homologues thereof;
(f) IS5I (SEQ ID NOs: 14 or 54), or a homologue thereof;
(g) IS1A (SEQ ID NO: 2), IS1B (SEQ ID NO: 3), IS1C (SEQ ID NO:4), IS1D (SEQ ID NOs: 5 or 47), IS1E (SEQ ID NOs: 6 or 48), IS1F (SEQ ID NO: 7), IS1H (SEQ ID NOs: 8 or 49), IS5A (SEQ ID NO: 9), IS5B (SEQ ID NOs: 10 or 50), IS5D (SEQ ID NOs: 11 or 51), IS5F (SEQ ID NOs: 12 or 52), IS5H (SEQ ID NOs: 13 or 53), IS5I (SEQ ID NOs: 14 or 54), IS5K (SEQ ID NOs: 15 or 55), IS5LO (SEQ ID NO: 16), IS5R (SEQ ID NOs: 17 or 56), IS5T (SEQ ID NOs: 18 or 57), IS5Y (SEQ ID NOs: 19 or 58), IS4 (SEQ ID NOs: 20 or 62), IS2A (SEQ ID NOs: 21 or 65), IS2D (SEQ ID NOs: 22 or 67), IS2E (SEQ ID NO: 23), IS2F (SEQ ID NOs: 24 or 69), IS2H (SEQ ID NO: 25), IS2I (SEQ ID NOs: 26 or 70), IS2K (SEQ ID NOs: 27 or 71), IS3A (SEQ ID NO: 28), IS3B (SEQ ID NOs: 29 or 66), IS3C (SEQ ID NO: 30), IS3D (SEQ ID NO: 31), IS3E (SEQ ID NOs: 32 or 68), IS30A (SEQ ID NO: 33), IS30B (SEQ ID NO: 34), IS30C (SEQ ID NOs: 35 or 63), IS30D (SEQ ID NO: 36), IS150 (SEQ ID NO: 37), IS186A (SEQ ID NOs: 38 or 59), IS186B (SEQ ID NOs: 39 or 60), IS186C (SEQ ID NOs: 40 or 61), IS600 (SEQ ID NO: 41), IS609 (SEQ ID NOs: 42 or 64), IS911A (SEQ ID NO: 43), IS911B (SEQ ID NO: 44), ISX (SEQ ID NO: 45), and/or ISZ (SEQ ID NO: 46), or homologues thereof; or
(h) insL-2 (SEQ ID NOs: 1 or 218) or a homologue thereof.

In embodiments, the method further comprises modifying the bacterial strain to revert spontaneous mutations. This prevents spontaneous mutations from impacting the phenotype of the modified bacterial strain produced.

In embodiments, the method further comprises introducing one or more mutations into the bacterial strain, optionally wherein the mutation is in one or more of the following genes: *ompT*, *hsdSB, hsdRMS, dcm, trxB, gor, recA, lacR, endA, luxS, acrAB,* or *acrR.* Modifications to one or more of these genes may further improve the phenotype of bacterial strains for biotechnological processes.

### BRIEF DESCRIPTION OF THE DRAWINGS

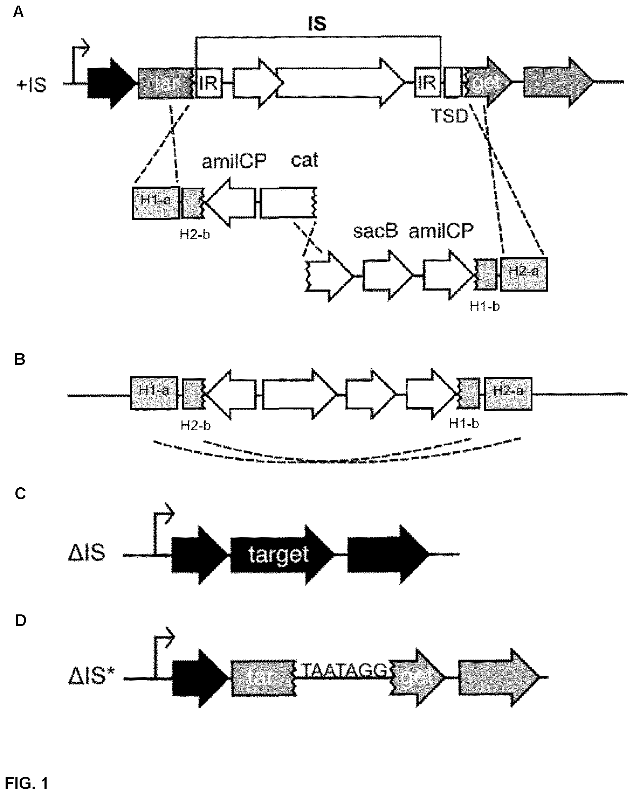

**Figure 1****. Example of a scarless and seamless deletion method as described in Nävsall (2017).** A) An insertion sequence (IS) lies between two inverted repeats (IR) in a target genome sequence ('tar' and 'get'). Some insertion sequences are also found with a corresponding target site duplication (TSD) that was generated by the IS upon insertion into the genome. To delete the IS and corresponding TSD, two partial insertion cassettes are electroporated into the target bacterium. The first partial insertion cassette comprises a long (~40 nucleotide) sequence (H1-a) identical to the end of the 'tar' genome sequence immediately before (5') the IS, a short (~15 nucleotide) sequence (H2-b) identical to the beginning of the `get' genome sequence immediately after (3') the IS, and truncated *cat* sequence. The first partial insertion cassette used also includes additional selection gene *amilCP* (blue colour). The second partial insertion cassette comprises the remainder of the truncated *cat* selection gene used in the first partial insertion cassette, and the truncated *cat* sequences in the first and second partial cassette have ~277 nucleotide overlap, such that the truncated selection gene in the first and second partial insertion cassette, when recombined, form a functional *cat* sequence. The second partial insertion cassette used also includes additional selection gene *amilCP* in inverted orientation to that in the first partial cassette (blue colour) and a short (~15 nucleotide) 'tar' sequence (H1-b) and a long (~40 nucleotide) 'get' sequence (H2-a). Three recombination events (indicated by dotted lines) replace the IS with the two partial insertion cassettes to generate the metastable intermediate shown in (B) between two copies of the desired seamless deletion junction H1-H2 directly adjacent the end of the 'tar' and the beginning of the 'get' sequence. The 5' copy of the H1-H2 junction is comprised of a long (~40 nucleotide) 'tar' sequence (H1-a) and a short (~15 nucleotide) 'get' sequence (H2-b). The 3' copy of the H1-H2 junction is comprised of a short (~15 nucleotide) 'tar' sequence (H1-b) and a long (~40 nucleotide) 'get' sequence (H2-a). The recombination events can be catalysed by lambda *red* induction, for example using pSIM plasmids such as pSIM5-Tet. B) the metastable intermediate then undergoes a spontaneous homologous recombination event between the duplicated H1-H2 homology regions (i.e., the H1-a-H2-b region recombines with the H1-b-H2-a region) to remove the cassette and generates the desired deletion shown in (C). The dotted arrows in B) indicate the homologous regions that recombine. C) Deletion of the IS and corresponding TSD can allow expression of the target gene, and adjacent genes, in which the IS/TSD was inserted. D) Alternatively, the first and second partial insertion cassettes are modified to contain a double stop and frame-shift mutation sequence such as TAATAGG shown here, to prevent potential target expression upon deleting the insertion sequence.

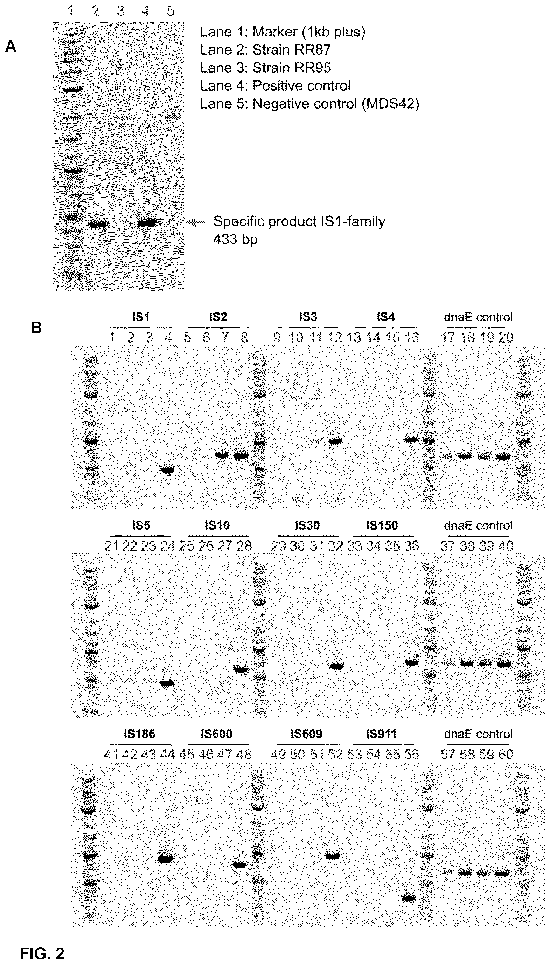

**Figure 2****. Confirming deletion of insertion sequences.** A) Strain RR87 in lane 2 has a single copy of IS1 and produces the expected positive band, confirming specific IS1 detection. Strain RR95 in lane 3 is the same as RR87 with IS1 deleted and does not produce the specific IS1 band, confirming that all IS1 are deleted in RR95. B) shows the successful deletion of IS' in strains RR452 (all IS deleted, indicated by lane numbers 1, 5, 9, 13, 17, 21, 25, 29, 33, 37, 41, 45, 49, and 53), RR530 (all IS deleted and stop sequences inserted instead of various IS', indicated by lane numbers 2, 6, 10, 14, 18, 22, 26, 30, 34, 38, 42, 46, 50, and 54) and RR516 (only IS2 and IS3 retained, indicated by lane numbers 3, 7, 11, 15, 19, 23, 27, 31, 35, 39, 43, 47, 51, and 55). Results for positive controls are indicated in lane numbers 4, 8, 12, 16, 20, 24, 28, 32, 36, 40, 44, 48, 52, and 56). Control PCR for the essential gene *dnaE* produced the expected band of 682 bp in all strains (lane numbers 57-60).

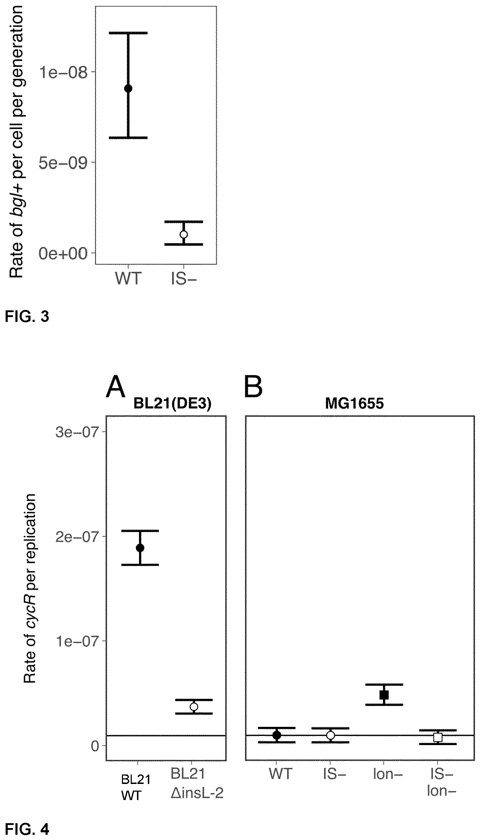

**Figure 3****. Bacterial strains lacking all insertion sequences show a reduced *bgl*+ mutation rate.** Comparison of bacterium of strain MG1655 which lack all insertion sequences (*IS*-, RR452) compared to the wild type MG1655 strain (WT). The error bar indicates 95% confidence interval of the estimate derived from 40 parallel cultures for each strain.
**Figure 4****. Interaction between the Lon protease and insertion sequences on mutation rate.** A) Reduced *cycR* mutation rates were observed in the gene *cycA* for strain BL21(DE3) where IS insL-2 was deleted (BL21 ΔinsL-2, strain RR517) compared to the wild type BL21 (DE3) bacteria (BL21 *Ion*-)*.* B) Equal *cycR* mutation rate was observed for strain RR452 MG1655 where all insertion sequences are deleted (*IS*-) compared to the wild type MG1655 bacteria (WT). Where Lon protease alone is deleted without any modification of the insertion sequences an increased *cycR* mutation rate was observed (*Ion*-, strain RR532) compared to wild type MG1655 bacteria (WT), however the impact of deleting Lon protease is negated by deleting all insertion sequences (*IS- lon-,* strain RR497). The error bar indicates the 95% confidence interval of the estimate derived from 40-80 parallel cultures for each strain.

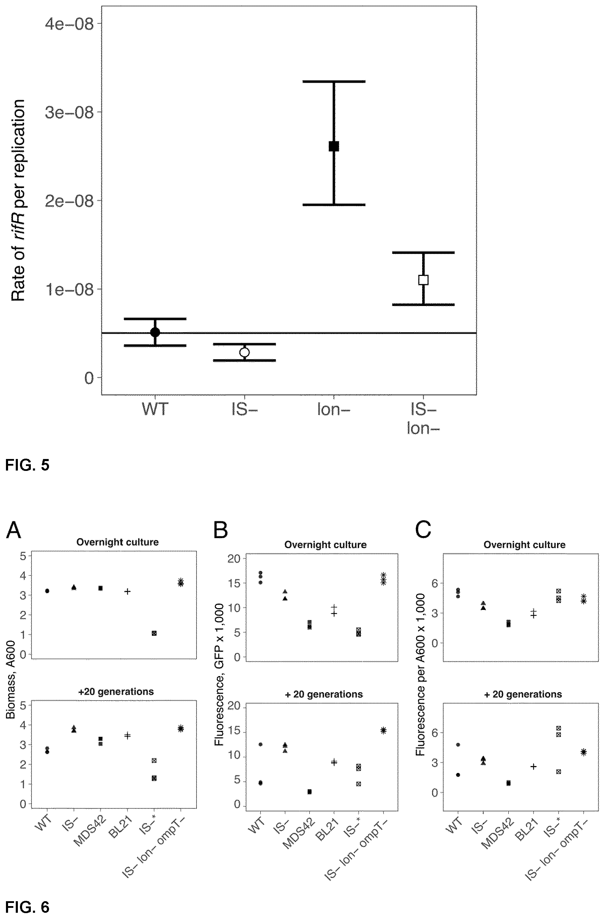

**Figure 5****. Reduced *rifR* mutation rates in the gene *rpoB* for MG1655 bacteria lacking insertion sequences (*IS*-) compared to the wild type MG1655 bacteria (WT).** Results for MG1655 bacteria lacking Lon protease (*Ion*-) or lacking Lon protease and all insertion sequences (*IS- Ion*-) are also shown. WT is MG1655 wild type strain, *IS*- is MG1655 strain RR452, *Ion-* is MG1655 strain RR532, and *IS- Ion-* is MG1655 strain RR497. The error bar indicates the 95% confidence interval of the estimate derived from 40 parallel cultures for each strain.
**Figure 6****. Assessment of plasmid stability and production of recombinant GFP after growth overnight (upper panels) or after 20 generations of growth (lower panels)** A) biomass, B) fluorescence, and C) normalized fluorescence to measured biomass. WT is the MG1655 strain, *IS-*is MG1655 strain RR452, MDS42 is the Blattner et al. strain, BL21 is the BL21 wild type strain, *IS*-* is MG1655 strain RR530, and *IS- Ion- ompT-* is MG1655 strain RR535. Data points indicate measurements of three technical replicates.

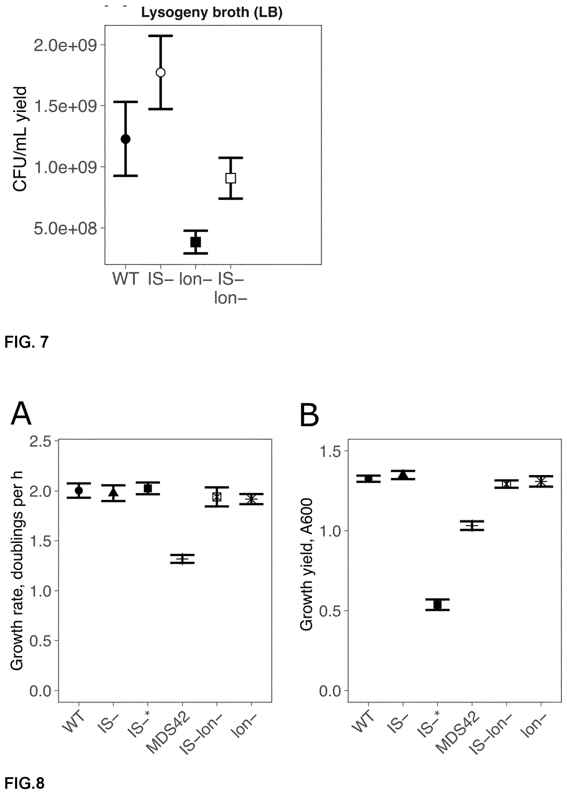

**Figure 7****. Increased yield of colony forming units per mL compared to the wild type MG1655 in lysogeny broth.** *IS*- is MG1655 strain RR452, *Ion-* is MG1655 strain RR532 and *IS- Ion-* is MG1655 strain RR497. The error bar indicates the standard deviation of the mean for 6-10 biological replicates.
**Figure 8****. Growth parameters of bacterial strains.** A) growth rate of bacterial strains in terms of doublings per hour, B) total biomass yield in saturated cultures after 22h of cultivation was measured as absorbance at 600 nm (A600) without dilution. WT is MG1655 wild type, *IS*- is MG1655 strain RR452, *IS*-* is MG1655 strain RR530, MDS42 is the Blattner et al. BL21 strain, *IS- Ion-* is MG1655 strain RR497 and *Ion-* is MG1655 strain RR532. Error bars show standard deviation of 30 technical replicates.

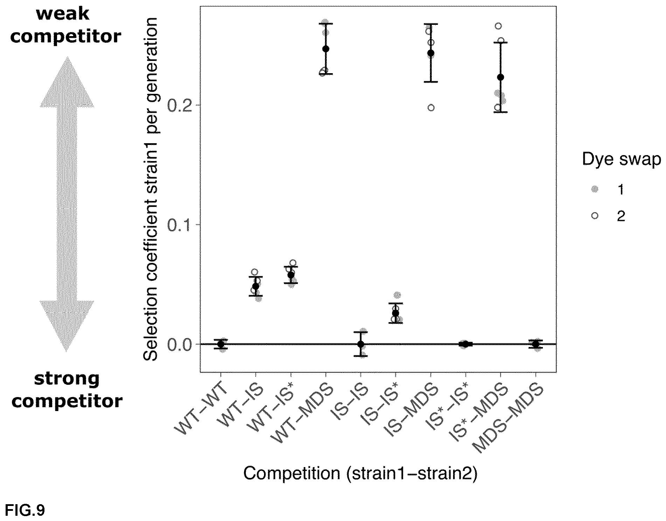

**Figure 9****. Competitive growth in co-culture competition experiments.** Co-culture combinations are indicated with the format strain 1 - strain 2. Values are shown as a selection coefficient for strain 1 per generation of growth. Positive values indicate that strain 1 is more competitive compared to strain 2. Low values indicate high competitive ability of strain 2, while high values indicate that strain 2 is a weak competitor against strain 1. Competition was performed using dye-swap design and across 60 generations of growth. "WT" refers to MG1655, *IS*- refers to MG1655 strain RR452, *IS*-* refers to MG1655 strain RR530 and MDS refers to MDS42 (Blattner et al.). Error bars indicate standard deviation of 3-6 biological replicates. Filled black circles indicate the average value. Dye swap is indicated with grey and white circles.

### DETAILED DESCRIPTION OF THE INVENTION

### General definitions

Unless otherwise defined below, all technical terms used herein have the same meaning as commonly understood by one of the ordinary skill in the art in the field to which this disclosure belongs.

Any reference to 'or' herein is intended to encompass 'and/or' unless otherwise stated.

As used herein, the singular forms 'a', 'an', and `the' include both singular and plural referents unless the context clearly dictates otherwise.

The terms 'comprising', 'comprises' and 'comprised of' as used herein are synonymous with including, includes or containing, contains, and are inclusive or open-ended and do not exclude additional, non-recited members, elements or method steps. The term also encompasses 'consisting of' and 'consisting essentially of'.

Whereas the term 'one or more', such as one or more members of a group of members, is clear per se, by means of further exemplification, the term encompasses inter alia a reference to any one of said members, or to any two or more of said members, such as, e.g., any >3, >4, >5, >6 or >7 etc. of said members, and up to all said members.

'Consecutive' or 'contiguous' may be used herein to describe that something is located immediately adjacent to something else, i.e., without a gap. Therefore, consecutive or contiguous nucleotides refers to a set polynucleotide sequence.

The term 'nucleotide' refers to any nucleoside having one or more phosphate groups joined in ester linkages to the sugar moiety. Exemplary nucleotides include nucleoside monophosphates, diphosphates and triphosphates. The term nucleotide is used generally to refer to nucleotides found in DNA and RNA; adenine (A), guanine (G), cytosine (C), thymine (T) and uracil (U). The terms 'polynucleotide' refer to a polymer of nucleotides joined together by a phosphodiester or phosphorothioate linkage between 5' and 3' carbon atoms, including DNA and RNA. As used herein, the term `DNA' or 'deoxyribonucleotide' is used to specifically refer to a polymer of A, T, C and G nucleotides. A polynucleotide as referred to herein, may be any length, including at least 2, 3, 4, 5, 10, 15, 20, 25, 30, 50, 100, 1000, 10,000 or more nucleotides.

The term 'sequence' may refer to any contiguous string of nucleotides, such as a genomic sequence or oligomer. A 'sequence' may be at least 2, 5, 10, 15, 20, 30, 40, 50, 100, 200, 500, 1000 nucleotides in length.

'Encoding' or 'coding' used herein may mean a sequence of nucleotides which upon transcription into RNA and subsequent translation into protein, would lead to the synthesis of the stated protein, peptide, or amino acid sequence. Therefore a 'coding sequence' may refer to a polynucleotide sequence that upon transcription into RNA and subsequent translation into protein, would lead to the synthesis of the stated protein, peptide, or amino acid sequence. Therefore, 'non-coding sequence' may refer to polynucleotide sequences that may be considered not to code a protein.

'Genome size' refers to the number of nucleotide residues that make up the entire genome of a specified bacterial strain.

The term 'sequence' may refer to any contiguous string of nucleotides, such as a genomic sequence or oligomer. A 'sequence' may be at least 2, 5, 10, 15, 20, 30, 40, 50, 100, 200, 500, 1000 nucleotides in length. Unless otherwise specified, DNA sequences are by convention written from 5' to 3' end.

'Bacterial genome sequence' may refer to a polynucleotide sequence that makes up the entire coding and non-coding sequence (genome) of a bacterial strain. This includes both coding and non-coding sequences. 'Bacterial genome sequence' as used herein may refer to the whole genome sequence, or only part (or section) of the genome sequence. In examples where 'bacterial genome sequence' refers to a part (or section) of the genome sequence, this may be used to refer to a polynucleotide sequence within the genome sequence e.g., where the polynucleotide sequence is endogenous to the bacterial strain. In examples, the polynucleotide sequence is between 2-200, 5-200, 5-180, 5-170, 5-160, 5-150, 5-125, 5-100, 5-90, 5-80, 5-70, 5-60, 8-100, 8-90, 8-80, 8-70, 8-60, 10-150, 10-125, 10-100, 10-90, 10-80, 10-70, 10-60 nucleotide residues in length.

The term 'gene' is used in line with the art. Briefly, 'gene' refers to a basic unit of DNA or genomic sequence which may instructions for a specific molecule such as RNA or a protein.

'Pseudogene' may refer to a gene that resembles a functional gene but has accumulated mutations that prevent it from being transcribed or translated into a functional protein.

'Stop codon' may refer to a sequence of three nucleotides (i.e., a codon) in DNA or RNA that signals the termination of protein synthesis (i.e., translation) in a cell. A stop codon may be TAG, TAA, or TGA in DNA.

'Homology', 'sequence identity' or 'sequence similarity' in the context or two or more polynucleotides may refer to the extent to which the sequence of nucleotides are the same over a specified region. Therefore, a sequence that is 'homologous' to another sequence is the same as (or equivalent to) that sequence. The extent of homology may also be reported as a 'percentage sequence similarity', 'percentage sequence identity' or 'percentage homology', which may be calculated by aligning the two sequences, comparing the two sequences over the specified region, determining the number of positions at which identical nucleotides occurs in both sequences to yield the number of matched positions, dividing the number of matched positions by the total number of positions in the specified region, and multiplying the result by 100 to yield the percentage of sequence identity. Methods of aligning sequences for comparison are well known in the art. Various programs and alignment algorithms are described in: Smith and Waterman, Adv. Appl. Math. 2:482, 1981; Needleman and Wunsch, J. Mol. Biol. 48:443, 1970; Pearson and Lipman, Proc. Natl. Acad. Sci. U.S.A. 85:2444, 1988; Higgins and Sharp, Gene 73:237, 1988; Higgins and Sharp, CABIOS 5:151, 1989; Corpet et al., Nucleotides Research 16:10881, 1988; and Pearson and Lipman, Proc. Natl. Acad. Sci. U.S.A. 85:2444, 1988. Altschul et al., Nature Genet. 6:119, 1994, presents a detailed consideration of sequence alignment methods and homology calculations. The NCBI Basic Local Alignment Search Tool (BLAST) (Altschul et al., J. Mol. Biol. 215:403, 1990) is available from several sources, including the National Center for Biotechnology Information (NCBI, Bethesda, Md.) and on the internet, for use in connection with the sequence analysis programs blastp, blastn, blastx, tblastn and tblastx. A description of how to determine sequence identity using this program is available on the NCBI website on the internet. Identity may be determined manually or by using a computer sequence algorithm such as ClustalW, ClustalX, BLAST, FASTA or Smith-Waterman. The popular multiple alignment program ClustalW *(*Nucleotides Research (1994) 22, 4673-4680; Nucleotides Research (1997), 24, 4876-4882) is a suitable way for generating multiple alignments of polypeptides or polynucleotides. Suitable parameters for ClustalW maybe as follows: For polynucleotide alignments: Gap Open Penalty= 15.0, Gap Extension Penalty= 6.66, and Matrix= Identity. For polypeptide alignments: Gap Open Penalty = 10. o, Gap Extension Penalty = 0.2, and Matrix = Gannet. For DNA alignments: ENDGAP = -1, and GAPDIST = 4. Those skilled in the art will be aware that it may be necessary to vary these and other parameters for optimal sequence alignment. Suitably, calculation of percentage identities is then calculated from such an alignment as (N/T), where N is the number of positions at which the sequences share an identical residue, and T is the total number of positions compared including gaps but excluding overhangs. The similarity between nucleotide sequences is expressed in terms of the similarity between the sequences, otherwise referred to as sequence identity. Sequence identity is frequently measured in terms of percentage identity (or similarity or homology); the higher the percentage, the more similar the two sequences are. Homologues or variants of the nucleotide sequence will possess a relatively high degree of sequence identity when aligned using standard methods.

The term 'bind' or 'hybridize' may refer to the pairing of substantially complementary or complementary nucleotide sequences within two different molecules. Pairing can be achieved by any process in which a nucleotide sequence joins with a partially, substantially or fully complementary sequence through base pairing to form a hybridization complex. For purposes of hybridization, two nucleotide sequences or segments of sequences are 'substantially complementary' if at least 80%, 85, 90, 95 or 99% of their individual bases are complementary to one another. Two nucleotide sequences or segments of sequences are `partially complementary' if at least 50% of their individual bases are complementary to one another. The specificity of single-stranded DNA to hybridize complementary fragments is determined by the 'stringency' of the reaction conditions (Sambrook et al., Molecular Cloning and Laboratory Manual, Second Ed., Cold Spring Harbor (1989)). Hybridization stringency increases as the propensity to form DNA duplexes decreases. In polynucleotide hybridization reactions, the stringency can be chosen to favour specific hybridizations (high stringency), which can be used to identify, for example, full-length clones from a library. Less-specific hybridizations (low stringency) can be used to identify related, but not exact (homologous, but not identical), DNA molecules or segments. DNA duplexes are stabilised by: (1) the number of complementary base pairs; (2) the type of base pairs; (3) salt concentration (ionic strength) of the reaction mixture; (4) the temperature of the reaction; and (5) the presence of certain organic solvents, such as formamide, which decrease DNA duplex stability. In general, the longer the probe, the higher the temperature required for proper annealing. A common approach is to vary the temperature; higher relative temperatures result in more stringent reaction conditions. To hybridize under 'stringent conditions' describes hybridization protocols in which polynucleotides at least 60% homologous to each other remain hybridized. Generally, stringent conditions are selected to be about 5°C lower than the thermal melting point (Tm) for the specific sequence at a defined ionic strength and pH. The Tm is the temperature (under defined ionic strength, pH, and polynucleotide concentration) at which 50% of the probes complementary to the given sequence hybridize to the given sequence at equilibrium. Since the given sequences are generally present at excess, at Tm, 50% of the probes are occupied at equilibrium. 'Stringent hybridization conditions' or 'high stringency conditions' are conditions that enable a polynucleotide to hybridize only to its specific sequence. Stringent conditions are sequence- 5 dependent and will differ. Stringent conditions typically comprise: (1) low ionic strength and high temperature washes, for example 15 mM sodium chloride, 1.5 mM sodium citrate, 0.1 % sodium dodecyl sulphate, at 50°C; (2) a denaturing agent during hybridization, for example, 50% (v/v) formamide, 0.1 % bovine serum albumin, 0.1 % Ficoll, 0.1 % polyvinylpyrrolidone, 50 mM sodium phosphate buffer (750 mM sodium chloride, 75 mM sodium citrate; pH 6.5), at 42°C; or (3) 50% formamide. Washes typically also comprise 5xSSC (0.75 M NaCl, 75 mM sodium citrate), 50 mM sodium phosphate (pH 6.8), 0.1 % sodium pyrophosphate, 5xDenhardt's solution, sonicated salmon sperm DNA (50 µg/mL), 0.1 % SOS, and 10% dextran sulphate at 42°C, with a wash at 42°C in 0.2xSSC (sodium chloride/sodium citrate) and 50% formamide at 55°C, followed by a high-stringency wash consisting of 0.1 xSSC containing EDTA at 55°C. Suitably, the conditions are such that sequences at least about 65%, 70%, 75%, 85%, 90%, 95%, 98%, or 99% homologous to each other typically remain hybridized to each other. 'Moderately stringent conditions' or 'moderate stringency conditions' use washing solutions and hybridization conditions that are less stringent, such that a polynucleotide will hybridize to the entire, fragments, derivatives, or analogues of the polynucleotide. One example comprises hybridization in 6xSSC, 5xDenhardt's solution, 0.5% SOS and 100 µg/mL denatured salmon sperm DNA at 55°C, followed by one or more washes in 1xSSC, 0.1 % SOS at 37°C. The temperature, ionic strength, etc., can be adjusted to accommodate experimental factors such as probe length. Other moderate stringency conditions have been described (see Ausubel et al., Current Protocols in Molecular Biology, Volumes 1-3, John Wiley & Sons, Inc., Hoboken, N.J. (1993); Kriegler, Gene Transfer and Expression: A Laboratory Manual, Stockton Press, New York, N.Y. (1990); Perbal, A Practical Guide to Molecular Cloning, 2nd edition, John Wiley & Sons, New York, N.Y (1988)). 'Low stringent conditions' or 'low stringency conditions' use washing solutions and hybridization conditions that are less stringent than those for moderate stringency, such that a polynucleotide will hybridize to the entire, fragments, derivatives, or analogues of the polynucleotide. A non-limiting example of low stringency hybridization conditions includes 10% formamide, 5x Denhardt's solution, 6x SSPE, 0.2% SDS at 22°C, followed by washing in Ix SSPE, 0.2% SDS, at 37°C. Denhardt's solution contains 1% Ficoll, 1% polyvinylpyrolidone, and 1% bovine serum albumin (BSA). 20x SSPE (sodium chloride, sodium phosphate, ethylene diamide tetraacetic acid (EDTA)) contains 3M sodium chloride, 0.2M sodium phosphate, and 0.025 M (EDTA). Other conditions of low stringency are well-described (see Ausubel et al., 1993; Kriegler, 1990).

'Truncation' may be used herein to refer to a portion of a complete polynucleotide sequence. Therefore, in some examples, 'truncation' may be with reference to a polynucleotide sequence that normally encodes a specified protein. The truncation may be such that the selection gene consists of less than 90, 80, 70, 60, 50, 40, 30, 20 or 10% of the reference sequence.

'Selection marker' may be used to refer to a polynucleotide sequence (normally a gene) which confers a phenotype to a bacterial strain that can be selected for. This may include resistance to an antibiotic, resistance to an herbicide, colorimetric markers, enzymes, fluorescent markers, and the like. Examples of selectable marker genes known and used in the art include genes providing resistance to ampicillin, streptomycin, gentamycin, spectinomycin, kanamycin, zeocin, chloramphenicol, hygromycin, and the like.

'Mutation' may refer to any change to nucleotide residues within a sequence (such as insertions, deletions or changing one or more nucleotide residues).

'Frameshift mutation' may refer to a mutation which changes the reading frame for the sequence following the mutation site. As the DNA is read as codons (triplets of nucleotides), `frameshift mutation' may refer to an insertion or deletion of a number of nucleotide residues that is not divisible by three (i.e., insertion or deletion of 1, 2 or 4 nucleotide residues). A frameshift mutation can therefore result in a different grouping of nucleotides into codons and can therefore result in different amino acids being used to form a protein and/or the creation or removal of a premature stop codon.

'Target' may be used herein to refer to a polynucleotide, IS, gene, or bacterial strain of interest, i.e., that is the intended subject of an experimental manipulation.

'Homologue' may be used to refer to a structurally similar or related polynucleotide sequence. In some examples, `homologue' may refer to a structurally similar or related polynucleotide sequence found in a different organism, such as a different bacterial strain. The term 'homologue' is used to define that there is a high degree of sequence similarity between the homologue and the specified polynucleotide sequence. In examples, the sequence similarity is at least 60, 65, 70, 75, 80, 85, 90, 95 or 99% between the homologue and the specified polynucleotide.

'Exogenous' may refer to something, such as a nucleotide, that originates from out with the bacterial strain. When recombination occurs between a target polynucleotide sequence and a partial insertion cassette as described herein, the resulting sequence in the bacterial genome comprises the recombined sequence from the partial insertion cassette, which may be considered exogenous sequence. However, as the recombination is between polynucleotide sequences with a high degree of similarity (if not identical), then the term 'additional exogenous sequence elements' may be used herein to refer to the insertion of extra nucleotides that were not originally found in the bacterial genome sequence being targeted.

'Native sequence' may refer to the genome sequence in a bacterial strain before one or more specified IS were inserted. This may be identified by sequence alignment to other strains. Therefore, 'native coding sequence' may refer to the coding sequence of a bacterial genome before one or more specified IS were inserted.

'Plasmid' may refer extrachromosomal genetic elements composed of DNA that are not part of the bacterial genome but can propagate themselves autonomously in bacteria. A plasmid may refer to native plasmids in bacteria, but also any synthetic, exogenous, modified or chimeric plasmids that can be, or have been inserted into the bacteria.

'Phage' may refer to an extrachromosomal bacteriophage capable of propagating in bacteria, such as bacteriophage P1. A phage may refer to naturally occurring bacteriophage, as well as any modified or chimeric phages (e.g., having deletions, additions or substitutions or assembled from functional parts of different phage).

'Cassette' may be used to refer to a polynucleotide sequence.

'Introducing' may refer to a process by which polynucleotides are delivered to a bacterial cell in such a manner that the polynucleotide gains access to the interior of the bacterial cell and includes such terms as 'transformation', 'transfection' and/or 'transduction'. Where more than one polynucleotide, e.g., partial insertion cassette, is to be introduced these can be introduced into cells in a single transformation event and/or in separate transformation events. Thus, in some aspects of the present invention one or more polynucleotides can be introduced into a cell of a bacterial cell.

Unless otherwise specified, reference to the partial insertion cassette may be understood to refer to the partial insertion cassette *per se,* including when the cassette when recombined into the genome of a bacterial strain.

'Bacterial strain' may be used herein to refer to a population of bacteria (i.e., a group of two or more bacteria) with specific characteristics that distinguish the bacteria from other bacterial populations. For example, one group of bacteria may be a genetic variant of a second group of bacteria. Therefore 'bacterial strain' may be used interchangeably with 'bacteria'.

'Modified bacterial strain' may refer to a strain of bacteria that have a modification (i.e., experimental manipulation) of the bacteria. In examples, the modification may be deletion of one or more target IS as described herein.

Therefore, 'unmodified bacterial strain' may refer to a bacterial strain before the specified modification has been made, e.g., before a target IS has been deleted. Therefore, the unmodified bacterial strain may have all IS present. Alternatively, the unmodified strain may have had most IS deleted but retain a target IS. Therefore, an unmodified bacterial strain may have been modified in another way e.g., to delete one more non-target IS elements, modification of one or more genes etc. In other words, the unmodified bacterial strain may not have had the specified modification (e.g. deletion of a target IS) but has other modifications present. The nucleotide sequence of the genome of the 'unmodified bacterial strain' may be partially or completely known. In examples where the bacterial strain is a synthetic bacterial strain, the unmodified bacterial strain may refer to a reference bacterial strain sequence.

'Lacking' may have the normal definition of meaning that an element is not present in the bacterial strain. This can be comparison to the bacterial strain before any modification was made (i.e., before any experimental manipulation or intervention on the bacterial strain). This can also be by comparison to a reference genome of the bacterial strain. Where a bacterial strain is 'lacking all IS', this may mean that the bacterial strain contains no (zero) IS.

'Inverted repeat' or 'IR' may refer to a polynucleotide sequence around 5-60, 5-55, 5-50, 5-45, 5-40, 10-60, 10-55, 10-50, 10-45 or 10-40 nucleotides in length. Inverted repeats are mirror sequences of each other that may be imperfect. In other words, the inverted repeats may not be an identical mirror of each other i.e., one inverted repeat may have a mismatch, insertion or deletion compared to it's paired inverted repeat. A pair of IR may define an IS. A pair of IR may be located on the same strand. The inverted repeats for some IS families/sub-families are known (see, for example, Siguier et al., 2015, which is incorporated herein by reference).

`Insertion sequence' of 'IS' may refer to a polynucleotide sequence around 600-2500, 700-2500, 750-2500, 700-2000, 750-2000, 700-1800, 750-1800, 700-1600, 750-1600, 700-1500, or 700-1500 nucleotides in length, and comprises, consists or consists essentially of nucleotide residues located between and including two inverted repeats (IR). In other words, 'IS' may include the IR. However, some IS have a sequence that overlaps with the bacterial genome sequence. In this case, 'IS' may comprise, consist or consist essentially of only the IS nucleotide residues between two IR that do not overlap with bacterial genome sequence. An IS may encode a transposase enzyme. An IS may be linear or circular. 'IS' may also include an associated TSD.

'Target site duplication' ( 'TSD') and 'direct repeat' (`DR') may be used interchangeably to refer to a polynucleotide sequence that is located immediately adjacent to a specified IS in a bacterial genome. A TSD may be 2-20, 2-17, 2-15, 2-13, 2-12, 2-11, 2-10, 3-20, 3-17, 3-15, 3-13, 3-12, 3-11, 3-10, 4-20, 4-17, 4-15, 4-13, 4-12, 4-11, or 4-10 nucleotide in length. The TSD may be positioned 5' or 3' to the IS. For example, the TSD may be positioned 5' or 3' to IS sequence. It is understood that, when referring to DNA, there is a TSD on each DNA strand, such that the overall IS may be considered to be flanked by a TSD. The TSD is generated upon insertion of the IS into the bacterial genome.

'IS-associated sequence' may refer to TSD/DR.

'Non-IS sequences' may be used to refer to bacterial genome sequence before the IS was inserted (native sequence) that do not contribute to the IS as defined herein. In some examples, non-IS sequences may define bacterial genome sequence that is not contained between and including two inverted repeats. However, in examples where IS sequence overlaps with bacterial genome sequence, 'non-IS sequences' excludes IS-only sequence that does not overlap with bacterial genome sequence. 'Non-IS sequences' may not include any TSD. In other words, native (target) sequences generated by transposase activity during entry of an insertion sequence may be deleted in the modified bacterium.

'Non-IS sequences flanking the IS' may refer to native sequences present immediately adjacent to the IS, i.e. sequences which were present in the bacterium before entry of the insertion sequence. This may refer to sequences immediately adjacent to the inverted repeats at either end of the insertion sequence.

'Specific deletion' may convey that the IS is deleted from the modified bacterial strain without deletion of non-IS sequences flanking the IS.

'Overlap' in the context of two or more polynucleotide sequences may refer to a region of the two or more polynucleotides that have an identical, or nearly identical (i.e., at least 95, 96, 97, 98 or 99% sequence identity), sequence. The region of overlap between the two or more polynucleotides may be at least 2, 3, 4, 5, 6, 7, 8, 9, 10, 12 or 15 nucleotides in length. Therefore, in some examples a polynucleotide sequence, such as a polynucleotide sequence in a bacterial genome, may comprise, consist or consist essentially of overlapping flanking polynucleotide sequences. In examples, a polynucleotide sequence that simultaneously comprises polynucleotide sequences of more than one sequence element.

'Flanking' may refer to first polynucleotide sequence being positioned adjacent to a second polynucleotide sequence. Adjacent or flanking may mean that the first polynucleotide sequence is positioned 5' or 3' to the second polynucleotide sequence. Flanking may refer to a first polynucleotide sequence being immediately adjacent to a second polynucleotide sequence (i.e., there is no gap between the first and second polynucleotide). 'Flanking' may refer to a sequence being positioned on both sides of a specified sequence.

'First' and 'second' may be used only to differentiate between elements such as a first and second IS, first and second partial insertion cassette, a first and second polynucleotide, or a first and second bacterial strain. These terms may not indicate positioning or order of steps unless otherwise specified.

'End' may be used to refer to a 5' or 3' region of a defined polynucleotide sequence. The 'end' may be the 5'-most and 3'-most nucleotide of a defined polynucleotide sequence. The 'end' may define a change or boundary of a defined sequence within a genome.

'Derivative strain' may be used to describe a bacterial strain described herein that has been further genetically modified, e.g., deletion or alteration of existing genes, introduction of foreign material or other genetic changes, to produce a derivative strain. 'Hybrid' strain may refer to a bacterial strain that has genetic material from two or more parental bacterial strains.

### a. Bacterial strains

The bacterial strains described herein may be an *E. coli.* The bacterial strains may be an *E. coli* of strain K-12 or B, or any strain having at least 60%, 65%, 70%, 75%, 80%, 85%, 90%, 95%, 97%, 98%, or 99% sequence identity therewith, or any hybrid or derivative strain thereof. In some embodiments, the bacterial strain may be an *E. coli* of strain B, BL21, REL606, C, Nissle 1917, W, K-12 MG1655, K-12 EMG2, K-12 WG1, K-12 W2637, K-12 W3110, K-12, DH5alpha, HMS173, DH10B, Invα, Top10, Top10F, Jm103, JM105, JM109, MC1061, MC4100, XL1-Blue, EC100 or EC300, or a hybrid or derivative strain thereof.

The complete genomic sequence of several strains of *E. coli* and other commonly used laboratory microorganisms is known (see, e.g., Blattner et al., Science, 277:1453-74, 1997; GenBank

Accession No. U00096; NCBI database, Accession No. AP009048, Perna et al., Nature, 409, 529-533, 2001; Hayashi et al., DNA Res., 8, 11-22, 2001; Welch et al., Proc. Natl. Acad. Sci., USA 99:17020-17024, 2002 and GenBankAccession No. AE014075, each of which is incorporated herein by reference). However, the partial or complete genomic sequence of the unmodified bacterial strain may be determined using routine sequencing methods. Sequencing methods are standard in the art and are described in Besser et al., (2018. Next generation sequencing technologies and their application to the study and control of bacterial infections. Clinical Microbiology and Infection, 24(4): 335-341). Examples of known sequencing methods include Sanger sequencing and Oxford Nanopore Sequencing.

The reference genome of bacterial strain MG1655 is set forth in NCBI accession number U00096.3 (SEQ ID NO: 201). The sequence of the MG1655 wild type strain used herein is set out in SEQ ID NO: 219).

### b. IS and TSD

Identification of IS copy numbers and chromosomal locations is routine in the art. Indeed, IS copy numbers and chromosomal locations can be identified using semi-automatic annotation software, including ISsaga (which is integrated into the ISfinder platform (Siguier et al. 2006. ISfinder: the reference centre for bacterial insertion sequences. Nucleic Acids Res, 34: D32-6. Doi: 10.1093/nar/gkj014)), ISScan (Wagner et al., 2007. A survey of bacterial insertion sequences using IScan. Nucleic Acids Res, 35: 1-10. doi:10.1093/nar/gkm597), Oasis (Robinson et al., 2012. An automated program for global investigation of bacterial and archaeal insertion sequences. Nucleic Acids Res, 40(22): e174. https://doi.org/10.1093/nar/gks778) and ISEScan (Xie & Tang 2017, Bioinformatics 33(21), 3340-3347, https://pubmed.ncbi.nlm.nih.gov/29077810/). Any identified IS can be manually checked to ensure accuracy.

Some IS have sequences that overlap with the bacterial genome (also referred to herein as overlapping with non-IS ORF). These IS can be manually identified in a bacterial sequence by comparing the bacterial genome sequence and the known IS sequence.

In addition, some IS have a second IS inserted into the IS sequence. In other words, the IS is 'split'. Split IS can be identified using the methods described above.

Target site duplication (TSD) sequences may vary in position, sequence and sequence length depending on where the IS was inserted. A TSD adjacent to an IS can be identified by comparing the DNA sequence immediately flanking the terminal inverted repeats according to known characteristics of TSDs for the respective IS families (Siguier et al. 2014, FEMS Microbiol Rev, 38(5): 865-891 https://doi.org/10.1111/1574-6976.12067). For example, insertions of IS1 are known to be flanked by 8-9 bp TSDs, and insertions of IS5 by shorter 4 bp TSDs (Siguier et al. 2014).

The MG1655 strain used herein comprises 45 IS shown in **Table 1** below.

**Table 1. IS (and associated TSD) present in MG1655 Strain**

| **IS** | **SEQ ID NO of IS only** | **Size of IS (excluding TSD) (nucleotide residues)** | **SEQ ID NO of IS + TSD** | **Size of TSD (nucleotide residues)** | **Position in SEQ ID NO: 219** | **Position with reference to U00096.3 strain** |
|---|---|---|---|---|---|---|
| 1A | 2 | 768 | - | - | 19796..20563 | 19796..20563 |
| 1B | 3 | 768 | - | - | 278387..279154 | 279163..279930 |
| 1C | 4 | 763 | - | - | 289863..290625 | 290639..291401 |
| 1D | 5 | 768 | 47 | 9 | 1048884.. 1049660 | 1049769.. 1050545 |
| 1E | 6 | 768 | 48 | 9 | 3581525..3582301 | 3583428..3584204 |
| 1F | 7 | 768 | - | - | 4516458..4517225 | 4518472..4519239 |
| 1H | 8 | 768 | 49 | 9 | 1782403..1783179 | 1784305.. 1784306 |
| 5A | 9 | 1157 | - | - | 273217..274373 | 273993..275149 |
| 5B | 10 | 1195 | 50 | 4 | 573811.. 575009 | 574587..575785 |
| 5D | 11 | 1195 | 51 | 4 | 687075..688273 | 687851..689049 |
| 5F | 12 | 1195 | 52 | 4 | 1394137.. 1395335 | 1396040.. 1397238 |
| 5H | 13 | 1195 | 53 | 4 | 2064257..2065455 | 2066159..2067357 |
| 5I | 14 | 1195 | 54 | 4 | 2099843..2101041 | 2101745..2102943 |
| 5K | 15 | 1195 | 55 | 4 | 2287011..2288209 | 2288915..2290113 |
| 5LO | 16 | 1125 | - | - | 3128312..3129436 | 3130216..3131340 |
| 5R | 17 | 1195 | 56 | 4 | 3363652..3364850 | 3365556..3366754 |
| 5T | 18 | 1195 | 57 | 4 | 3650133..3651331 | 3652037..3653235 |
| 5Y | 19 | 1196 | 58 | 4 | 1425696.. 1426895 | 1427599.. 1428798 |
| 4 | 20 | 1426 | 62 | 12 | 4500076..4501513 | 4502090..4503527 |
| 2A | 21 | 1331 | 65 | 5 | 380479..381814 | 381255..382590 |
| 2D | 22 | 1331 | 67 | 5 | 1466007..1467342 | 1467910..1469245 |
| 2E | 23 | 513 | - | - | 1649096.. 1649608 | 1650999..1651511 |
| 2F | 24 | 1331 | 69 | 5 | 2067039..2068374 | 2068941..2070276 |
| 2H | 25 | 1328 | - | - | 2994457..2995784 | 2996361..2997688 |
| 2I | 26 | 1331 | 70 | 5 | 3184192..3185527 | 3186096..3187431 |
| 2K | 27 | 1331 | 71 | 5 | 4496167..4497502 | 4498181..4499516 |
| 3A | 28 | 1205 | - | - | 314469..315673 | 315245..316449 |
| 3B | 29 | 1258 | 66 | 3 | 390933..392193 | 391709..392969 |
| 3C | 30 | 1258 | - | - | 566001..567258 | 566777..568034 |
| 3D | 31 | 1258 | - | - | 1093360..1094617 | 1094245..1095502 |
| 3E | 32 | 1258 | 68 | 3 | 2168266..2169526 | 2170168..2171428 |
| 30A | 33 | 1221 | - | - | 269765..270985 | 270541..271761 |
| 30B | 34 | 182 | - | - | 279155..279336 | 279931..280112 |
| 30C | 35 | 1221 | 63 | 2 | 1467393..1468615 | 1469296..1470518 |
| 30D | 36 | 1221 | - | - | 4505445..4506665 | 4507459..4508679 |
| 150 | 37 | 1443 | - | - | 3718730..3720172 | 3720633..3722075 |
| 186A | 38 | 1343 | 59 | 6 | 15382..16730 | 15382..16730 |
| 186B | 39 | 1343 | 60 | 6 | 607232..608580 | 608008..609356 |
| 186C | 40 | 1343 | 61 | 6 | 2512364..2513712 | 2514268..2515616 |
| 600 | 41 | 327 | - | - | 4506666..4506992 | 4508680..4509006 |
| 609 | 42 | 1744 | 64 | 3 | 1501255..1503001 | 1503158..1504904 |
| 911A | 43 | 335 (site 1) | - | - | 269430..269764 (site 1) | 270206..270540 (site 1) |
| | | 429 (site 2) | | - | | |
| | | | | | 270986..271414 (site 2) | 271762..272190 (site 2) |
| 911B | 44 | 334 (site 1) | - | - | 4505111..4505444 (site 1) | 4507125..4507458 (site 1) |
| | | 795 (site 2) | | - | | |
| | | | | | 4506993..4507787 (site 2) | 4509007..4509801 (site 2) |
| ISX | 45 | 272 | - | - | 279337..279608 | 280113..280384 |
| ISZ | 46 | 999 | 170 | - | 1293713..1294711 | 1294417..1295415 |

IS that overlap with bacterial genome sequence in the MG1655 strain used herein are described in **Table 2.** Note that a single IS may overlap only at one end of the IS (e.g., IS5A, ISX, IS1C, IS 609, IS2H and IS5LO, or at both ends of the IS (e.g., IS3A and IS2E). Therefore, in embodiments where IS5A, ISX, IS1C, IS609, IS2H, IS5LO, IS3A and/or IS2E are deleted in a bacterial strain, a portion of the sequence located between the two IR is retained to prevent truncating any genes in the bacterial genome sequence. The retained sequences are shown in **Table 2.**

**Table 2. MG1655 IS that overlap with non-IS open reading frame (ORF)**

| **IS** | **Non-IS ORF** | **Portion of IS sequence overlapping with non-IS ORF** | **Start Coordinate of retained IS sequence in U00096.3** | **End Coordinate of retained IS sequence in U00096.3** |
|---|---|---|---|---|
| IS5A | *ykfC* | TCAGCCGAGAAGAATTTCCCCGCTTATTCGCACCT TCC (SEQ ID NO: 208) | 273955 | 273992 |
| ISX | *yagB* | TGAATCGCCACGGGTTTAACAGACACCTCCGAGTC ATTTAA (SEQ ID NO: 209) | 280385 | 280425 |
| IS1C | *ykgS* | GGTAA (SEQ ID NO: 210) | 290634 | 290638 |
| IS3A* | *eaeH* | TCTTACCCAGCAATAG (SEQ ID NO: 211) | 315229 | 315244 |
| IS3A* | *ykgA* | TTAGGCCTGTGTCCATATTACGTGGGTAGGATCA (SEQ ID NO: 212) | 316450 | 316483 |
| IS609 | *ydcO* | TCAA (SEQ ID NO: 213) | 1504905 | 1504908 |
| IS2E* | *ydfE* | TATGGGCGCTGCTTCGCAGACAGGCAGAACTTGAT GGTATGCCTGCGATCAATGCCAAACGTGTTTACCG GATCATGCGCCAGAATGCGCTGTTGCTTGAGCGAA AACCTGCTGTACCGCCATCGAAACGGGCACATACA GGCAGAGTGGCCGTGA (SEQ ID NO: 214) | 1650843 | 16550998 |
| IS2E* | *intQ* | GTGATAACAGATGTCTGGAAATATAGGGGCAAATC CA (SEQ ID NO: 215) | 1651512 | 1651548 |
| IS2H | *ygeP* | CTA (SEQ ID NO: 216) | 2997689 | 2997691 |
| IS5LO | *yghO* | GGAAGGTGCGAACAAGTCCCTGATATGAGATCATG TTTGTCATCTGGAGCCATAGAACAGGGTTCATCAT (SEQ ID NO: 217) | 3130146 | 3130215 |

| | | | | |
|---|---|---|---|---|
| **IS3A and IS2E overlap with flanking coding sequence at both of their respective ends.* | | | | |

Split IS sequences in MG1655 are IS911A (split by IS30A) and IS911B (split by IS30D and IS600).

In addition, the ISFinder database (www-is.biotoul.fr) contains the sequences of various members of the IS families.

### c. Modified bacterial strains

Provided is a modified bacterial strain lacking an IS. The IS may belong to one of the following families or sub-families: insL-2, IS1, IS2, IS3, IS4, IS5, IS6, IS629, IS50, IS21, IS200, IS256, IS621, IS630, IS257, IS15, IS26, IS10, IS30, IS150, IS186, IS600, IS609, IS911, ISX and/or ISZ. In embodiments, the IS may be a homologue of one or more IS elements of an IS family or sub-family. InsL-2 is part of the IS186 subfamily. In some embodiments, the IS may belong to IS1 or IS5 families, as these are the most active IS family in MG1655 bacterial strains. In embodiments, the IS may be insL-2 or a homologue thereof.

The IS sequence may vary between strains and position of the insertion site. Therefore, in embodiments, the IS is, or has at least 60, 65, 70, 75, 80, 85, 90, 91, 92, 93, 94, 95, 96, 97, 98, 99% sequence identity to, insL-2 (SEQ ID NO: 1), IS1A (SEQ ID NO: 2), IS1B (SEQ ID NO: 3), IS1C (SEQ ID NO:4), IS1D (SEQ ID NO: 5), IS1E (SEQ ID NO: 6), IS1F (SEQ ID NO: 7), IS1H (SEQ ID NO: 8), IS5A (SEQ ID NO: 9), IS5B (SEQ ID NO: 10), IS5D (SEQ ID NO: 11), IS5F (SEQ ID NO: 12), IS5H (SEQ ID NO: 13), IS5I (SEQ ID NO: 14), IS5K (SEQ ID NO: 15), IS5LO (SEQ ID NO: 16), IS5R (SEQ ID NO: 17), IS5T (SEQ ID NO: 18), IS5Y (SEQ ID NO: 19), IS4 (SEQ ID NO: 20), IS2A (SEQ ID NO: 21), IS2D (SEQ ID NO: 22), IS2E (SEQ ID NO: 23), IS2F (SEQ ID NO: 24), IS2H (SEQ ID NO: 25), IS2I (SEQ ID NO: 26), IS2K (SEQ ID NO: 27), IS3A (SEQ ID NO: 28), IS3B (SEQ ID NO: 29), IS3C (SEQ ID NO: 30), IS3D (SEQ ID NO: 31), IS3E (SEQ ID NO: 32), IS30A (SEQ ID NO: 33), IS30B (SEQ ID NO: 34), IS30C (SEQ ID NO: 35), IS30D (SEQ ID NO: 36), IS150 (SEQ ID NO: 37), IS186A (SEQ ID NO: 38), IS186B (SEQ ID NO: 39), IS186C (SEQ ID NO: 40), IS600 (SEQ ID NO: 41), IS609 (SEQ ID NO: 42), IS911A (SEQ ID NO: 43), IS911B (SEQ ID NO: 44), ISX (SEQ ID NO: 45), ISZ (SEQ ID NO: 46), IS629 (SEQ ID NO: 161), IS10 (SEQ ID NO: 162), IS50 (SEQ ID NO: 163), IS257 (SEQ ID NO: 164), IS15 (SEQ ID NO: 165), IS26 (SEQ ID NO: 166), IS21 (SEQ ID NO: 202), IS200 (SEQ ID NO: 203), IS256 (SEQ ID NO: 204), IS621 (SEQ ID NO: 205) and/or IS630 (SEQ ID NO: 206), and/or a homologue thereof. In embodiments the modified bacterial strain also lacks a corresponding TSD.

Therefore, in embodiments, the IS may be a homologue of one or more IS elements and associated TSD of an IS family or sub-family. In examples, the IS is, or has at least 60, 65, 70, 75, 80, 85, 90, 91, 92, 93, 94, 95, 96, 97, 98, 99% sequence identity to, insL-2 & TSD (SEQ ID NO: 218), IS1D & TSD (SEQ ID NO: 47), IS1E + TSD (SEQ ID NO: 48), IS1H + TSD (SEQ ID NO: 49), IS5B + TSD (SEQ ID NO: 50), IS5D + TSD (SEQ ID NO: 51), IS5F + TSD (SEQ ID NO: 52), IS5H + TSD (SEQ ID NO:53), IS5I + TSD (SEQ ID NO: 54), IS5K + TSD (SEQ ID NO: 55), IS5T + TSD (SEQ ID NO: 56), IS5Y + TSD (SEQ ID NO: 58), IS186A + TSD (SEQ ID NO: 59), IS186B + TSD (SEQ ID NO: 60), IS186C + TSD (SEQ ID NO: 61), IS4 + TSD (SEQ ID NO: 62), IS30C + TSD (SEQ ID NO: 63), IS609 + TSD (SEQ ID NO: 64), IS2A + TSD (SEQ ID NO: 65), IS3B + TSD (SEQ ID NO: 66), IS2D + TSD (SEQ ID NO: 67), IS3E + TSD (SEQ ID NO: 68), IS2F + TSD (SEQ ID NO: 69), IS2I + TSD (SEQ ID NO: 70), IS2K + TSD (SEQ ID NO: 71), ISZ + TSD (SEQ ID NO: 170) or insL-2 + TSD (SEQ ID NO: 74).

The modified bacterial strain may have at deletions of at least 2, 3, 5, 10, 15, 20, 30, 40 or 45 IS present in the unmodified bacterial strain. The modified bacterial strain may have deletion of at least 2, 5, 10, 15, 20, 25, 30, 35, 40, 45, 50, 55, 60, 65, 70, 75, 80, 85, 90, or 95%, or 100%, of the IS elements present in the unmodified bacterial strain. The modified bacterial strain may have all IS deleted. In other words, the modified bacterial strain may not contain any IS.

The modified bacterial strain may retain a genome size of at least 98, 98.1, 98.2, 98.3, 98.4, 98.5, 98.6, 98.7, 98.8, 98.85, 98.9, 98.95, 99, 99.1, 99.2, 99.3, 99.4, 99.5, 99.6, 99.7, 99.8, or 99.9% compared to the genome size of the unmodified bacterial strain. In embodiments, the modified bacterial strains retain at least 98, 98.1, 98.2, 98.3, 98.4, 98.5, 98.6, 98.7, 98.8, 98.85, 98.9, 98.95, 99, 99.1, 99.2, 99.3, 99.4, 99.5, 99.6, 99.7, 99.8, or 99.9%, or 100%, of non-IS genome sequence.

In embodiments, the deleted IS may be replaced with a stop codon and/or frameshift mutation as described herein. In embodiments, the IS replaced by a stop codon and/or frameshift mutation has at least 60, 65, 70, 75, 80, 85, 90, 91, 92, 93, 94, 95, 96, 97, 98, 99% sequence identity to, IS1E (SEQ ID NO: 6), IS1H (SEQ ID NO: 8), IS2A (SEQ ID NO: 21), IS2D (SEQ ID NO: 22), IS2F (SEQ ID NO: 24), IS2I (SEQ ID NO: 26), IS3B (SEQ ID NO: 29), IS3C (SEQ ID NO: 30), IS3E (SEQ ID NO: 32), IS5B (SEQ ID NO: 10), IS5I (SEQ ID NO: 14), IS5K (SEQ ID NO: 15), IS5R (SEQ ID NO: 17), IS5T (SEQ ID NO: 18), and/or IS5Y (SEQ ID NO: 19), and/or a homologue thereof. In some embodiments, the IS replaced by a stop codon and/or frameshift mutation may have at least 60, 65, 70, 75, 80, 85, 90, 91, 92, 93, 94, 95, 96, 97, 98, 99% sequence identity to IS5I (SEQ ID NO: 14), and/or a homologue thereof.

### d. Method of producing modified bacterial strains

Provided herein is a method for producing a modified bacterial strain lacking an insertion sequence (IS) as described herein, the method comprising: (a) providing a bacterial strain comprising an IS flanked by non-IS nucleotide residues; and (b) specifically deleting the IS in the bacterial strain without deleting the non-IS nucleotide residues flanking the IS.

To delete more than one IS, the method may be repeated to delete at least 2, 3, 5, 10, 20, 30, 40, 45 or all IS present in the unmodified bacterial strain. Therefore, bacterial strain (a) may have already been modified to delete one or more IS. A TSD adjacent to the IS may be deleted with the IS.

The IS deleted in (b) may belong to one of the following families or sub-families: insL-2, IS1, IS2, IS3, IS4, IS5, IS6, IS629, IS50, IS21, IS200, IS256, IS621, IS630, IS257, IS15, IS26, IS10, IS30, IS150, IS186, IS600, IS609, IS911, ISX and/or ISZ. The IS may be a homologue of one or more IS elements of an IS family or sub-family. In embodiments, the IS is, or has at least 60, 65, 70, 75, 80, 85, 90, 91, 92, 93, 94, 95, 96, 97, 98, 99% sequence identity to, insL-2 (SEQ ID NO: 1), IS1A (SEQ ID NO: 2), IS1B (SEQ ID NO: 3), IS1C (SEQ ID NO:4), IS1D (SEQ ID NO: 5), IS1E (SEQ ID NO: 6), IS1F (SEQ ID NO: 7), IS1H (SEQ ID NO: 8), IS5A (SEQ ID NO: 9), IS5B (SEQ ID NO: 10), IS5D (SEQ ID NO: 11), IS5F (SEQ ID NO: 12), IS5H (SEQ ID NO: 13), IS5I (SEQ ID NO: 14), IS5K (SEQ ID NO: 15), IS5LO (SEQ ID NO: 16), IS5R (SEQ ID NO: 17), IS5T (SEQ ID NO: 18), IS5Y (SEQ ID NO: 19), IS4 (SEQ ID NO: 20), IS2A (SEQ ID NO: 21), IS2D (SEQ ID NO: 22), IS2E (SEQ ID NO: 23), IS2F (SEQ ID NO: 24), IS2H (SEQ ID NO: 25), IS2I (SEQ ID NO: 26), IS2K (SEQ ID NO: 27), IS3A (SEQ ID NO: 28), IS3B (SEQ ID NO: 29), IS3C (SEQ ID NO: 30), IS3D (SEQ ID NO: 31), IS3E (SEQ ID NO: 32), IS30A (SEQ ID NO: 33), IS30B (SEQ ID NO: 34), IS30C (SEQ ID NO: 35), IS30D (SEQ ID NO: 36), IS150 (SEQ ID NO: 37), IS186A (SEQ ID NO: 38), IS186B (SEQ ID NO: 39), IS186C (SEQ ID NO: 40), IS600 (SEQ ID NO: 41), IS609 (SEQ ID NO: 42), IS911A (SEQ ID NO: 43), IS911B (SEQ ID NO: 44), ISX (SEQ ID NO: 45), ISZ (SEQ ID NO: 46), IS629 (SEQ ID NO: 161), IS10 (SEQ ID NO: 162), IS50 (SEQ ID NO: 163), IS257 (SEQ ID NO: 164), IS15 (SEQ ID NO: 165), IS26 (SEQ ID NO: 166), IS21 (SEQ ID NO: 202), IS200 (SEQ ID NO: 203), IS256 (SEQ ID NO: 204), IS621 (SEQ ID NO: 205) and/or IS630 (SEQ ID NO: 206), and/or a homologue thereof.

In embodiments, an IS and corresponding TSD is deleted. In these embodiments, the IS is, or has at least 60, 65, 70, 75, 80, 85, 90, 91, 92, 93, 94, 95, 96, 97, 98, 99% sequence identity to, insL-2 & TSD (SEQ ID NO: 218), IS1D & TSD (SEQ ID NO: 47), IS1E + TSD (SEQ ID NO: 48), IS1H + TSD (SEQ ID NO: 49), IS5B + TSD (SEQ ID NO: 50), IS5D + TSD (SEQ ID NO: 51), IS5F + TSD (SEQ ID NO: 52), IS5H + TSD (SEQ ID NO:53), IS5I + TSD (SEQ ID NO: 54), IS5K + TSD (SEQ ID NO: 55), IS5T + TSD (SEQ ID NO: 56), IS5Y + TSD (SEQ ID NO: 58), IS186A + TSD (SEQ ID NO: 59), IS186B + TSD (SEQ ID NO: 60), IS186C + TSD (SEQ ID NO: 61), IS4 + TSD (SEQ ID NO: 62), IS30C + TSD (SEQ ID NO: 63), IS609 + TSD (SEQ ID NO: 64), IS2A + TSD (SEQ ID NO: 65), IS3B + TSD (SEQ ID NO: 66), IS2D + TSD (SEQ ID NO: 67), IS3E + TSD (SEQ ID NO: 68), IS2F + TSD (SEQ ID NO: 69), IS2I + TSD (SEQ ID NO: 70), IS2K + TSD (SEQ ID NO: 71), ISZ + TSD (SEQ ID NO: 170) or INSL-2 + TSD (SEQ ID NO: 74).

In embodiments, the deleted IS may be replaced with a stop codon and/or frameshift mutation. In embodiments, the deleted IS being replaced by a stop codon and/or frameshift mutation may belong to one of the following families or sub-families: insL-2, IS1, IS2, IS3, IS4, IS5, IS6, IS629, IS50, IS21, IS200, IS256, IS621, IS630, IS257, IS15, IS26, IS10, IS30, IS150, IS186, IS600, IS609, IS911, ISX and/or ISZ. The IS may be a homologue of one or more IS elements of an IS family or sub-family. In embodiments, the IS is, or has at least 60, 65, 70, 75, 80, 85, 90, 91, 92, 93, 94, 95, 96, 97, 98, 99% sequence identity to, insL-2 (SEQ ID NO: 1), IS1A (SEQ ID NO: 2), IS1B (SEQ ID NO: 3), IS1C (SEQ ID NO:4), IS1D (SEQ ID NO: 5), IS1E (SEQ ID NO: 6), IS1F (SEQ ID NO: 7), IS1H (SEQ ID NO: 8), IS5A (SEQ ID NO: 9), IS5B (SEQ ID NO: 10), IS5D (SEQ ID NO: 11), IS5F (SEQ ID NO: 12), IS5H (SEQ ID NO: 13), IS5I (SEQ ID NO: 14), IS5K (SEQ ID NO: 15), IS5LO (SEQ ID NO: 16), IS5R (SEQ ID NO: 17), IS5T (SEQ ID NO: 18), IS5Y (SEQ ID NO: 19), IS4 (SEQ ID NO: 20), IS2A (SEQ ID NO: 21), IS2D (SEQ ID NO: 22), IS2E (SEQ ID NO: 23), IS2F (SEQ ID NO: 24), IS2H (SEQ ID NO: 25), IS2I (SEQ ID NO: 26), IS2K (SEQ ID NO: 27), IS3A (SEQ ID NO: 28), IS3B (SEQ ID NO: 29), IS3C (SEQ ID NO: 30), IS3D (SEQ ID NO: 31), IS3E (SEQ ID NO: 32), IS30A (SEQ ID NO: 33), IS30B (SEQ ID NO: 34), IS30C (SEQ ID NO: 35), IS30D (SEQ ID NO: 36), IS150 (SEQ ID NO: 37), IS186A (SEQ ID NO: 38), IS186B (SEQ ID NO: 39), IS186C (SEQ ID NO: 40), IS600 (SEQ ID NO: 41), IS609 (SEQ ID NO: 42), IS911A (SEQ ID NO: 43), IS911B (SEQ ID NO: 44), ISX (SEQ ID NO: 45), ISZ (SEQ ID NO: 46), IS629 (SEQ ID NO: 161), IS10 (SEQ ID NO: 162), IS50 (SEQ ID NO: 163), IS257 (SEQ ID NO: 164), IS15 (SEQ ID NO: 165), IS26 (SEQ ID NO: 166), IS21 (SEQ ID NO: 202), IS200 (SEQ ID NO: 203), IS256 (SEQ ID NO: 204), IS621 (SEQ ID NO: 205) and/or IS630 (SEQ ID NO: 206), and/or a homologue thereof. In some embodiments, the IS is, or has at least 60, 65, 70, 75, 80, 85, 90, 91, 92, 93, 94, 95, 96, 97, 98, 99% sequence identity to, IS1H (SEQ ID NO: 8), IS2A (SEQ ID NO: 21), IS2D (SEQ ID NO: 22), IS2F (SEQ ID NO: 24), IS2I (SEQ ID NO: 26), IS3B (SEQ ID NO: 29), IS3C (SEQ ID NO: 30), IS3E (SEQ ID NO: 32), IS5B (SEQ ID NO: 10), IS5I (SEQ ID NO: 14), IS5K (SEQ ID NO: 15), IS5R (SEQ ID NO: 17), IS5T (SEQ ID NO: 18), and/or IS5Y (SEQ ID NO: 19), and/or a homologue thereof. In some embodiments, the IS replaced by a stop codon and/or frameshift mutation may have at least 60, 65, 70, 75, 80, 85, 90, 91, 92, 93, 94, 95, 96, 97, 98, 99% sequence identity to IS5I (SEQ ID NO: 14), and/or a homologue thereof.

The IS may be deleted in order of their activity. i.e., with the most active IS family or subfamily being deleted first, with the next most active IS family or subfamily being deleted, and so on. Methods for measuring activity of an IS are known in the art and are described, for example, in Lee et al. (2016. Insertion Sequence-caused large-scale rearrangements in the Genome of Escherichia Coli. *Nucleic Acids Research,* gw647, https://doi.org/10.1093/nar/gkw647). Therefore, the IS1 and/or IS5 family/subfamily may be deleted before other IS families/subfamilies are deleted from the modified bacterial strain. This is particularly preferable in MG1655 strains. A recommended order of deletion based on the activity of IS is as follows: 1) IS1 family, 2) IS5 family, 3) IS186 subfamily (part of the IS4 family), 4) IS4 subfamily (part of the IS4 family), 5) IS2 subfamily (part of the IS3 family), 6) IS3 subfamily (part of the IS3 family), 7) IS150 subfamily and 8) IS609 subfamily.

The resulting modified bacterial strain may be as described herein.

The modified bacterial strains described herein may alternatively be made using any genetic editing method known in the art that creates precise deletions of target nucleotides (i.e., so that no additional flanking sequences are unintentionally deleted, and where the full target nucleotide is deleted without leaving any residual sequence), and where no additional exogenous sequence is inserted.

In embodiments, the modified bacterial strains described herein may be made using a lambda Red recombineering technique, such as the method described in Nävsall (2017. Direct and Inverted Repeat stimulated excision (DIRex): Simple, single-step, and scar-free mutagenesis of bacterial genes. *PLoS ONE* 12(8): e0184126. https://doi.org/10.1371/journal.pone.0184126).

In embodiment, an IS is deleted using a first and second partial insertion cassette. The first partial insertion cassette may comprise a first (long) polynucleotide sequence (referred to herein as H1-a) having at least 10, 15, 17, 20, 22, 25, 27, 28, 29, or 30 nucleotide residues. The H1-a sequence may be between 10-100, 10-90, 10-80, 10-70, 10-60, 10-50, 10-40, 15-100, 15-90, 15-80, 15-70, 15-60, 15-50, 15-40, 20-100, 20-90, 20-80, 20-70, 20-60, 20-50, 20-40, 20-35, 25-100, 25-90, 25-80, 25-70, 25-60, 25-50, 25-40, 25-35, 30-100, 30-90, 30-80, 30-70, 30-60, 30-50, or 35-45 nucleotide residues. The H1-a sequence may have at least 85, 87, 90, 91, 92, 93, 94, 95, 96, 97, 99% sequence identity, or is 100% identical, to the nucleotide residues that are immediately 5' to the IS in the bacterial strain (H1 in **Figure 1**). The nucleotide residues immediately 5' to the IS may be non-IS flanking nucleotide residues. The first partial insertion cassette may also comprise a second (short) nucleotide sequence (referred to herein as H2-b) having 5-50, 5-45, 5-40, 5-35, 5-30, 8-50, 8-45, 8-40, 8-35, 8-30, 10-50, 10-45, 10-40, 10-35, 10-30, 12-50, 12-45, 12-40, 12-35, 12-30, 15-50, 15-45, 15-40, 15-35, 15-30, 20-50, 20-45, 20-40, 20-35, 20-30 or 25-35 nucleotide residues. The H2-b nucleotide sequence may have at least 85, 87, 90, 91, 92, 93, 94, 95, 96, 97, 99% sequence identity, or may be 100% identical, to the nucleotide residues that are immediately 3' to the IS in the bacterial strain (H2 in **Figure 1**). The nucleotide residues immediately 3' to the IS may be non-IS flanking nucleotide residues. The first partial insertion cassette may also comprise a truncated nucleotide sequence encoding part of a selection marker. In embodiments, the first partial insertion cassette may comprise, consist or consist essentially of a sequential 5' to 3' sequence of: H2-b, H1-a and a truncated selection marker sequence. In embodiments, the first partial insertion cassette may comprise, consist or consist essentially of H2-b, H1-a, a truncated selection marker, and *amilCP.*

The second partial insertion cassette may comprise a first (long) polynucleotide sequence (referred to herein as H2-a) having at least 10, 15, 17, 20, 22, 25, 27, 28, 29, or 30 nucleotide residues. The H2-a sequence may be between 10-100, 10-90, 10-80, 10-70, 10-60, 10-50, 10-40, 15-100, 15-90, 15-80, 15-70, 15-60, 15-50, 15-40, 20-100, 20-90, 20-80, 20-70, 20-60, 20-50, 20-40, 20-35, 25-100, 25-90, 25-80, 25-70, 25-60, 25-50, 25-40, 25-35, 30-100, 30-90, 30-80, 30-70, 30-60, 30-50, or 35-45 nucleotide residues. The H2-a sequence may have at least 85, 87, 90, 91, 92, 93, 94, 95, 96, 97, 99% sequence identity, or is 100% identical, to the nucleotide residues that are immediately 3' to the IS in the bacterial strain (H2 in **Figure 1**). The nucleotide residues immediately 3' to the IS may be non-IS flanking nucleotide residues. The second partial insertion cassette may also comprise a second (short) polynucleotide sequence (referred to herein as H1-b) having 5-50, 5-45, 5-40, 5-35, 5-30, 8-50, 8-45, 8-40, 8-35, 8-30, 10-50, 10-45, 10-40, 10-35, 10-30, 12-50, 12-45, 12-40, 12-35, 12-30, 15-50, 15-45, 15-40, 15-35, 15-30, 20-50, 20-45, 20-40, 20-35, 20-30 or 25-35 nucleotide residues. The H1-b polynucleotide sequence may have at least 85, 87, 90, 91, 92, 93, 94, 95, 96, 97, 99% sequence identity, or may be 100% identical, to the nucleotide residues that are immediately 5' to the IS in the bacterial strain (H1 in **Figure 1**). The nucleotide residues immediately 5' to the IS may be non-IS flanking nucleotide residues. The second partial insertion cassette may also comprise a truncated polynucleotide sequence comprising, consisting, or consisting essentially of the remaining nucleotide sequence of the truncated selection marker present in the first partial insertion cassette. In other words, the first and second partial insertion cassette each comprise part corresponding (or respective) parts of a selection marker gene, such that the first and second partial insertion cassette together make up a whole selection marker gene. The truncated polynucleotide sequence of the first and second partial insertion cassette may contain at least 50, 100, 125, 150, 175, 200, 225 or 250 identical consecutive nucleotide residues. The truncated polynucleotide sequence of the first and second partial insertion cassette may contain 50-500, 50-450, 50-400, 50-350, 100-500, 100-450, 100-400, 100-350, 150-500, 150-450, 150-400, 150-350, 200-500, 200-450, 200-400, 200-350 or 250-350 identical consecutive nucleotide residues. In other words, the truncated polynucleotide sequence of the first and second partial insertion cassette may have a degree of overlap, such that spontaneous recombination is promoted between the overlapping polynucleotide sequence, so that the selection marker can be expressed. In embodiments, the second partial insertion cassette may comprise, consist or consist essentially of a sequential 5' to 3' sequence of: the truncated selection marker sequence, H1-b and H2-a. In embodiments, the second partial insertion cassette may comprise, consist or consist essentially of the truncated selection marker, *sacB, amilCP* inverted relative to the first partial cassette, H1-b and H2-a.

The truncation may be such that the selection gene consists of less than 90, 80, 70, 60, 50, 40, 30, 20 or 10% of the complete selection gene sequence. Selection markers are standard in the art and may be a positive selection marker in the art, such as an antibiotic resistance gene (e.g., *kan* for kanamycin resistance, *cat* for chloramphenicol resistance, neo for neomycin resistance). The first partial insertion cassette may comprise further polynucleotide sequences encoding further selection markers, for example, *amilCP.*

In embodiments, a first and second partial insertion cassette is required for each IS to be deleted. However, in embodiments where two or more IS overlap (i.e., are split, such as IS911A and IS911B), a single first and second partial insertion cassette may be used to delete the two or more overlapping IS. Therefore, reference to the non-IS nucleotide residues immediately 5' or 3' to the IS in the description of the first and second partial insertion cassette above can be understood as referring to the non-IS sequence immediately 5' or 3' to the two or more IS.

In embodiments, the first and/or second partial insertion cassette may comprise a stop codon and/or frameshift mutation. The stop codon and/or frameshift mutation may be positioned between H1-a and H2-b in the partial cassette and/or H1-b and H2-a. Positioning the stop codon and/or frameshift mutation in both partial cassettes ensures presence of the mutation in derived clones after cassette excision. Positioning in either partial cassette requires screening of derived clones for maintenance of the mutation after segregation. The stop codon and/or frameshift mutation may be in the form of a double stop codon, or a double stop codon and frameshift mutation. In examples, this may have the sequence set out in SEQ ID NOs: 72 (CCTATTA) or 73 (TAATAGG). SEQ ID NO: 73 may be used in the Fp1 oligomer where the target IS is located on a forward DNA strand in the bacterial genome, and SEQ ID NO: 72 may be used in the Fp1 oligomer where the target IS is located on a reverse DNA strand in the bacterial genome. Where SEQ ID NO: 73 is used in the Fp1 oligomer, SEQ ID NO: 72 is used in the Rp1 oligomer. Where SEQ ID NO: 72 is used in the Fp1 oligomer, SEQ ID NO: 73 is used in the Rp1 oligomer. The frameshift mutation allows the stop codons to be in the reading frame.

The first and second partial insertion cassettes can be generated using standard techniques in the art, such as polymerase chain reaction (PCR). However, the skilled person understands that this method may be modified for alternative nucleotide amplification techniques, or polynucleotide synthesis methods that are known in the art.

A separate PCR reaction may be performed for each partial insertion cassette. The PCR reaction for the first and second partial insertion cassette may use a DNA template having at least 50, 55, 60, 65, 70, 75, 80, 85, 90, 95, or 99% sequence identity, or 100% sequence identity, to *Acatsac1* (SEQ ID NO: 167), *Akansac1* (SEQ ID NO: 169), *Acatsac3* (SEQ ID NO: 168) or *Akansac3* (SEQ ID NO: 170). The skilled person appreciates that the template may be altered to include alternative selection markers as described herein. For embodiments requiring complete deletion of an IS, the PCR reaction may comprise a DNA template as described herein and two oligomers having at least 70, 75, 80, 85, 90, 95, or 99%, or 100%, sequence identity to any of SEQ ID NOs: 75-160. For embodiments requiring replacement of an IS with a stop codon and/or frameshift mutation, the PCR reaction may comprise a DNA template as described herein and two oligomers having at least 70, 75, 80, 85, 90, 95, or 99%, or 100%, sequence identity to any of SEQ ID NOs: 171-200.

To achieve deletion of an IS in one embodiment, a first and second partial insertion cassette as described herein is introduced to the bacterial strain. Introduction may be by transformation or any other standard technique in the art. Methods of transforming bacteria are routine in the art, and include, for example, electroporation or heat shock. The bacterial strain may be made 'competent' before transformation. Representative methods of making the bacteria competent may be found in U.S. Pat. No. 4,981,797 and U.S. Patent Publication No. 20050032225, which are hereby incorporated by reference.

To delete the insertion sequence, three recombination events occur (dotted lines in Figure 1A) in the bacterium. The three recombination events are: 1) a recombination event between the H1-a sequence in the first partial insertion cassette and the corresponding H1-a sequence in the bacterial genome, 2) a recombination event between the H2-a sequence in the second partial insertion cassette and the corresponding H2-a sequence in the bacterial genome, and 3) a recombination event between the truncated, overlapping *cat* (~277 nucleotide overlap) or *kan* (~304 nucleotides overlap) selection gene sequences in the first and second partial insertion cassettes. This produces a metastable intermediate bacterium shown in **Figure 1B** where the IS is replaced with the sequences of the first and second partial insertion cassettes. The metastable intermediate may undergo a further, single recombination event (indicated by dotted lines in **Figure 1B**) between the duplicated homology regions (i.e., between the H1-a-H2-b sequence and the H1-b-H2-a sequence) that removes the cassette as shown in **Figure 1C****.**

Recombination may be induced using routine methods in the art, for example, using lambda Red as recombineering e.g., as described in Marinelli et al. (2012. Recombineering. Bacteriophage, 2(1): 5-14) or Nävsall (2017). Alternatively, recombination may be through spontaneous, homologous recombination.

Routine sequencing methods in the art can be used to determine if deletion of the IS, insertion of a stop codon and/or frameshift mutation where performed, or deletion of the stop codon and/or frameshift mutation where performed, was successful i.e., the IS was deleted, identify any additionally inserted sequence, and identify if any non-IS flanking sequence was deleted. This may be sequencing such as Sanger sequencing, Oxford Nanopore Sequencing, or whole genome sequencing (e.g., as described in Gautam et al. (2019. A step-by-step beginner's protocol for whole genome sequencing of human bacterial pathogens. J Biol Methods, 6(1): e110), or using IS detection kits (e.g., Scarab Genomics, IS-1109-10).

Where a stop codon and/or frameshift mutation replaces an IS, the stop codon and/or frameshift mutation may then be deleted. This may be performed using any standard techniques known in the art, or the method described herein. If using the method described herein, a first and second partial insertion cassette are used that do not comprise the stop codon and/or frameshift mutation.

To produce combinations of two or more IS deletions, the method described herein can be repeated for each target IS. However, deletion of two or more IS in a single bacterial strain can also be achieved by generating multiple bacterial strains comprising a metastable intermediate with a single IS deletion in parallel (e.g., where each strain has a different IS deletion). The metastable intermediates can then each be introduced into a recipient bacterial strain, for example using known transduction techniques, such as generalized phage transduction. In embodiments the metastable intermediates are introduced consecutively into the recipient bacterial strain.

Spontaneous mutations that may occur during the method described herein can be tested for using routine sequencing methods, e.g., as described herein. Any identified spontaneous mutations identified can be reverted (modified back to the original sequence) using any known genetic editing technique, such as lambda Red recombineering, CRISPR-Cas9 e.g., as described in WO 2016/205623 (incorporated by reference in its entirety) or the DIRex method described by Nävsall (2017).

In embodiments, the bacterial strain may be further modified to alter one or more genes of interest in the bacterial strain. The gene of interest may encode a protease e.g., *Ion* or *ompT,* or *hsdSB, hsdRMS, dcm, trxB, gor, recA, lacR, endA, luxS, acrAB,* or *acrR.* Methods of modifying genes in bacteria are known in the art and may include lambda Red recombineering, CRISPR-Cas9 e.g., as described in WO 2016/205623 or the DIRex method described by Nävsall (2017. The gene of interest may be modified to increase, decrease or inhibit expression of the gene, or to alter the structure or functionality of any RNA or polypeptide encoded by the gene.

To reduce the mutation rate in bacterial strains, where *Ion* is present in the bacterial strain, the method described herein may be used to delete all IS in the bacterial strain. Alternatively, in some examples, the method may be used to produce a bacterial strain where the *Ion* gene is deleted, or where the *Ion* gene or its promoter sequence is modified to prevent or reduce expression of Lon protease, and one or more IS are retained in the modified bacterial strain. In embodiments, the IS is an IS present in a promoter of *Ion* protease in the bacterial strain (a), an IS present in a coding sequence of *Ion* protease and/or an IS present in a *wbbL* gene in the bacterial strain (a).

In some examples, the method described herein may be used to replace an IS that is present in a *wbbL* gene in a bacterial strain with a stop codon and/or frameshift mutation, preferably IS5I in the MG1655 strain, preferably with SEQ ID NO: 72. This retains phage susceptibility in the bacterial strain which is required for some generalised transduction techniques. However, the stop codon and/or frameshift mutation in the *wbbL* gene can then be deleted. This confers phage resistance to the bacterial strain which may be beneficial for biotechnological processes. In some examples, an IS that is present in a *wbbL* gene may be deleted to confer phage resistance to the bacterial strain.

### EXAMPLES

### Example 1: production of modified bacterial strains

### Introduction

*E. coli* strain MG1655 has IS elements that are very active, and therefore may be different compared to a reference strain. Thus, the exact copy numbers and chromosomal locations need to be verified in each strain copy. This was performed by whole-genome sequencing the wild type strain (Oxford Nanopore Technologies), *de-novo* assembly of the genome into a single closed contig, improvement of sequence accuracy with additional Illumina sequencing data and then running an IS-detection tool ISEscan on the entire genome (Xie & Tang 2017, Bioinformatics 33(21), 3340-3347, https://pubmed.ncbi.nlm.nih.gov/29077810/). The hits were then manually checked to ensure accuracy using the following inclusion criteria: (i) Experimental documentation of element insertion, excision, or recombination between element copies, (ii) Annotation of the gene as IS element, e.g. *tnpA1, tnpA2, insZ* on NCBI and/or EcoCyc (https://ecocyc.org) databases, (iii) Localization within an annotated ORF or gene, indicating a recent insertion event. The exclusion criteria to identify false-positive IS annotations are: (i) presence of a annotation for a specific function, e.g. gene names *dna* (DNA replication) or *rpn* (recombination promoting nuclease), (ii) Lack of element copies in the genome (ncopy4is = 1), Short length (isLen shorter 700 nt), Non-canonical IS classification (e.g., ISNCY = IS not classified yet; ISAS1 = *Aeromonas salmonocida;* IS481 = common in *Bordetella pertussis*), Truncation (type = p) and poor TIR prediction (score4tir, value is low). This pipeline allows the correct annotation also of split IS elements, e.g. IS911A which is split into two parts by the insertion of IS30A in MG1655.

The reference genome of wild type MG1655 (EMBL: U00096.3, SEQ ID NO: 201) contains 47 IS elements. The MG1655 wild type strain used herein lacked two of these IS elements (IS1I and IS5U). The MG1655 wild type strain used herein therefore has 45 of the IS elements of reference sequence U00096.3 (SEQ ID NO: 201) of which 44 IS were in the same location as in the reference sequence. One IS (IS1H) in the U00096.3 reference sequence (SEQ ID NO: 201) at position 1978495.. 1979270 was located in a different position (1784305..1784306 in SEQ ID NO: 201) in the MG1655 wild type strain used herein and is now positioned in *fadK.*

Target site duplication (TSD) sequences surrounding the IS were also identified by comparing the DNA sequence immediately flanking the terminal inverted repeats according to known characteristics of TSDs for the respective IS families (Siguier et al. 2014, FEMS Microbiol Rev, 38(5): 865-891 https://doi.org/10.1111/1574-6976.12067). For example, insertions of IS1 are known to be flanked by 8-9 bp TSDs, and insertions of IS5 by shorter 4 bp TSDs (Siguier et al. 2014).

### Methods

### Bacterial strains

Strains of Escherichia coli MG1655 were generated with various deletions of insertion sequences as shown in **Table 3** below.

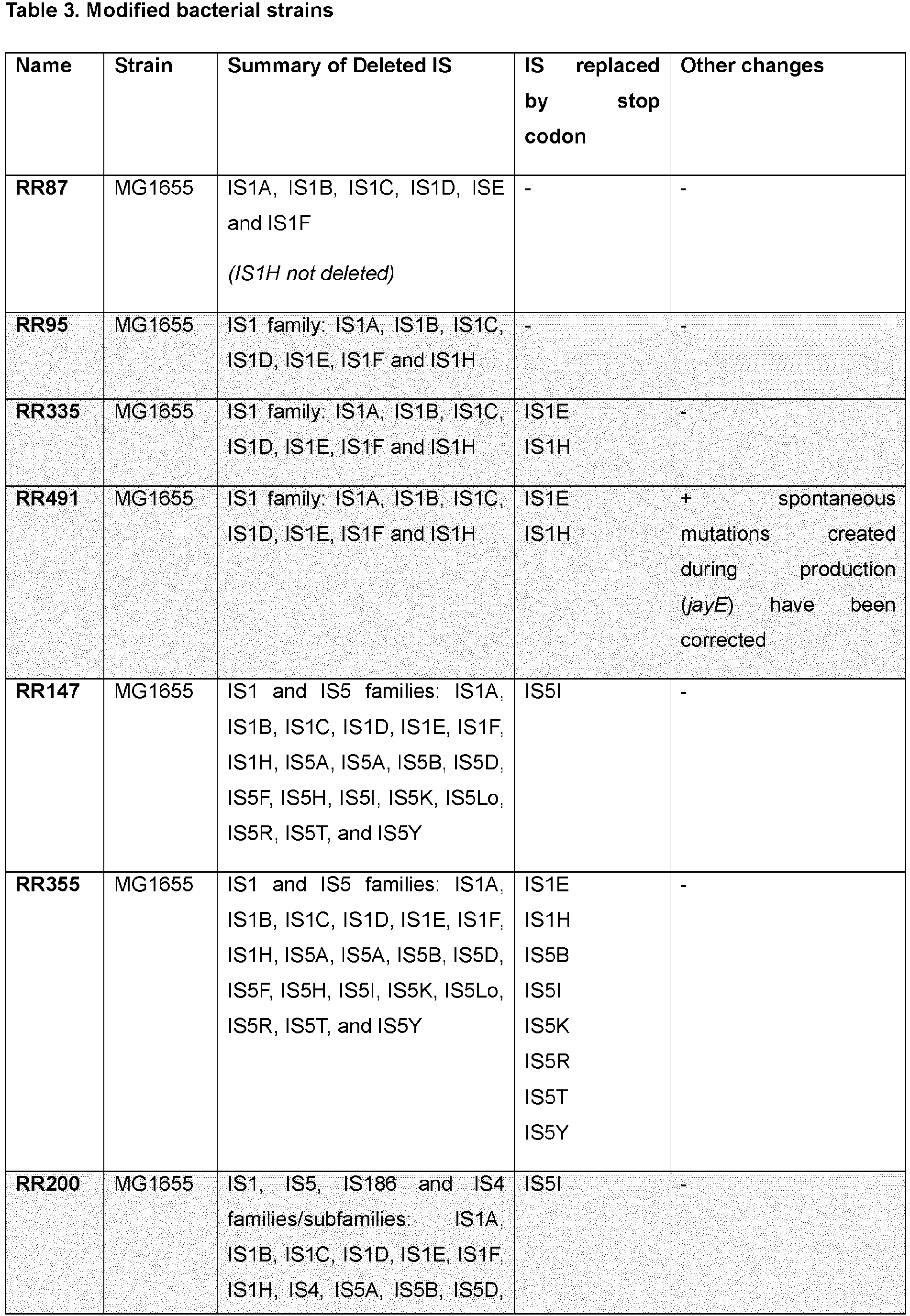

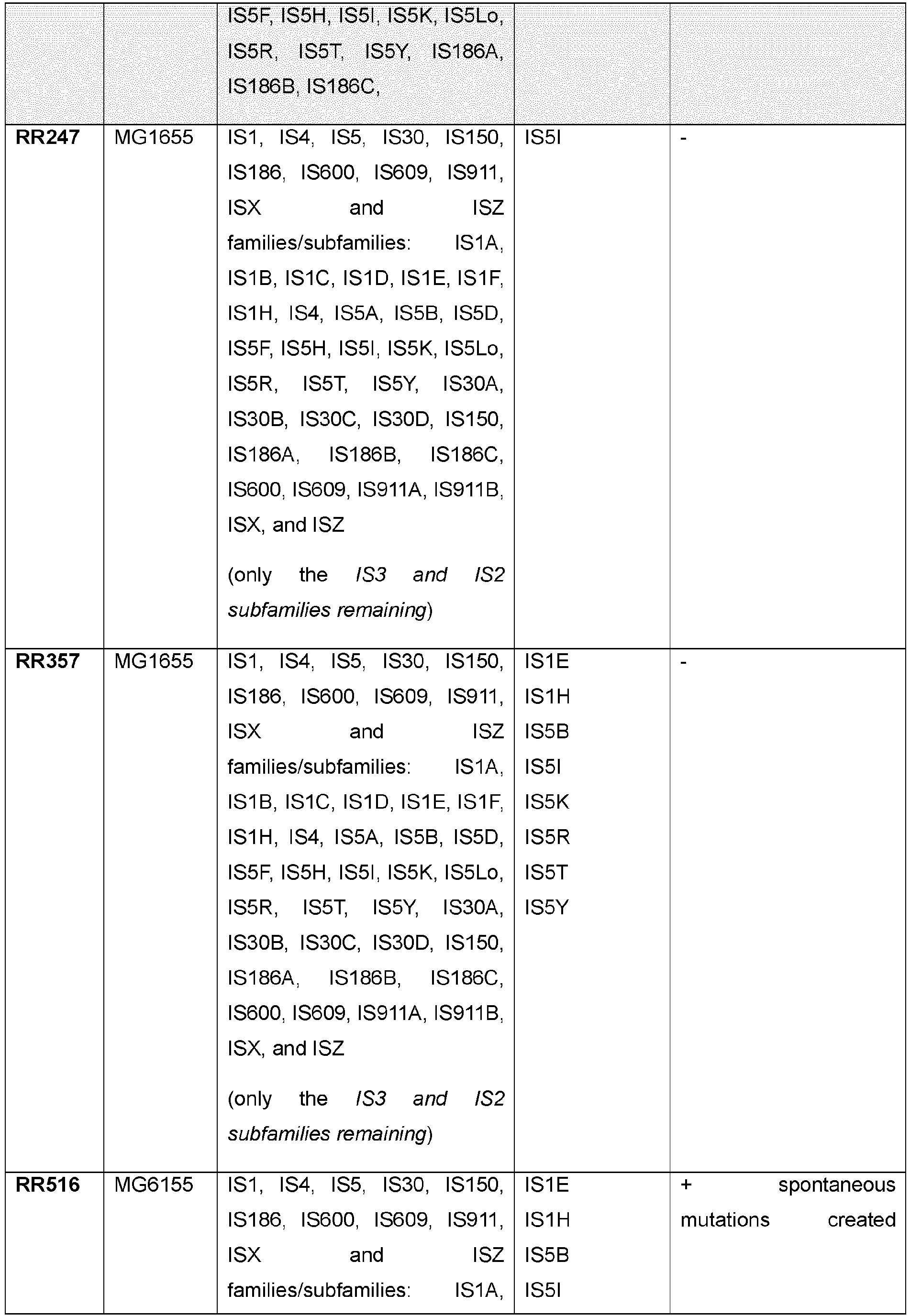

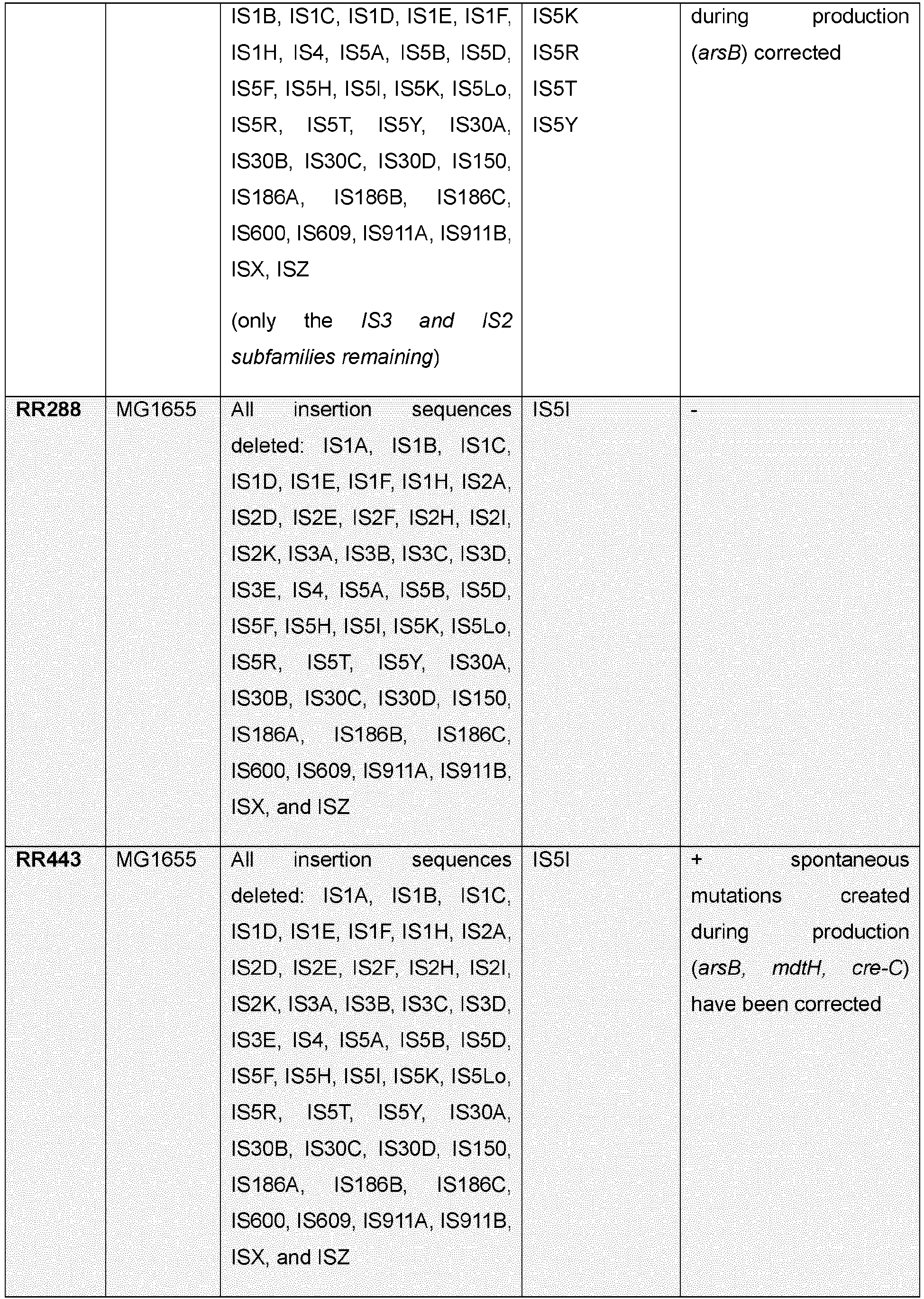

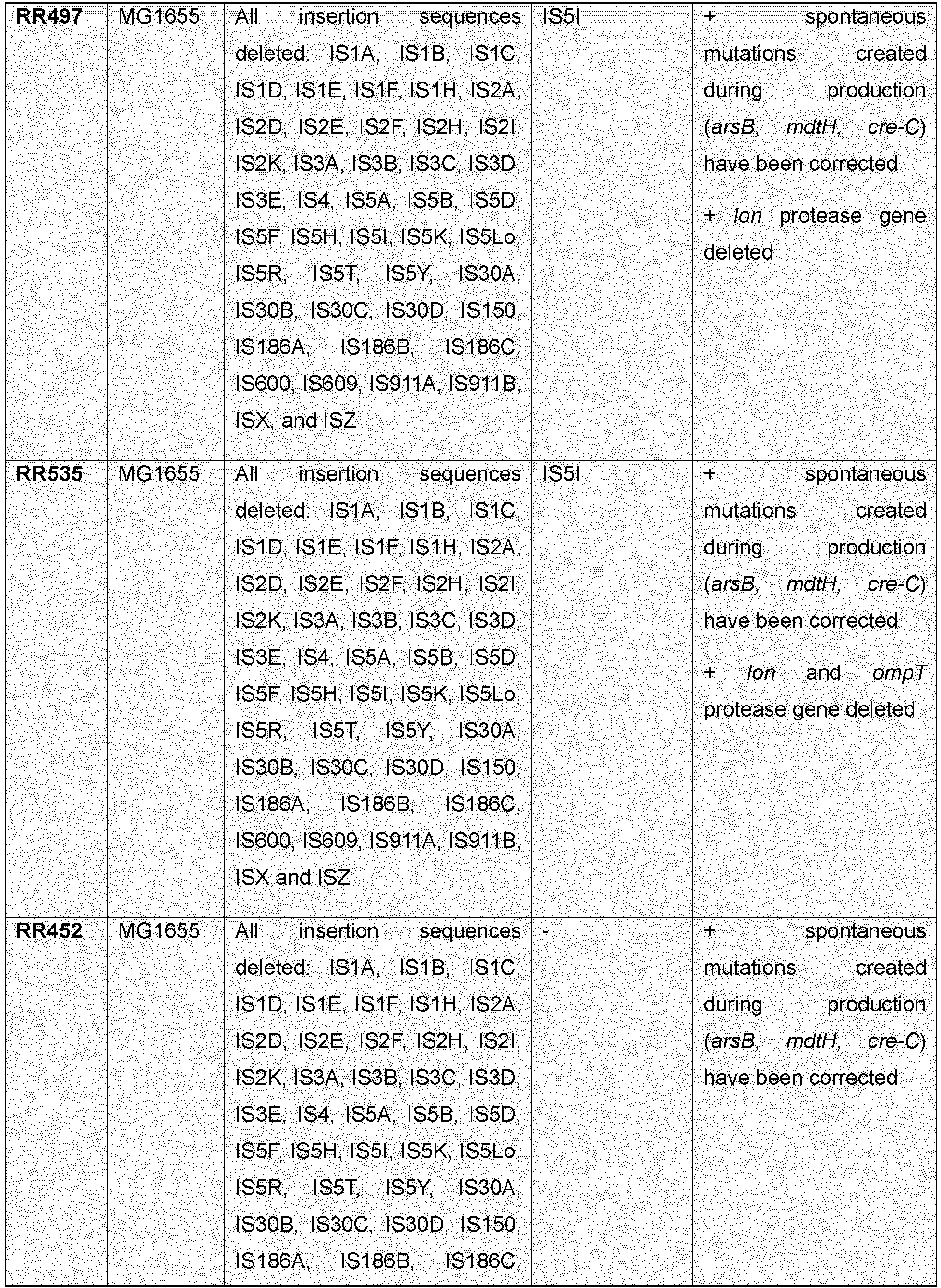

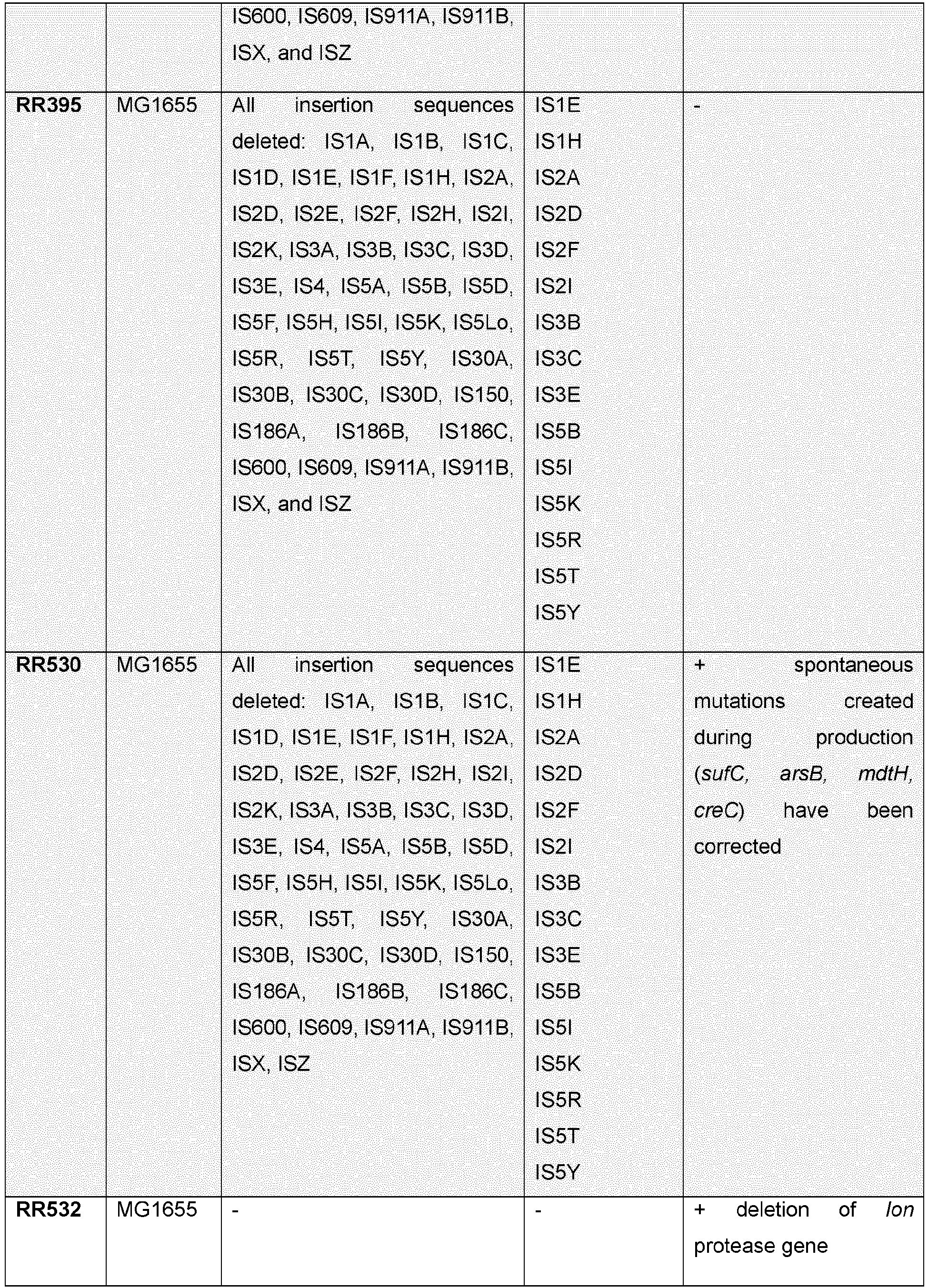

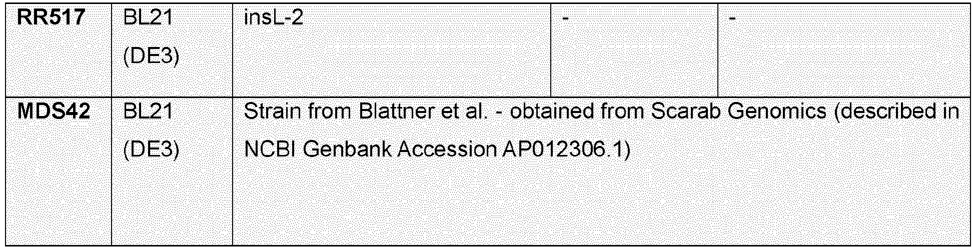

To prepare the modified bacterial strains, the DIRex method as described in Nävsall (2017. PLOS ONE, 12 (8): e0184126) was used (as shown in **Figure 1**).

In brief, two partial insertion cassettes (shown in Figure 1A) were generated by two separate polymerase chain reactions (PCR), for each IS that is to be deleted or replaced by a stop codon/frame-shift mutation.

The first partial insertion cassette comprises: (i) a long (~40 nucleotide) sequence (H1-a) identical to the genome sequence immediately before (5') the target IS, (ii) a short (~15 nucleotide) sequence identical to the genome sequence immediately after (3') the target IS (H2-b), and (iii) a truncated selection gene (e.g. *cat* for chloramphenicol resistance or *kan* for kanamycin resistance, as used below).

The PCR reaction for the first partial insertion cassette uses an *Acatsac1* (SEQ ID NO: 167) or *Akansac1* (SEQ ID NO: 169) DNA template which comprise the selection genes *cat* and *kan,* respectively, along with long inverted repeat sequences. The PCR reaction also uses two oligomers. The first oligomer `Fp1' comprises sequences H2-b and H1-a as discussed above (see **Table 4** for a list of oligomers). When using *Acatsac1* as the DNA template, the second oligomer was cat-midR (SEQ ID NO: 158). When using *Akansac1* as the DNA template, the second oligomer was kan-midR (SEQ ID NO: 160). Details of the PCR reaction mixture and amplification conditions are shown below.

The second partial insertion cassette comprises: (i) the remainder of the truncated selection gene used in the first partial insertion cassette, and the truncated *cat* sequences in the first and second partial cassette have ~277 nucleotide overlap, such that the truncated selection gene in the first and second partial insertion cassette, when recombined, form a functional *cat* sequence, (ii) a short (~15 nucleotide) sequence identical to the genome sequence immediately before (5') the target IS (H1-b) and (iii) a long (~40 nucleotide) sequence identical to the genome sequence immediately after (3') the target IS (H2-a). As shown in Figure 1A, the two partial insertion cassettes may also include other selection genes if desired.

The PCR reaction for the second partial insertion cassette uses an *Acatsac3* (SEQ ID NO: 168) or *Akansac3* (SEQ ID NO: 170) DNA template and two oligomers. The first oligomer `Rp1' comprises sequences H2-a and H1-b as discussed above (see **Table 4** for a list of oligomers). When using *Acatsac3* as the DNA template, the second oligomer was cat-midF (SEQ ID NO: 157). When using *Akansac3* as the DNA template, the second oligomer was kan-midF (SEQ ID NO: 159). Details of the PCR reaction mixture and amplification conditions are shown below.

**Table 4. Oligomers**

| **SEQ ID NO** | **Oligomer Name** | **Sequence** |
|---|---|---|
| **75** | **Fp1 IS1A** | TACTTCCTTCATGGGGATGCTGAAAAGAGTAGTAATTGCTTACCTCAATGTGTATGTGT AGGCTGGAGCTGCTTC |
| **76** | **Rp1 IS1A** | CCCCACAAAGAATATGGATATTGTGATACACATTGAGGTAAGCAATTACTACTCTGTGT AGGCTGGAGCTGCTTC |
| **77** | **Fp1 IS1B** | GCTGAGTGAGGCTTCATCGAGCGATTTATCGATTTGGCCCGAGAACACAAATGGGGTGT AGGCTGGAGCTGCTTC |
| **78** | **Rp1 IS1B** | TTTTAGGAAAGTACTGCCGAATTAGCCCATTTGTGTTCTCGGGCCAAATCGATAAGTGT AGGCTGGAGCTGCTTC |
| **79** | **Fp1 IS1C** | CTTCAGCGGTGATGGAGGCGATATATCTGTTACCGGGTAAGAATTTTCAGCCCGGGTGT AGGCTGGAGCTGCTTC |
| **80** | **Rp1 IS1C** | TATTCGACATAGGTACGTGAAACATCCGGGCTGAAAATTCTTACCCGGTAACAGAGTGT AGGCTGGAGCTGCTTC |
| **81** | **Fp1 IS1D** | AAAGCTTGTGTTTCATAACTTTTCCTTTATTCATCGCATGGACAATACGTACGCTGTGT AGGCTGGAGCTGCTTC |
| **82** | **Rp1 IS1D** | CTCAAAGTAGACAATCCTGATCTCAAGCGTACGTATTGTCCATGCGATGAATAAAGTGT AGGCTGGAGCTGCTTC |
| **83** | **Fp1 IS1E** | CGGGGATTATAGCATTTCAATATTTACTTATGACATTAAAAGTAACTTTTTTGAAGTGT AGGCTGGAGCTGCTTC |
| **84** | **Rp1 IS1E** | TTTCTGTGCCAATATTATCTCTAATTTCAAAAAAGTTACTTTTAATGTCATAAGTGTGT AGGCTGGAGCTGCTTC |
| **85** | **Fp1 IS1F** | TATTATTGACATCCTCACTGCCCTAAAGGATGGGGATTTCGACCATGTTTATTTCGTGT AGGCTGGAGCTGCTTC |
| **86** | **Rp1 IS1F** | AATAAGAACAATACCCACAATGTATGAAATAAACATGGTCGAAATCCCCATCCTTGTGT AGGCTGGAGCTGCTTC |
| **87** | **Fp1 IS1H** | GTATTACAGCGGCGATCTCTGCCGTATGGATGAGGCTGGCTATATAAAAATTACCGTGT AGGCTGGAGCTGCTTC |
| **88** | **Rp1 IS1H** | GGACAATAATATCTTTTTTGCGTCCGGTAATTTTTATATAGCCAGCCTCATCCATGTGT AGGCTGGAGCTGCTTC |
| **89** | **Fp1 IS5A** | ATGGAAGGTGCGAATAAGCGGGGAAATTCTTCTCGGCTGAGGAGGCGTTATGAGCGTGT AGGCTGGAGCTGCTTC |
| **90** | **Rp1 IS5A** | TAATCAGATACAAAAAGGGCCGCCAGCTCATAACGCCTCCTCAGCCGAGAAGAATGTGT AGGCTGGAGCTGCTTC |
| **91** | **Fp1 IS5B** | ACCTACAGCAACGATGTCATCAGTGCTTACACCGAGTGCTTTAGTGAAGTCATTTGTGT AGGCTGGAGCTGCTTC |
| **92** | **Rp1 IS5B** | ATTACAAAATCAACCTGCTTGACAAAAATGACTTCACTAAAGCACTCGGTGTAAGGTGT AGGCTGGAGCTGCTTC |
| **93** | **Fp1 IS5D** | TATCGTCTGCAACTTTATTGTGCAGTGTTGTGCCTGTTAGGTAACATTTAGTTTGGTGT AGGCTGGAGCTGCTTC |
| **94** | **Rp1 IS5D** | AAACAGCAATATCTTTACATTTAGCCAAACTAAATGTTACCTAACAGGCACAACAGTGT AGGCTGGAGCTGCTTC |
| **95** | **Fp1 IS5F** | TCAGATCATCAATAGTCAGGGAAGGAAGTAGCAACATTAGCTAACTAATCAATGCGTGT AGGCTGGAGCTGCTTC |
| **96** | **Rp1 IS5F** | CCTCTTAGCTGGATTTACAATCAACGCATTGATTAGTTAGCTAATGTTGCTACTTGTGT AGGCTGGAGCTGCTTC |
| **97** | **Fp1 IS5Y** | GACTTTCTCCGGGGATTATCTCCGCGTAACTGACAACAAGGGGAAAACGCACGACGTGT AGGCTGGAGCTGCTTC |
| **98** | **Rp1 IS5Y** | GACCGTCATCACTTCCGGTCAGCACGTCGTGCGTTTTCCCCTTGTTGTCAGTTACGTGT AGGCTGGAGCTGCTTC |
| **99** | **Fp1 IS5H** | CGTAATAGAATTATCCGGAATGGTGGCGACTATGCACTAGAACACCACAATTTCGGTGT AGGCTGGAGCTGCTTC |
| **100** | **Rp1 IS5H** | CTGTGACAGTGCCTTACCGAGAGAGCGAAATTGTGGTGTTCTAGTGCATAGTCGCGTGT AGGCTGGAGCTGCTTC |
| **101** | **Fp1 IS5I** | CGTATCAGAATAGATACTTTCTTTAGGAATTTTTGTTTTATTAATCCCTAACATAGTGT AGGCTGGAGCTGCTTC |
| **102** | **Rp1 IS5I** | TGCTTTCTGATTTTATTGTGTCATTTATGTTAGGGATTAATAAAACAAAAATTCCGTGT AGGCTGGAGCTGCTTC |
| **103** | **Fp1 IS5K** | TCACCATACTCAACAGTTGATGCCAATGCTATGGGTGAATATAACGCTGTAGCCAGTGT AGGCTGGAGCTGCTTC |
| **104** | **Rp1 IS5K** | TGTCGTTCCGCAATATCTGTTCTGGTGGCTACAGCGTTATATTCACCCATAGCATGTGT AGGCTGGAGCTGCTTC |
| **105** | **Fp1 IS5LO** | TCATGTTTGTCATCTGGAGCCATAGAACAGGGTTCATCATCTAAATCAGGTCATAGTGT AGGCTGGAGCTGCTTC |
| **106** | **Rp1 IS5LO** | GTTTGGCGTTAAGTATCTCGAAGCGTATGACCTGATTTAGATGATGAACCCTGTTGTGT AGGCTGGAGCTGCTTC |
| **107** | **Fp1 IS5R** | AAAATGACAATCCGGGCAGCACAGGAACAGCATCAATTAGTACTTCCCCCAATAAGTGT AGGCTGGAGCTGCTTC |
| **108** | **Rp1 IS5R** | ACCATTTTGCATAAACACCAGCCAATTATTGGGGGAAGTACTAATTGATGCTGTTGTGT AGGCTGGAGCTGCTTC |
| **109** | **Fp1 IS5T** | AAGATCTACTTAATATAAATTTCACTGACGCAAATTTAGGTGACACTATTTTTTTGTGT AGGCTGGAGCTGCTTC |
| **110** | **Rp1 IS5T** | TTTGGGGGTAGGGTGTTCTTTATGTAAAAAAATAGTGTCACCTAAATTTGCGTCAGTGT AGGCTGGAGCTGCTTC |
| **111** | **Fp1 IS186A** | TTGAAGATATGCGAGCACCTGTAAAGTGGCGGGATCACTCCCCGCCGTTGCTCTGTGT AGGCTGGAGCTGCTTC |
| **112** | **Rp1 IS186A** | TTTTCACGGCTTACGAATCCGAGTAAGAGCAACGGCGGGGAGTGATCCCCGCCACGTGT AGGCTGGAGCTGCTTC |
| **113** | **Fp1 IS186B** | GCTTACGAACCGAAGAAGTAGCCGTTGTGCGGGGAGTAATCCCCGCATATCCGGTGTGT AGGCTGGAGCTGCTTC |
| **114** | **Rp1 IS186B** | GGTGCCTTACCATCCTGACCTGACAACCGGATATGCGGGGATTACTCCCCGCACAGTGT AGGCTGGAGCTGCTTC |
| **115** | **Fp1 IS186C** | TGGTGCTGTAAGACCATACATAAAAAAGCCGGGATAATTCCCCGGTTTTTTTACGTGT AGGCTGGAGCTGCTTC |
| **116** | **Rp1 IS186C** | CGCAGCCATTAGCTGATTAACAGGCGTAAAAAAACCGGGGAATTATCCCCGGCTTGTGT AGGCTGGAGCTGCTTC |
| **117** | **Fp1 IS4** | TAACAGTTGATGCTGAAACGAAATAGGCATCTTGGTTCTGGTGTTTGTAACAAACGTGT AGGCTGGAGCTGCTTC |
| **118** | **Rp1 IS4** | ATTGAGCATATTTTTGTAGCTGATAGTTTGTTACAAACACCAGAACCAAGATGCCGTGT AGGCTGGAGCTGCTTC |
| **119** | **Fp1 IS911A & IS30A** | TTTGCTAAGAGCTTCATTCATGAATCCTTTTCACTAGGGAGTGTGCCCGGAGTTCGTGT AGGCTGGAGCTGCTTC |
| **120** | **Rp1 IS911A & IS30A** | TCATTTAAGCATCCATGCCCGCCCTGAACTCCGGGCACACTCCCTAGTGAAAAGGGTGT AGGCTGGAGCTGCTTC |
| **121** | **Fp1 IS30B** | GCTGAGTGAGGCTTCATCGAGCGATTTATCGATTTGGCCCTTAAATGACTCGGAGGTGT AGGCTGGAGCTGCTTC |
| **122** | **Rp1 IS30B** | GAATCGCCACGGGTTTAACAGACACCTCCGAGTCATTTAAGGGCCAAATCGATAAGTGT AGGCTGGAGCTGCTTC |
| **123** | **Fp1 ISZ** | AAGTTGCTTTGGGTAAACAAAAAATACGGCCCCAGAAGGGGCCGTTTATGTTGCCGTGT AGGCTGGAGCTGCTTC |
| **124** | **Rp1 ISZ** | ATCCGCTTAATCAGTAGCGCTGTCTGGCAACATAAACGGCCCCTTCTGGGGCCGTGTGT AGGCTGGAGCTGCTTC |
| **125** | **Fp1 IS30C** | TTATGCTGATTCCGGTGCGAAAATTGTTAATAAAGGTACTGTTGAAATTCAGAATGTGT AGGCTGGAGCTGCTTC |
| **126** | **Rp1 IS30C** | CACCAGTAACAAAAGCAAAACCTAAATTCTGAATTTCAACAGTACCTTTATTAACGTGT AGGCTGGAGCTGCTTC |
| **127** | **Fp1 IS609** | AAAAGTAGTTCCAGACGCGCCATCGTTAGATGGCGCTTTTTCAATATCGGTTTCTGTGT AGGCTGGAGCTGCTTC |
| **128** | **Rp1 IS609** | CGTGGTGTTGAATTTAATCGCTGACAGAAACCGATATTGAAAAAGCGCCATCTAAGTGT AGGCTGGAGCTGCTTC |
| **129** | **Fp1 IS911, IS30D & IS600** | CATGCGTCCAGATAAGTCATTGTAAAAGTCGAATCAGGGCGACTACCTGAAGGAGTGT AGGCTGGAGCTGCTTC |
| **130** | **Rp1 IS911B, IS30D & IS600** | ACTGCAACTGCCAGGCGACCGCGGCTCCCTTCAGGTAGTCGCCCTGATTCGACTTGTGT AGGCTGGAGCTGCTTC |
| **131** | **Fp1 IS150** | TGCGGCATTCCCAGCCTCCTGTGTTGATTTCCAACGAGTAAACTTTGATTTATAGGTGT AGGCTGGAGCTGCTTC |
| **132** | **Rp1 IS150** | GCAGACAGAGAAAAGCCCCACCTGACTATAAATCAAAGTTTACTCGTTGGAAATCGTGT AGGCTGGAGCTGCTTC |
| **133** | **Fp1 IS3A** | CGAGGTGACCTATACGCCAGTGCCTCTTACCCAGCAATAGTTAGGCCTGTGTCCAGTGT AGGCTGGAGCTGCTTC |
| **134** | **Rp1 IS3A** | ACTTTATGATCCTACCCACGTAATATGGACACAGGCCTAACTATTGCTGGGTAAGGTGT AGGCTGGAGCTGCTTC |
| **135** | **Fp1 IS2A** | CGCCATTGCTGATTATTGTGCCAGGACGAATAAACGCATAATTACCTATCAGGCAGTGT AGGCTGGAGCTGCTTC |
| **136** | **Rp1 IS2A** | GGGTCCGGTAGTGATTGGCGCAAACTGCCTGATAGGTAATTATGCGTTTATTCGTGTGT AGGCTGGAGCTGCTTC |
| **137** | **Fp1 IS3B** | TACTGTTGCGTTGACCGAATCTACTGGGTGGGGGCTGATTACAACCTGTACGCCGTGT AGGCTGGAGCTGCTTC |
| **138** | **Rp1 IS3B** | CGCCGTTAGTTACGGTGATAGTATTGGCGTACAGGTTGTAATCAGCCCCCCACCCGTGT AGGCTGGAGCTGCTTC |
| **139** | **Fp1 IS3C** | TGCACAGGACGTTGCCGCGGTAACAGGCGCAACCGTAATCACCAGCATAAATCAGGTGT AGGCTGGAGCTGCTTC |
| **140** | **Rp1 IS3C** | CTGCCCGCGCCATTTTAGCCGCAGCCTGATTTATGCTGGTGATTACGGTTGCGCCGTGT AGGCTGGAGCTGCTTC |
| **141** | **Fp1 IS3D** | GATGTGGTGAATTTTTGTGAGTCACCATAAAGCGGCATTTGTGCCTGGCACACGCGTGT AGGCTGGAGCTGCTTC |
| **142** | **Rp1 IS3D** | GCCTGAACGCCGTCTTCCATCAAACGCGTGTGCCAGGCACAAATGCCGCTTTATGGTGT AGGCTGGAGCTGCTTC |
| **143** | **Fp1 IS2D** | CAGGATATTGACAGTTTTGAACATCATGGGACAAGAACCCCAGAACTTACTTATGGTGT AGGCTGGAGCTGCTTC |
| **144** | **Rp1 IS2D** | TTAACAATTTTCGCACCGGAATCAGCATAAGTAAGTTCTGGGGTTCTTGTCCCATGTGT AGGCTGGAGCTGCTTC |
| **145** | **Fp1 IS3E** | GGCGATAAGCATTGTCGTACTTCCGCTGTCGAGAATAACGGTCATCCCTTCGTGGGTGT AGGCTGGAGCTGCTTC |
| **146** | **Rp1 IS3E** | TAGCGCAGGCGGCAGTCAAAATGATCCACGAAGGATGACCGTTATTCTCGACAGGTGT AGGCTGGAGCTGCTTC |
| **147** | **Fp1 IS2E** | CCGCCATCGAAACGGGCACATACAGGCAGAGTGGCCGTGAGTGATAACAGATGTCGTGT AGGCTGGAGCTGCTTC |
| **148** | **Rp1 IS2E** | CTGTGGATTTGCCCCTATATTTCCAGACATCTGTTATCACTCACGGCCACTCTGCGTGT AGGCTGGAGCTGCTTC |
| **149** | **Fp1 IS2F** | CAACTCCTTGGCAGGCCGTAAACAAATCCGCATTCGTGGCGAAGCATCCTCCCGTGTGT AGGCTGGAGCTGCTTC |
| **150** | **Rp1 IS2F** | CCTGACCATCAATGAGAATTAAAACACGGGAGGATGCTTCGCCACGAATGCGGATGTGT AGGCTGGAGCTGCTTC |
| **151** | **Fp1 IS2H** | TGAGTAAACAGATCAATGGAAGTAAATTCCAATTGTTTATCTAAACTTGCTCTTTGTGT AGGCTGGAGCTGCTTC |
| **152** | **Rp1 IS2H** | TCTTTCAAAGATAAACACCAGAAGAAAAGAGCAAGTTTAGATAAACAATTGGAATGTGT AGGCTGGAGCTGCTTC |
| **153** | **Fp1 IS2I** | GCGAATTGATTAAAAATATTTATTGTTCATTATCCGTTATCATCATTGGTTGTGCGTGT AGGCTGGAGCTGCTTC |
| **154** | **Rp1 IS2I** | GTTGAATTCAACGGCATAAGCTGACGCACAACCAATGATGATAACGGATAATGAAGTGT AGGCTGGAGCTGCTTC |
| **155** | **Fp1 IS2K** | ACTGACGTAAGGAGTGCTGCAGGGATTTGATGGTCCCTTGTGCTGATATGAATACGTGT AGGCTGGAGCTGCTTC |
| **156** | **Rp1 IS2K** | GAAAAGTATTCTGTATAAAATATCCGTATTCATATCAGCACAAGGACCATCAAAGTGT AGGCTGGAGCTGCTTC |
| **157** | **Cat-midF** | CGACGATTTCCGGCAGTTTC |
| **158** | **Cat-midR** | GCCGACATGGAAGCCATCAC |
| **159** | **Kan-midF** | CCTGATGATGCGTGGTTACT |
| **160** | **Kan-midR** | AATCCGGTGAGAATGGCAAG |
| **171** | **Fp1-STOP IS2A** | CGCCATTGCTGATTATTGTGCCAGGACGAATAAACGCATACCTATTAATTACCTATCAG GGTGTAGGCTGGAGCTGCTTC |
| **172** | **Rp1-STOP IS2A** | GGGTCCGGTAGTGATTGGCGCAAACTGCCTGATAGGTAATTAATAGGTATGCGTTTATT CGTGTAGGCTGGAGCTGCTTC |
| **173** | **Fp1-STOP IS3A** | TACTGTTGCGTTGACCGAATCTACTGGGTGGGGGCTGATTAATAGGTACAACCTGTAC GGTGTAGGCTGGAGCTGCTTC |
| **174** | **Rp1-STOP IS3A** | CGCCGTTAGTTACGGTGATAGTATTGGCGTACAGGTTGTACCTATTAATCAGCCCCCCA CGTGTAGGCTGGAGCTGCTTC |
| **175** | **Fp1-STOP IS3C** | TGCACAGGACGTTGCCGCGGTAACAGGCGCAACCGTAATCTAATAGGACCAGCATAAAT CGTGTAGGCTGGAGCTGCTTC |
| **176** | **Rp1-STOP IS3C** | CTGCCCGCGCCATTTTAGCCGCAGCCTGATTTATGCTGGTCCTATTAGATTACGGTTGC GGTGTAGGCTGGAGCTGCTTC |
| **177** | **Fp1-STOP IS5B** | GACCTACAGCAACGATGTCATCAGTGCTTACACCGAGTGCCCTATTATTTAGTGAAGTC AGTGTAGGCTGGAGCTGCTTC |
| **178** | **Rp1-STOP IS5B** | TTACAAAATCAACCTGCTTGACAAAAATGACTTCACTAAATAATAGGGCACTCGGTGTA AGTGTAGGCTGGAGCTGCTTC |
| **179** | **Fp1-STOP IS5Y** | GACTTTCTCCGGGGATTATCTCCGCGTAACTGACAACAAGTAATAGGGGAAAACGCAC GGTGTAGGCTGGAGCTGCTTC |
| **180** | **Rp1-STOP IS5Y** | GACCGTCATCACTTCCGGTCAGCACGTCGTGCGTTTTCCCCCTATTACTTGTTGTCAGT TGTGTAGGCTGGAGCTGCTTC |
| **181** | **Fp1-STOP IS2D** | GCAGGATATTGACAGTTTTGAACATCATGGGACAAGAACCTAATAGGCCAGAACTTACT TGTGTAGGCTGGAGCTGCTTC |
| **182** | **Rp1-STOP IS2D** | TAACAATTTTCGCACCGGAATCAGCATAAGTAAGTTCTGGCCTATTAGGTTCTTGTCCC AGTGTAGGCTGGAGCTGCTTC |
| **183** | **Fp1-STOP IS1H** | GTATTACAGCGGCGATCTCTGCCGTATGGATGAGGCTGGCTAATAGGTATATAAAAATT AGTGTAGGCTGGAGCTGCTTC |
| **184** | **Rp1-STOP IS1H** | GGACAATAATATCTTTTTTGCGTCCGGTAATTTTTATATACCTATTAGCCAGCCTCATC CGTGTAGGCTGGAGCTGCTTC |
| **185** | **Fp1-STOP IS2F** | CAACTCCTTGGCAGGCCGTAAACAAATCCGCATTCGTGGCTAATAGGGAAGCATCCTCC CGTGTAGGCTGGAGCTGCTTC |
| **186** | **Rp1-STOP IS2F** | CCTGACCATCAATGAGAATTAAAACACGGGAGGATGCTTCCCTATTAGCCACGAATGCG GGTGTAGGCTGGAGCTGCTTC |
| **187** | **Fp1-STOP IS5I** | CGTATCAGAATAGATACTTTCTTTAGGAATTTTTGTTTATTCCTATTAAATCCCTAAC AGTGTAGGCTGGAGCTGCTTC |
| **188** | **Rp1-STOP IS5I** | TGTGCTTTCTGATTTTATTGTGTCATTTATGTTAGGGATTTAATAGGAATAAAACAAAA AGTGTAGGCTGGAGCTGCTTC |
| **189** | **Fp1-STOP IS3E** | CGGCGATAAGCATTGTCGTACTTCCGCTGTCGAGAATAACCCTATTAGGTCATCCCTTC GGTGTAGGCTGGAGCTGCTTC |
| **190** | **Rp1-STOP IS3E** | AGCGCAGGCGGCAGTCAAAATGATCCACGAAGGATGACCTAATAGGGTTATTCTCGAC AGTGTAGGCTGGAGCTGCTTC |
| **191** | **Fp1-STOP IS5K** | TCACCATACTCAACAGTTGATGCCAATGCTATGGGTGAATACCTATTATAACGCTGTAG CGTGTAGGCTGGAGCTGCTTC |
| **192** | **Rp1-STOP IS5K** | TTGTCGTTCCGCAATATCTGTTCTGGTGGCTACAGCGTTATAATAGGTATTCACCCATA GGTGTAGGCTGGAGCTGCTTC |
| **193** | **Fp1-STOP IS2I** | CGAATTGATTAAAAATATTTATTGTTCATTATCCGTTATCTAATAGGATCATTGGTTGT GGTGTAGGCTGGAGCTGCTTC |
| **194** | **Rp1-STOP IS2I** | TGTTGAATTCAACGGCATAAGCTGACGCACAACCAATGATCCTATTAGATAACGGATAA TGTGTAGGCTGGAGCTGCTTC |
| **195** | **Fp1-STOP IS5R** | AAATGACAATCCGGGCAGCACAGGAACAGCATCAATTAGTTAATAGGACTTCCCCCAAT AGTGTAGGCTGGAGCTGCTTC |
| **196** | **Rp1-STOP IS5R** | GACCATTTTGCATAAACACCAGCCAATTATTGGGGGAAGTCCTATTAACTAATTGATGC TGTGTAGGCTGGAGCTGCTTC |
| **197** | **Fp1-STOP IS1E** | CGGGGATTATAGCATTTCAATATTTACTTATGACATTAAATAATAGGAGTAACTTTTTT GGTGTAGGCTGGAGCTGCTTC |
| **198** | **Rp1-STOP IS1E** | TTTCTGTGCCAATATTATCTCTAATTTCAAAAAAGTTACTCCTATTATTTAATGTCATA AGTGTAGGCTGGAGCTGCTTC |
| **199** | **Fp1-STOP IS5T** | CGAAGATCTACTTAATATAAATTTCACTGACGCAAATTTATAATAGGGGTGACACTATT TGTGTAGGCTGGAGCTGCTTC |
| **200** | **Rp1-STOP IS5T** | TGGGGGTAGGGTGTTCTTTATGTAAAAAAATAGTGTCACCCCTATTATAAATTTGCGTC AGTGTAGGCTGGAGCTGCTTC |

| | | |
|---|---|---|
| *Note: sequences indicated in **bold** in the Table above are the P1 binding site of each oligomer that binds to the template Acatsac1, Acatsac3, Akansac1 and Akansac3. Sequences indicated by underline relate to SEQ ID NOs:* 72 *or* 73 *(i.e., a double stop codon and frameshift mutation).* | | |

Each PCR reaction for both the first and second partial insertion cassettes, for each target IS, used water (33 µL), 2.5 µL of forward oligomer (10 µM), 2.5 µL of reverse oligomer (10 µM), 1 µL dNTP (10 mM), 10 µL buffer, 0.5 µL template (20 ng/µL) and 0.5 µL Q5 polymerase (NEB #M0491L). The PCR program shown in **Table 5** was used to generate both the first and second partial insertion cassettes for each target IS.

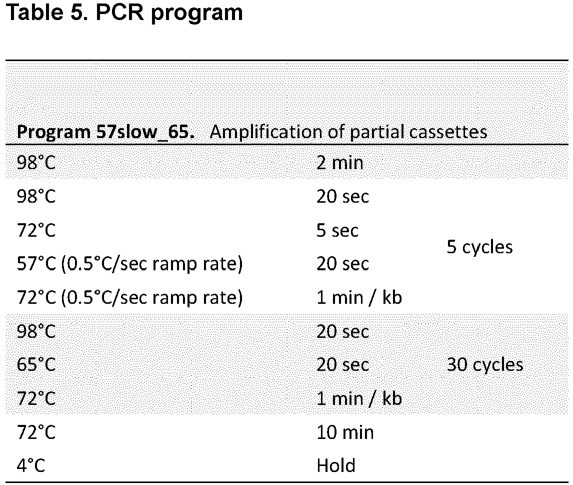

The PCR product for the first and second partial insertion cassettes for each target IS was then purified using NEB PCR cleanup kit #T1030.

The two partial cassettes for a single target IS were then mixed in equimolar amounts of 0.15 pmol each (forAcatsac1 PCR product of 1643 bp this is 153 ng, for the Acatsac3 PCR product of 2798 bp this is 260 ng, for the Akansac1 PCR product of 1611 bp this is 149 ng, and for the Akansac3 PCR product of 3013 bp this is 280 ng) and electroporated into the target bacterial strain.

To replace, and thus effectively delete, the insertion sequence, three recombination events occur (dotted lines in Figure 1A) in the bacterium. The three recombination events are: 1) a recombination event between the H1-a sequence in the first partial insertion cassette and the corresponding H1-a sequence in the bacterial genome, 2) a recombination event between the H2-a sequence in the second partial insertion cassette and the corresponding H2-a sequence in the bacterial genome, and 3) a recombination event between the truncated, overlapping *cat* (~277 nucleotide overlap) or *kan* (~304 nucleotides overlap) selection gene sequences in the first and second partial insertion cassettes. This produces the metastable intermediate bacterium shown in **Figure 1B** where the IS is replaced with the sequences of the first and second partial insertion cassettes.

To induce these three recombination events, lambda red induction of the *E. coli* carrying the pSIM5-Tet lambda red plasmid is performed, with slight modifications to the general description by Näsvall 2017. Briefly, an overnight aerobic culture is prepared in LB with 7.5 mg/L tetracycline at 30°C as previously described. However, it is then diluted in 20 mL of fresh LB with 0.2% glucose and 7.5 mg/L tetracycline to a starting OD of 0.1 and incubated at 30°C until OD reaches 0.5 and it is shifted to a 42°C water bath for 15 min to induce lambda red expression. This modification increases the number of cells and ensures that multiple replication forks are present in each cell, as compared to the method of Näsvall. After induction, cells are chilled on ice for 15 min, washed twice with cold 10% glycerol and concentrated by resuspending the pellet in 0.45 mL 10% glycerol. 0.05 mL of the prepared cells are electroporated with both PCR products (0.15 pmol each) in a 1mm gap cuvette with 1.8 kV and immediately collected in 1 mL of SOC medium and allowed to recover for 30 min before selection on antibiotic plates. Cat cassettes are selected with 12.5 mg/L chloramphenicol, *kan* cassettes are selected with 10 mg/L kanamycin.

The metastable intermediate spontaneously undergoes a fourth self-recombination event (indicated by dotted lined in **Figure 1B**) between the duplicated homology regions (i.e., between the H1-a-H2-b sequence and the H1-b-H2-a sequence) that removes the cassette as shown in **Figure 1C****.** The resulting recombinants are sucrose-resistant and therefore can be selected after several generations of growth in antibiotic free medium by plating on LB with 5% sucrose.

Sanger sequencing of purified clones was used to determine if the deletion was successful, identify any remaining sequence i.e., to determine whether any additional nucleotides had been deleted other than the IS (and TSD sequence where appropriate), to determine whether any IS (or TSD) sequence remained and to determine whether any unintended insertions occurred in the flanking sequence.

### Deletion of split IS

Where a target IS is split (e.g., IS911A and IS911B), the Fp1 oligomer provides a sequence relating to a first site of the split IS, and the Rp1 oligomer provides a sequence relating to a second site of the split IS. In this way, the first and second partial insertion cassettes target the first and second sites of the split IS, so that the entire split IS element is deleted.

### Deleting more than one IS

To produce combinations of two or more IS deletions, the process of electroporation, recombination, excision, and Sanger sequencing described above can be repeated for each target IS. However, a faster method is to generate multiple strains in parallel, where each strain carries a metastable intermediate (discussed above, i.e., after lambda red induction) for an IS deletion of interest. The metastable intermediates were then each transduced into a recipient bacterial strain using generalized transduction using P1vir phage. For transduction, the strain with the metastable intermediate is lysed by P1vir and residual cells removed by addition of chloroform and centrifugation. The clear lysate then contains the metastable intermediate inside P1vir particles is then mixed with fresh recipient bacterial cells, incubated for 23 min at 37°C for DNA injection, washed and after 60 min of recovery, plated onto antibiotic plates to select for cells that received the DIRex intermediate. After transduction, cells with spontaneous excision of the cassette (the fourth recombination event discussed above) were selected using sucrose.

### Deletion of IS and insertion of a stop codon and/or frame-shift mutation

In some examples, the H1 and H2 oligomers described above **(Table 4)** were modified to contain a double stop codon and frame-shift mutation (SEQ ID NOs: 72 and 73) to prevent potential target expression upon deleting the target IS **(****Figure 1D****).** SEQ ID NO: 73 was added where the target IS is located on a forward strand in the bacterial genome, and SEQ ID NO: 72 was added where the target IS is located on a reverse strand in the bacterial genome. The frameshift mutation allows the stop codons to be in the reading frame.

SEQ ID NOs: 72 or 73 were therefore inserted into the Fp1 and Rp1 oligomers with SEQ ID NOs: 75-156 between the H1 and H2 sequences to create Fp1 and Rp1 stop oligomers with SEQ ID NOs: 171-200. However, a slight modification was made to the H1-b and H2-b sequences to be 13 nucleotides in length to minimize the length of the stop oligomers (SEQ ID NOs: 171-200). In addition, the H1-H2 deletion junction was shifted by 1 or 2 nucleotides for some of the existing Fp1 and Rp1 oligomers (SEQ ID NOs: 75-156) to ensure SEQ ID NOs: 72 and 73 were in frame.

The same method was then followed as described above for each target IS to generate the strains of interest **(Table 3).**

In examples where a stop codon replaced an insertion sequence, Sanger sequencing was used to determine the correct insertion of the double stop and frame-shift mutation sequences.

### Reverting spontaneous mutations

Spontaneous mutations were tested for by genome sequencing starting with genome *de-novo* assembly from Oxford Nanopore long sequencing reads and whole-genome alignment to test for structural variants (inversions, deletions, duplications). We then tested for single nucleotide polymorphisms (SNPs) using Illumina data. Finally, the identified spontaneous mutations were validated using Sanger sequencing. Any confirmed spontaneous mutations that affected protein sequences were reverted in the mutant strains, using the DIRex method described above, with Fp1 and Rp1 oligomers that contain the correct reference sequence.

### Insertion sequence detection

IS detection was performed using the IS detection kit (Scarab Genomics, IS-1109-10) following the manufacturer's protocol.

### Results

As shown in **Figure 2A****,** the insertion sequence detection kit accurately identified the presence or absence of insertion sequences. As shown in **Figure 2A****,** a clear IS1-family band was visible for strain RR87 (lane 2) due to the presence of IS1H (**Table 3**), and in the positive control (lane 4), whereas the IS1-family band was not visible for strain RR95 where all IS1 family members were deleted (**Table 3**).

Therefore, all strains listed in **Table 3** were tested using the IS detection kit to further confirm deletion of the target IS. In each strain the expected bands were found. Examples are shown in **Figure 2B** which demonstrates that, as expected, no bands are visible for any of the IS families for RR452 (where all insertion sequences deleted, see lanes 1, 5, 9, 13, 17, 21, 25, 29, 33, 37, 41, 45, 49, and 53 in **Figure 2B**). The replacement of insertion sequences with a double stop frame shift sequence also did not affect deletion of the insertion sequences in strain RR530 (where all insertion sequences deleted but a double stop frame shift sequence was inserted into fifteen IS locations, see lanes 2, 6, 10, 14, 18, 22, 26, 30, 34, 38, 42, 46, 50, and 54 in **Figure 2B**)**.** Similarly, strain RR516 (see lanes 3, 7, 11, 15, 19, 23, 27, 31, 35, 39, 43, 47, 51, and 55 in **Figure 2B**), where only the IS2 and IS3 subfamilies were retained and double stop frame shift sequences inserted at the position of eight insertion sequences, only showed a positive band for IS2 and IS3 (see lanes 7 and 11 of **Figure 2B**). Control PCR for the essential gene *dnaE* produced the expected band of 682 bp in all strains (see lanes 57-60 in **Figure 2B**).

All of the deletions and mutations of the bacterial strains shown in **Table 3** were Sanger sequenced. **Table 6** summarises this data for each insertion site when full deletion was performed. As is evidenced by **Table 6,** sanger sequencing surprisingly confirmed that each of the deletions were 'seamless' in that only the intended IS (and corresponding TSD where present) was deleted, without any additional sequence flanking the IS and TSD being deleted. In addition, **Table 6** shows that Sanger sequencing surprisingly confirmed that nearly all deletions were 'scarless' in that they did not leave any residue of the IS/TSD and no other sequence was inserted. However, as can be seen from **Table 6,** single base pair insertions, deletion or mutations were identified in four of the 45 insertion sequences. However, all of these mutations were tested and confirmed that they had no impact on the protein coding DNA sequence. However, each mutation was then corrected using the method described above using the Fp1 and Rp1 oligomers (Table 4).

**Table 6. Scarless and seamless deletion of IS when IS fully deleted**

| **IS & associated TSD** | **Fp1 oligomer** | **Rp1 oligomer** | **Scar?** | **Seamless?** |
|---|---|---|---|---|
| IS1A | 75 | 76 | No | Yes |
| IS1B | 77 | 78 | No | Yes |
| IS1C | 79 | 80 | No | Yes |
| IS1D + 9bp TSD | 81 | 82 | No | Yes |
| IS1E + 9bp TSD | 83 | 84 | No | Yes |
| IS1F | 85 | 86 | Del. G | Yes |
| IS1H + 9bp TSD | 87 | 88 | No | Yes |
| IS5A | 89 | 90 | No | Yes |
| IS5B + 4bp TSD | 91 | 92 | No | Yes |
| IS5D + 4bp TSD | 93 | 94 | No | Yes |
| IS5F + 4bp TSD | 95 | 96 | No | Yes |
| IS5Y + 4bp TSD | 97 | 98 | No | Yes |
| IS5H + 4bp TSD | 99 | 100 | Del. C | Yes |
| IS5I + 4bp TSD | 101 | 102 | No | Yes |
| IS5K + 4bp TSD | 103 | 104 | No | Yes |
| IS5Lo | 105 | 106 | No | Yes |
| IS5R + 4bp TSD | 107 | 108 | T → G | Yes |
| IS5T + 4bp TSD | 109 | 110 | Del. TGA | Yes |
| IS186A + 6bp TSD | 111 | 112 | No | Yes |
| IS186B + 6bp TSD | 113 | 114 | No | Yes |
| IS186C + 6bp TSD | 115 | 116 | No | Yes |
| IS4 + 12bp TSD | 117 | 118 | No | Yes |
| IS911A - site 1 | 119 | NaN | No | Yes |
| IS911A - site 2 | NaN | 120 | No | Yes |
| IS30A | 119 | 120 | Deleted together with IS911A | |
| IS30B | 123 | 124 | No | Yes |
| ISX | 123 | 124 | Deleted together with IS30B | |
| ISZ | 125 | 126 | No | Yes |
| IS30C + 2bp TSD | 127 | 128 | No | Yes |
| IS609 + 3bp TSD | 129 | 130 | No | Yes |
| IS911B - site 1 | 131 | NaN | No | Yes |
| IS911B - site 2 | NaN | 132 | No | Yes |
| IS30D | 131 | 132 | Deleted together with IS911B | |
| IS600 | 131 | 132 | Deleted together with IS911B | |
| IS150 | 135 | 136 | No | Yes |
| IS3A | 137 | 138 | No | Yes |
| IS2A + 5bp TSD | 139 | 140 | No | Yes |
| IS3B + 3bp TSD | 141 | 142 | No | Yes |
| IS3C | 143 | 144 | No | Yes |
| IS3D | 145 | 146 | No | Yes |
| IS2D + 5bp TSD | 147 | 148 | No | Yes |
| IS3E + 3bp TSD | 149 | 150 | No | Yes |
| IS2E | 151 | 152 | No | Yes |
| IS2F + 5bp TSD | 153 | 154 | No | Yes |
| IS2H | 155 | 156 | No | Yes |
| IS2I + 5bp TSD | 157 | 158 | No | Yes |
| IS2K + 5bp TSD | 159 | 160 | No | Yes |

**Table 7** shows that replacing an IS with a double stop codon and frame-shift mutation (SEQ ID NO: 72 or 73) also surprisingly did not impact the scarless and seamless deletion of the IS. When referring to a scar in this example, it is meant that no residue of the IS/TSD or any other *unintended* sequences are inserted. As can be seen from **Table 7,** single base pair insertions, or deletion were identified in only two out of fifteen targeted insertion sequences. However, all of these mutations were confirmed to be phenotypically silent, because the achieved insertion of a double stop codon and frameshift successfully disrupted the expression of the sequence.

**Table 7. Scarless and seamless deletion of IS when double stop, frame-shift mutation replaced IS**

| **IS & associated TSD** | **Fp1 oligomer** | **Rp1 oligomer** | **Stop SEQ ID NO inserted in Fp1** | **Scar?*** | **Seamless?** |
|---|---|---|---|---|---|
| IS2A + 5bp TSD | 171 | 172 | 72 | No | Yes |
| IS3B + 3bp TSD | 173 | 174 | 73 | No | Yes |
| IS3C | 175 | 176 | 73 | Ins. G | Yes |
| IS5B + 4bp TSD | 177 | 178 | 72 | No | Yes |
| IS5Y + 4bp TSD | 179 | 180 | 73 | No | Yes |
| IS2D + 5bp TSD | 181 | 182 | 73 | No | Yes |
| IS1H + 9bp TSD | 183 | 184 | 73 | No | Yes |
| IS2F + 5bp TSD | 185 | 186 | 73 | No | Yes |
| IS5I + 4bp TSD | 187 | 188 | 72 | No | Yes |
| IS3E + 3bp TSD | 189 | 190 | 72 | Del. A | Yes |
| IS5K + 4bp TSD | 191 | 192 | 72 | No | Yes |
| IS2I + 5bp TSD | 193 | 194 | 73 | No | Yes |
| IS5R + 4bp TSD | 195 | 196 | 73 | No | Yes |
| IS1E + 9 bp TSD | 197 | 198 | 73 | No | Yes |
| IS5T + 4bp TSD | 199 | 200 | 73 | No | Yes |

| | | | | | |
|---|---|---|---|---|---|
| **other than the intended insertion* of *SEQ ID NO:* 72 *or 73* | | | | | |

Deletion of all IS in the MG1655 strain using this method resulted in a genome size of 98.9% compared to the wild type MG1655 strain.

### Example 2: Mutation rate in modified bacterial strains

### Methods

Mutation rates were determined using Luria-Delbrück fluctuation analysis, with n = 40-80 parallel lineages for each strain, and using the gold standard MSS method that provides accurate estimates across a wide range of mutation rates (Rosche & Foster, 2000. Determining Mutation Rates in Bacterial Populations. *Methods,* 20(1): 4-17).

For these experiments, the following strains were used: MG1655 wild type, MG1655 with all insertion sequences deleted (IS-, RR452), MG1655 with the *Ion* protease gene deleted (*Ion-,* RR532), MG1655 with all insertion sequences deleted, a stop sequence replacing IS5I, and the *Ion* protease gene deleted (*IS- Ion-* IS5I:stop, RR497), BL21 (DE3) wild type, and BL21 (DE3) with only the IS insL-3 deleted (BL21 ΔinsL-2, RR517) (Table 3).

### Results

Using selection for *bgl+* with salicin, we determined that bacteria with all insertion sequences deleted (RR452, *IS*-) had a reduced *bgl+* mutation rate during exponential growth in liquid culture compared to the wild type MG1655 strain. The *bgl+* mutation rate of RR452 (*IS*-) was 1.019 x 10⁻⁹ per cell per replication compared to the mutation rate of 9.0771 × 10⁻⁹ per cell per replication measured in the wild type MG1655 strain. The relative reduction in *bgl+* mutation rate is 9-fold (**Figure 3**).

BL21 wild type bacteria have an IS, *insL*-2 (a homologue of IS186), in the promoter region of *Ion* which inhibits expression of *Ion,* and therefore inhibits the production of Lon protease. We found that deletion of the IS *insL-*2 restored functionality of the Lon protease in BL21 *E. coli* (BL21 Δ*insL*-2 data not shown, referred to as RR517). Using selection for *cycR* with D-cycloserine, we surprisingly observed that the deletion of *insL*-2 IS in RR517 (BL21 Δ*insL*-2) bacteria reduced the mutation rate in the gene *cycA* from 21.639 x 10⁻⁸ per cell per replication in the BL21(DE3) wild type (BL21) to 6.4622 x 10⁻⁸ per cell per replication in the modified RR517 (BL21 Δ*insL-*2) bacteria. The relative reduction in *cycR* mutation rate was 3-fold **(****Figure 4A****).**

We therefore considered how deletion of *Ion* gene in *E. coli* strain MG1655 affects mutation rate. As shown in **Figure 4B****,** deletion of *Ion* in MG1655 (strain RR532, *Ion*-) surprisingly resulted in increased mutation rate of in *cycA* compared to MG1655 wild type bacteria. The deletion of *Ion* in MG1655 elevated mutation rate in RR532 (/on-) bacteria to 7.5783 x 10⁻⁸ per cell per replication, which was a 2-fold increase compared to the MG1655 wild type bacteria where the *cycA* mutation rate was 3.7091 x 10⁻⁸ per cell per replication. This corresponds to the findings discussed for BL21. We therefore conclude that Lon protease impacts mutation rate in *E. coli.*

However, we surprisingly observed that the *cycA* mutation rate in bacteria lacking *Ion* was reduced to wild type levels when all of the IS elements were also deleted in strain RR497 *(IS- Ion-)* **(****Figure 4B****).** Indeed, the *cycA* mutation rate in RR497 (*IS*- *Ion*-) bacteria was 3.517 x 10⁻⁸ per cell per replication. We therefore conclude that the observed doubling of mutation rate in RR532 *(Ion-)* bacteria is dependent on IS elements. This is the first observation that the effect of Lon protease on mutation rate is mediated by insertion sequences.

Similarly, we also observed that bacterial strains lacking all IS but without deletion of *Ion* in strain RR452 (IS-) matched wild type bacteria (WT), and both are lower than in BL21(DE3) strains in **Figures 4A and 4B****.** The *cycA* mutation rate of RR452 (*IS*-) was 3.6936 x 10⁻⁸ per cell per replication.

Using selection for rifampicin resistance, we determined mutation rates in the essential gene *rpoB* that mediates rifampicin susceptibility. Because *rpoB* is essential, the spectrum of possible mutations was restricted to base pair substitutions and small in-frame indels (length of 3, 6 or 9 nt) that do not defunctionalize the gene. Following the pattern discussed above, deletion of *Ion* in RR532 *(Ion-)* bacteria resulted in a large increase in the mutation rate in *rpoB* to 26.1198 x 10⁻⁹ per cell per replication compared to the MG1655 wild type *rpoB* mutation rate (5.0308 × 10⁻⁹ per cell per replication) **(****Figure 5****).** Again, we surprisingly found that deletion of *Ion* and all IS elements in bacterial strain RR497 resulted in a 2-fold reduction in the *rpoB* mutation rates to 11.0191 × 10⁻⁹ per cell per replication. This supports that there is a connection between the IS and non-IS mutation mechanisms.

The *rpoB* mutation rate for strain RR452 (IS-) MG1655 bacteria (2.7897 x 10⁻⁹ per cell per replication) was also surprisingly reduced compared to the MG1655 wild type bacteria (5.0308 x 10⁻⁹ per cell per replication) **(****Figure 5****).**

### Example 3: Protein production in modified bacterial strains

### Methods

### Comparison strain

MDS42 (Blattner et al.) strain was obtained from Scarab Genomics (as described in NCBI Genbank Accession AP012306.1).

### Production of recombinant proteins

We determined the ability of various embodiments of the invention to produce recombinant proteins by transforming them with the plasmid pZE1R-GFPaav and measuring GFP production by fluorescence. The plasmid has a colE1 *ori*, with a lambda pR promoter from which a short lived GFP is expressed. As references we used the commercially available strains MDS42 (Scarab Genomics, Madison, Wl, USA) and BL21 (DE3) (New England Biolabs). After pure-streaking the transformants on selective media, we prepared overnight cultures in lysogeny broth with selection for the plasmid encoded ampicillin resistance (100 mg/L ampicillin). After overnight growth, we diluted the overnight cultures 10⁻⁶ into fresh media with ampicillin, allowing for log2(10⁶) = 20 further generations of growth. To assess plasmid stability over time, we measured biomass and fluorescence after both the first and second culturing steps. We measured biomass (using absorbance at 600 nm) and GFP fluorescence (using excitation 395 nm and emission 510 nm) following a 10-fold dilution in medium, to avoid saturation of Lambert-Beer's law.

### Yield of liquid culture

We determined the ability of various embodiments of the invention to produce high growth yields, measured as colony forming units per mL (CFU/mL) after cultures had grown to saturation. We measured CFU/mL in lysogeny broth (LB) as an example for a complex growth medium.

### Results

After the first overnight growth, all strains produced similar biomass of plasmid-containing cells, with absorbance at 600 nm of roughly 3, except for RR530 (*IS*-*), which produced a biomass of roughly 1 (**Figure 6A****,** upper panel). However, after an additional 20 generations of growth, the biomass of plasmid-containing cells decreased slightly in MG1655 wild type but surprisingly increased in RR452 (*IS*-) and RR535 (*IS- Ion- ompT-* IS5I:stop), indicating higher stability of the plasmid in these strains than in the MG1655 wild type. The biomass of plasmid-containing cells remained similar in BL21 wild type, MDS42 (Blattner et al.) and RR530 (*IS*-*) (**Figure 6A**, lower panel compared to upper panel). The poor yield of RR530 (*IS*-*) could be explained by the double stop codon and frameshift mutation disrupting the function of multiple genes, including those that are relevant for bacterial growth.

The total GFP fluorescence signal of the first overnight culture was highest for RR535 (*IS- Ion- ompT-*IS5I:stop) and wild type MG1655, with GFP fluorescence signals of roughly 15,000 units, which was around 3-fold higher than the GFP yields obtained for MDS42 (Blattner et al.) and RR530 (*IS*-*) (**Figure 6B**, upper panel). However, after 20 generations of growth, the fluorescence yields surprisingly further decreased in MDS42 (Blattner et al.), and wild type strains BL21(DE3) and MG1655 showed a minor decrease. However fluorescence yields surprisingly remained stable in RR452 (*IS*-), RR535 (*IS- Ion- ompT-* IS5I:stop) and RR530 (*IS*-*) (**Figure 6B**, lower panel compared to upper panel). In addition, RR452 (*IS*-) showed similar fluorescence yields to the MG1655 (wild type) strain, and RR535 (*IS- Ion- ompT-* IS5I:stop) surprisingly showed increased fluorescence yields compared to the MG1655 wild type strain. Therefore, RR535 (*IS- Ion- ompT-* IS5I:stop) demonstrated excellent plasmid stability.

To assess the efficiency of GFP production, we normalized the obtained fluorescence yield by the measured biomass (**Figure 6C**). Although the most efficient GFP production was initially achieved by the MG1655 wild type **(Figu**r**e 6C** upper panel), its capacity rapidly deteriorated during further growth of 20 generations (**Figure 6C** lower panel). In contrast, RR530 (IS-*) and RR535 (*IS- Ion-*IS5I:stop) surprisingly retained their ability for highly efficient GFP production. The efficiency of MDS42 (Blattner et al.) and BL21 (wild type) was consistent over 20 generations but MDS42 (Blattner et al.) was surprisingly low (**Figure 6C**).

With regard to the yield of liquid culture, yields were surprisingly increased in RR452 (IS-) bacteria compared to the wild type MG1655 strain (**Figure 7**). Surprisingly, deletion of *Ion-* reduced the yield in RR532 *(Ion-)* strains, but deletion of *Ion* and all IS, with replacement of IS5I with a double stop codon and frameshift mutation in RR497 (*IS- Ion-* in figure) resulted in improved yields closer to that of the wild type strain (**Figure 7**).

### Example 4: growth rate and competitive index in modified bacterial strains

### Methods

### Growth rate

We determined the ability of various IS-deleted strains to grow in lysogeny broth (LB) using a Bioscreen C plate reader (OY Growth Curves Ab Ltd., Turku, Finland).

### Growth yield

We approximated the total yield of bacterial biomass after 22h of cultivation using undiluted absorbance at 600 nm (within the saturation).

### Competitive index

As a measurement ecological strength, we determined the ability of preferred embodiments of the invention to withstand competition for shared resources when in co-culture with a competing strain. We prepared mixtures of fluorescently labelled cells and allowed them to compete in lysogeny broth for a total of 60 generations of growth. We determined the relative number of each strain after 0, 20, 40 and 60 generations and then analysed the trajectory of competition. As controls, we competed each strain against itself labelled with blue fluorescent protein (*bfp*) and yellow fluorescent protein (yfp). This baseline was used to correct the slope of the other competitions for the cost of the fluorescent protein in the respective strain backgrounds. We then analysed each strain pairing twice, such that each strain was present in either fluorescence configuration, following the gold standard "dye-swap" experimental design.

### Results

### Growth rate

Strains RR452 (*IS*-) and RR530 (*IS*-*) had exponential growth rates of 1.97 and 2.02 doublings per hour, respectively, which was equivalent to the growth rate of 2.00 of the MG1655 wild type strain (**Figure 8A**). In contrast, strain MDS42 (Blattner et al.) had a surprisingly decreased growth rate of 1.31 doublings per hour, meaning a 34% decrease compared to MG1655 wild type. Expressed as doubling times, MG1655 wild type bacteria divide every 20.8 min, RR452 (*IS*-) divide every 21.1 min, RR530 (IS-*) divide every 20.5 min and MDS42 (Blattner et al.) every 31.6 min. These results indicated that the RR452 (*IS*-) and RR530 (*IS*-*) strains grew equally fast as the wild type and significantly faster than the MDS42 (Blattner et al.) strain. We speculated that these dynamics reflected the high metabolic capacity of the RR452 (*IS*-) and RR530 (*IS*-*) strains compared to the minimal genome of MDS42 (**Figure 8A**). These results are consistent with the yield data in **Figure 7****.** Together these data demonstrate that it is possible to delete IS elements without significantly compromising the growth abilities of the strain.

We also determined the growth ability for strain RR497 (*IS*- *Ion*-), which has a deletion of the *Ion* protease in addition to the IS deletions present in strain RR452 (*IS*-). Lon mutations are used for biotechnological purposes to decrease proteolysis. We found that *Ion* mutations only slightly decreased growth rate (growth rate is 1.94 for RR497 (*IS*- *Ion*-) and 1.92 for RR532 (*Ion*-)) (**Figure 8A**). Thus, this data shows that *Ion* mutations can be combined with IS deletions without significantly impairing growth.

### Growth yield

We approximated the total yield and found that strain RR452 (IS-) surprisingly produced a greater biomass than MG1655 wild type strain (**Figure 8B**). In addition, strains RR497 (*IS*- *Ion*-) and RR532 *(Ion-)* was around that of the MG1655 wild type strain (**Figure 8B**). Following the pattern of the other experiments, MDS42 (Blattner et al.) strain showed a greatly reduced growth yield (**Figure 8B**). However, we surprisingly found that strain RR530 (*IS*-*) showed a strongly reduced growth yield compared to any of the other strains tested. These results are consistent with the yield data in **Figure 6A****,** and therefore, the low yield could be explained by the double stop codon and frameshift mutation disrupting the function of multiple genes, including those that are relevant for bacterial growth.

Together these data demonstrated that it is possible with our invention to delete IS elements without significantly compromising the growth abilities of the strain.

### Competitive index

The computed baseline-corrected selection coefficients demonstrated that the strains RR452 (*IS*-) and RR530 (*IS*-*) have a roughly 5-fold higher capacity to compete against the MG1655 wild type than MDS42 (Blattner et al) strains (**Figure 9**). As shown in **Figure 9****,** RR452 (*IS*-) and RR530 (*IS-**) are only slowly displaced by wild type MG1655 with selection coefficients of roughly 0.05 per generation, consistent with a mild cost of IS deletion. When comparing RR452 (*IS*-) and RR530 (*IS-**) directly, the strain RR452 (*IS*-) was slightly more competitive than RR530 (*IS*-*).

In contrast the MDS42 (Blattner et al.) strain was displaced with selection coefficients of roughly 0.25 by both the MG1655 wild type, RR452 (*IS*-) and RR530 (*IS*-*). These results are independent of the marker each strain was labelled with.

These results indicate that the *IS*- and *IS*-* show high ecological strength compared to wild type and strains known in the art (e.g. Blattner et al., MDS42) and would thus be relatively resistant to contamination by other strains.

### Example 5: deletion of insertion sequences in the wbbL gene produced resistance to lysis by phages

### Methods

We tested the specificity of bacterial protection in strains RR452 (IS-) and RR443 (all IS fully deleted except IS5I which has been replaced by SEQ ID NO: 72, herein IS-*) from phages by *wbbL* restoration by performing efficiency of plating (EOP) assays with 20 different phages of *E. coli.* Here, bacterial lawns were prepared by overlaying agar plates with a layer of 50°C soft agar (0.7% agar, 0.01% glucose and 0.2 mM CalCl₂ in LB) containing bacterial cells in exponential growth phase. After solidification of the overlay, serial dilutions of phage in buffered saline (100 mM NaCl, 8 mM MgSO₄, 200mM Tris-CI pH = 7.5) were spotted onto the overlay surface and allowed to absorb. Plates were incubated overnight at 37°C and then phage plaques counted for the spotted dilution volumes.

### Results

We surprisingly found that complete deletion of IS5I in RR452 MG6155 *E. coli* caused full resistance against 13 of the 19 tested phages, strong resistance increases against further 3 of the 19 tested phages and no change of susceptibility against the remaining 3 phages (**Table 7**). Therefore, RR452 (IS-) showed a strong and broad-range resistance against bacteriophages. In contrast, RR443 (IS-*) was susceptible to these phages.

In the MG1655 strain tested herein, IS5I is present in the gene *wbbL.* WbbL protein catalyses an early step in the biosynthesis of the O-antigen in *E. coli,* and so the wild type MG1655 has a truncated O-antigen. Therefore, the phage resistance in IS- strains is explained by a restoration of the *wbbL* gene when deleting IS5I, and the resulting production of a full-length O-antigen on the external surface of the bacterial cell that prevents infection by phage. In contrast, the phage susceptibility in IS-* strains appears to be due to continuing to prevent functionality of the *wbbL* gene.

Whilst phage resistance is generally beneficial for the stability of bacterial cultures, resistance to P1 phage prevents the use of certain transduction techniques that are commonly used in bacterial genetics. We therefore found that it was beneficial to first replace IS5I with a stop codon (e.g. a double stop and frameshift mutation as in SEQ ID NO: 72) using the same technique described in **Example 1,** using oligomers Fp1 IS5I-stop (SEQ ID NO: 187) and Rp1 IS5I-stop (SEQ ID NO: 188). This allowed us to continue modifying the bacterial strain using generalized transduction with P1 phage (e.g., to remove spontaneous mutations or further mutate genes of interest). Once all desired genetic alterations have been achieved, we then fully deleted the double stop codon and frameshift sequence using the same technique described in **Example 1** using oligomers Fp1 IS5I (SEQ ID NO: 101) and Rp1 IS5I (SEQ ID NO: 102).

### SEQUENCES

| S E Q I D N O | is N a m e | Sequence |
|---|---|---|
| 1 | IN S L-2 | ATAAGCGCTAACTTAAGGGTTGAACCATCTGAAGAATGCGACGCCTCGGTGCCTCGTTAAGACGATGC CTCGCGTTCTTCAATTGCGTTTTGTAGGCTGTCAGGGATACTGTCCCACGAATGGCCACCTGTAAGCT CCAGATGACCATTTTGTTATTCTCCACAACGAGTTAGTTCTTCTTTTCGGATCCGGCACTTCTGGGG GGGAAATCCAGCGATGGCTGGATTATGTCGTCAATTAAAAATGCGGCGAGTAGATTAGCAAATATCCA CGCTTTCGCGAGTTCAGGTTCCTTTGCACGCAAAGCATCCAGGTGCAGCAAACTTTTGAGCCGCTTAA AAGCCAGTTCAATTTGCCATCGCAGACGGTAACAATCAGCCACTTGCTCTGCTGAATATTCATCTTCC GGTAATGATGTTAGCAATAGCACATGGCCCGCTGCTTCCAGCGTTTCCGCCTGAACTACTCGTCCTTT TCGACGATTCTCGCTGAGCAGTCGGGTTTTACTGATTAATGCTTTTTCGGGAGGAAGTGATACGGCAA TGAGACGTGCCGGAAAGGGAGCTCCGGCTTTTTTATTACCTGAATTGCCTATCATTACAGTGGTTTCA CCGTTCTTACCGCAATCCAGCCCGCGCAGAAAACCCATCATGTCAAAGCGCATTCCTTCTGCAGTTAA CCAGCGCAATCCTCGCCAGTGAACCCGGACGATATAATCAGCTTCTCCAAAAGCAAGTGAGCGGATAC ATTCGGGACGCGAACCGAATCCCCGGTCAGCAATGCGTATCTCGTCTGCCGTTTGCGCAAATCGGTCC AGCCGTTCAGCGTCTCTGCTGTCGGTTAGCTCAAAATCAGTGAACTGACAGGTATGAGGATCATATCC CATATGTAGTCGCCATTCAGCGCTGCCGCCCCCGGGCGCACTGATTGCTGTTCCATCGACAAGACGCA ATCTCTTTCCGCTTGTACAACCCGTAACTGCGGCGCGTACAGCAAGTGTTTGTGCGGCAAGTATGCCA AACCAGTCGGCGGCATTCCGCAGCCGCTTCAGGAGAGCCACGTCAGATAATGTTGCAACGTCATGGAG CTGAGCCCATGCAGTGACTTCACGTAATGACATCCCCCCGGGGCCGTAAGCCAGCCCCAGACGTAGCA GAGTTGCAGCATCACGAATTTCGCGGCGGCGGGTTAGAGCCCCGGCATTACGTGCCGAAGTATCCAGT TCTTCGGGCTTACCAATATGGGCCAGAATTGCTGACCAGTTATCGTGAGAGTAATTCATCGGCACGTT AAATCATATCAGGCGTAATACCACAACCCTTAAGTTAGCGCTTAT |
| 2 | IS 1 A | GGTAATGACTCCAACTTATTGATAGTGTTTTATGTTCAGATAATGCCCGATGACTTTGTCATGCAGCT CCACCGATTTTGAGAACGACAGCGACTTCCGTCCCAGCCGTGCCAGGTGCTGCCTCAGATTCAGGTTA TGCCGCTCAATTCGCTGCGTATATCGCTTGCTGATTACGTGCAGCTTTCCCTTCAGGCGGGATTCATA CAGCGGCCAGCCATCCGTCATCCATATCACCACGTCAAAGGGTGACAGCAGGCTCATAAGACGCCCCA GCGTCGCCATAGTGCGTTCACCGAATACGTGCGCAACAACCGTCTTCCGGAGACTGTCATACGCGTAA AACAGCCAGCGCTGGCGCGATTTAGCCCCGACATAGCCCCACTGTTCGTCCATTTCCGCGCAGACGAT GACGTCACTGCCCGGCTGTATGCGCGAGGTTACCGACTGCGGCCTGAGTTTTTTAAGTGACGTAAAAT CGTGTTGAGGCCAACGCCCATAATGCGGGCTGTTGCCCGGCATCCAACGCCATTCATGGCCATATCAA TGATTTTCTGGTGCGTACCGGGTTGAGAAGCGGTGTAAGTGAACTGCAGTTGCCATGTTTTACGGCAG TGAGAGCAGAGATAGCGCTGATGTCCGGCGGTGCTTTTGCCGTTACGCACCACCCCGTCAGTAGCTGA |
| | | ACAGGAGGGACAGCTGATAGAAACAGAAGCCACTGGAGCACCTCAAAAACACCATCATACACTAAATC AGTAAGTTGGCAGCATCACC |
| 3 | IS 1 B | GGTAATGACTCCAACTTATTGATAGTGTTTTATGTTCAGATAATGCCCGATGACTTTGTCATGCTGCT CCACCGATTTTGAGAACGACAGCGACTTCCGTCCCAGCCGTGCCAGGTGCTGCCTCAGATTCAGGTTA TGCCGCTCAATTCGCTGCGTATATCGCTTGCTGATTACGTGCAGCTTTCCCTTCAGGCGGGATTCATA CAGCGGCCAGCCATCCGTCATCCATATCACCACGTCAAAGGGTGACAGCAGGCTCATAAGACGCCCCA GCGTCGCCATAGTGCGTTCACCGAATACGTGCGCAACAACCGTCTTCCGGAGCCTGTCATACGCGTAA AACAGCCAGCGCTGGCGCGATTTAGCCCCGACGTATCCCCACTGTTCGTCCATTTCCGCGCAGACGAT GACGTCACTGCCCGGCTGTATGCGCGAGGTTACCGACTGCGGCCTGAGTTTTTTAAATGGCGGAAAAT CGTGTTGAGGCCAACGCCCATAATGCGGGCGGTTGCCCGGCATCCAACGCCATTCATGGCCATATCAA TGATTTTCTGGTGCGTACCGGGTTGAGAAGCGGTGTAAGTGAACTGCAGTTGCCATGTTTTACGGCAG TGAGAGCAGAGATAGCGCTGATGTCCGGCAGTGCTTTTGCCGTTACGCACCACCCCGTCAGTAGCTGA ACAGGAGGGACAGCTGATAGAAACAGAAGCCACTGGAGCACCTCAAAAACACCATCATACACTAAATC AGTAAGTTGGCAGCATCACC |
| 4 | IS 1 C | TGACTCCAACTTATTGATAGTGTTTTATGTTCAGATAATGCCCGATGACTTTGTCATGCTGCTCCACC GATTTTGAGAACGACAGCGACTTCCGTCCCAGCCGTGCCAGGTGCTGCCTCAGATTCAGGTTATGCCG CTCAATTCGCTGCGTATATCGCTTGCTGATTACGTGCAGCTTTCCCTTCAGGCGGGATTCATACAGCG GCCAGCCATCCGTCATCCATATCACCACGTCAAAGGGTGACAGCAGGCTCATAAGACGCCCCAGCGTC GCCATAGTGCGTTCACCGAATACGTGCGCAACAACCGTCTTCCGGAGCCTGTCATACGCGTAAAACAG CCAGCGCTGGCGCGATTTAGCCCCGACGTATCCCCACTGTTCGTCCATTTCCGCGCAGACGATGACGT CACTGCCCGGCTGTATGCGCGAGGTTACCGACTGCGGCCTGAGTTTTTTAAATGGCGGAAAATCGTGT TGAGGCCAACGCCCATAATGCGGGCGGTTGCCCGGCATCCAACGCCATTCATGGCCATATCAATGATT TTCTGGTGCGTACCGGGTTGAGAAGCGGTGTAAGTGAACTGCAGTTGCCATGTTTTACGGCAGTGAGA GCAGAGATAGCGCTGATGTCCGGCAGTGCTTTTGCCGTTACGCACCACCCCGTCAGTAGCTGAACAGG AGGGACAGCTGATAGAAACAGAAGCCACTGGAGCACCTCAAAAACACCATCATACACTAAATCAGTAA GTTGGCAGCATCACC |
| 5 | IS 1 D | GGTGATGCTGCCAACTTACTGATTTAGTGTATGATGGTGTTTTTGAGGTTCTCCAGTGGCTTCTGTTT CTATCAGCTGTCCCTCCTGTTCAGCTACTGACGGGGTGGTGCGTAACGGCAAAAGCACTGCCGGACAT CAGCGCTATCTCTGCTCTCACTGCCGTAAAACATGGCAACTGCAGTTCACTTACACCGCTTCTCAACC CGGTACGCACCAGAAAATCATTGATATGGCCATGAATGGCGTTGGATGCCGGGCAACAGCCCGCATTA TGGGCGTTGGCCTCAACACGATTTTCCGCCATTTAAAAAACTCAGGCCGCAGTCGGTAACCTCGCGCA TACAGCCGGGCAGTGACGTCATCGTCTGCGCGGAAATGGACGAACAGTGGGGATACGTCGGGGCTAAA TCGCGCCAGCGCTGGCTGTTTTACGCGTATGACAGGCTCCGGAAGACGGTTGTTGCGCACGTATTCGG TGAACGCACTATGGCGACGCTGGGGCGTCTTATGAGCCTGCTGTCACCCTTTGACGTGGTGATATGGA TGACGGATGGCTGGCCGCTGTATGAATCCCGCCTGAAGGGAAAGCTGCACGTAATCAGCAAGCGATAT ACGCAGCGAATTGAGCGGTATAACCTGAATCTGAGGCAGCACCTGGCACGGCTGGGACGGAAGTCGCT GTCGTTCTCAAAATCGGTGGAGCTGCATGACAAAGTCATCGGGCATTATCTGAACATAAAACACTATC AATAAGTTGGAGTCATTACC |
| 6 | IS 1 E | GGTGATGCTGCCAACTTACTGATTTAGTGTATGATGGTGTTTTTGAGGTGCTCCAGTGGCTTCTGTTT CTATCAGCTGTCCCTCCTGTTCAGCTACTGACGGGGTGGTGCGTAACGGCAAAAGCACCGCCGGACAT CAGCGCTATCTCTGCTCTCACTGCCGTAAAACATGGCAACTGCAGTTCACTTACACCGCTTCTCAACC CGGTACGCACCAGAAAATCATTGATATGGCCATGAATGGCGTTGGATGCCGGGCAACAGCCCGCATTA TGGGCGTTGGCCTCAACACGATTTTACGTCACTTAAAAAACTCAGGCCGCAGTCGGTAACCTCGCGCA TACAGCCGGGCAGTGACGTCATCGTCTGCGCGGAAATGGACGAACAGTGGGGCTATGTCGGGGCTAAA TCGCGCCAGCGCTGGCTGTTTTACGCGTATGACAGTCTCCGGAAGACGGTTGTTGCGCACGTATTCGG TGAACGCACTATGGCGACGCTGGGGCGTCTTATGAGCCTGCTGTCACCCTTTGACGTGGTGATATGGA TGACGGATGGCTGGCCGCTGTATGAATCCCGCCTGAAGGGAAAGCTGCACGTAATCAGCAAGCGATAT ACGCAGCGAATTGAGCGGCATAACCTGAATCTGAGGCAGCACCTGGCACGGCTGGGACGGAAGTCGCT GTCGTTCTCAAAATCGGTGGAGCTGCATGACAAAGTCATCGGGCATTATCTGAACATAAAACACTATC AATAAGTTGGAGTCATTACC |
| 7 | IS 1F | GGTAATGCTGCCAACTTACTGATTTAGTGTATGATGGTGATTTTAAGGTGCTTGCGTGGCTTCCATTT CCATCAGATGTCCTTCCTGCTCCGCTACTGAAGGCGTGGTGCGTAACGGCAAAAGCACTGCCGGACAT CAGCGCTATCTCTGCTCTCCTTGCCGTAAAACATGGCAACTACAGTTCACTTACACCGCCTCTCAGCC CGGTAAGCACCAGAAAATCATTGATATGGCCATGAATGGCGTCGGATGTCGCGCCAGTGCACGCATTA TGGGCGTTGGCCTCAACACGGTTTTACGTCACTTAAAAAACTCAGGCCGCAGTCGGTAACCTCGCGCA TACAACCGGGCAGTGATGTGATTGTCTGCGCTGAAATGGACGAACAGTGGGGCTACGTCGGTGCTAAA TCACGTCAGCGCTGGCTGTTTTACGCGTATGACAGGATACGGAGGACGGTTGTGGCGCACGTCTTCGG TGAACGCACTCTGGCCACACTGGAGCGTCTTCTGAGCCTGCTGTCGGCCTTTGAGGTCGTGGTATAGA TGACGGATGGCTGGCCGCTGTATGAATCACGCCTGAAGGGAAAGCTGCACGTTATCAGCAAGCGTTAC ACTCAGCGCATTGAGCGACATAATCTGAATCTGAGACAACATCTGGCAAGGCTGGGACGGAAGTTACT GTCGTTCTCAAAATCGGTGGAGTTGCATGACAAGGTCATCGGGCATTATCTGAACATAAAACACTATC AGTAAGTTGGAGTCATTACC |
| 8 | IS 1 H | GGTAATGACTCCAACTTATTGATAGTGTTTTATGTTCAGATAATGCCCGATGACTTTGTCATGCAGCT CCACCGATTTTGAGAACGACAGCGACTTCCGTCCCAGCCGTGCCAGGTGCTGCCTCAGATTCAGGTTA TGCCGCTCAATTCGCTGCGTATATCGCTTGCTGATTACGTGCAGCTTTCCCTTCAGGCGGGATTCATA CAGCGGCCAGCCATCCGTCATCCATATCACCACGTCAAAGGGTGACAGCAGGCTCATAAGACGCCCCA GCGTCGCCATAGTGCGTTCACCGAATACGTGCGCAACAACCGTCTTCCGGAGACTGTCATACGCGTAA AACAGCCAGCGCTGGCGCGATTTAGCCCCGACATAGCCCCACTGTTCGTCCATTTCCGCGCAGACGAT GACGTCACTGCCCGGCTGTATGCGCGAGGTTACCGACTGCGGCCTGAGTTTTTTAAGTGACGTAAAAT CGTGTTGAGGCCAACGCCCATAATGCGGGCTGTTGCCCGGCATCCAACGCCATTCATGGCCATATCAA TGATTTTCTGGTGCGTACCGGGTTGAGAAGCGGTGTAAGTGAACTGCAGTTGCCATGTTTTACGGCAG TGAGAGCAGAGATAGCGCTGATGTCCGGCGGTGCTTTTGCCGTTACGCACCACCCCGTCAGTAGCTGA ACAGGAGGGACAGCTGATAGAAACAGAAGCCACTGGAGCACCTCAAAAACACCATCATACACTAAATC AGTAAGTTGGCAGCATCACC |
| 9 | IS 5 A | CTCAGTCATTTCATTTCTTCATGTTTGAGCCGATTTTTCTCCCGTAAATGCCTTGAATCAGCCTATT TAGACCGTTTCTTCGCCATTTAAGGCGTTATCCCCAGTTTTTAGTGAGATCTCTCCCACTGACGTATC ATTTGGTCCGCCCGAAACAGGTTGGCCAGCGTGAATAACATCGCCAGTTGGTTATCGTTTTTCAGCAA |
| | | CCCCTTGTATCTGGCTTTCACGAAGCCGAACTGTCGCTTGATGATGCGAAATGGGTGCTCCACCCTGG CCCGGATGCTGGCTTTCATGTATTCGATGTTGATGGCCGTTTTGTTCTTGCGTGGATGCTGTTTCAAG GTTCTTACCTTGCCGGGGCGCTCGGCGATCAGCCAGTCCACATCCACCTCGGCCAGCTCCTCGCGCTG TGGCGCCCCTTGGTAGCCGGCATCGGCTGAGACAAATTGCTCCTCTCCATGCAGCAGATTACCCAGCT GATTGAGGTCATGCTCGTTGGCCGCGGTGGTGACCAGGCTGTGGGTCAGGCCACTCTTGGCATCGACA CCAATGTGGGCCTTCATGCCAAAGTGCCACTGATTGCCTTTCTTGGTCTGATGCATCTCCGGATCGCG TTGCTGCTCTTTGTTCTTGGTCGAGCTGGGTGCCTCAATGATGGTGGCATCGACCAAGGTGCCTTGAG TCATCATGACGCCTGCTTCGGCCAGCCAGCGATTGATGGTCTTGAACAATTGGCGGGCCAGTTGATGC TGCTCCAGCAGGTGGCGGAAATTCATGATGGTGGTGCGGTCCGGCAAGGCGCTATCCAGGGATAACCG GGCAAACAGACGCATGGAGGCGATTTCGTACAGAGCATCTTCCATCGCGCCATCGCTCAGGTTGTACC AATGCTGCATGCAGTGAATGCGTAGCATGGTTTCCAGCGGATAAGGTCGCCGGCCATTACCAGCCTTG GGGTAAAACGGCTCGATGACTTCCACCATGTTTTGCCATGGCAGAATCTGCTCCATGCGGGACAAGAA AATCTCTTTTCTGGTCTGACGGCGCTTACTGCTGAATTCACTGTCGGCGAAGGTAAGTTGATGACTCA TGATGAACCCTGTTCTATGGCTCCAGATGACAAACATGATCTCATATCAGGGACTTGTTCGCACCTTC C |
| 1 0 | IS 5 B | GGAAGGTGCGAATAAGCGGGGAAATTCTTCTCGGCTGACTCAGTCATTTCATTTCTTCATGTTTGAGC CGATTTTTTCTCCCGTAAATGCCTTGAATCAGCCTATTTAGACCGTTTCTTCGCCATTTAAGGCGTTA TCCCCAGTTTTTAGTGAGATCTCTCCCACTGACGTATCATTTGGTCCGCCCGAAACAGGTTGGCCAGC GTGAATAACATCGCCAGTTGGTTATCGTTTTTCAGCAACCCCTTGTATCTGGCTTTCACGAAGCCGAA CTGTCGCTTGATGATGCGAAATGGGTGCTCCACCCTGGCCCGGATGCTGGCTTTCATGTATTCGATGT TGATGGCCGTTTTGTTCTTGCGTGGATGCTGTTTCAAGGTTCTTACCTTGCCGGGGCGCTCGGCGATC AGCCAGTCCACATCCACCTCGGCCAGCTCCTCGCGCTGTGGCGCCCCTTGGTAGCCGGCATCGGCTGA GACAAATTGCTCCTCTCCATGCAGCAGATTACCCAGCTGATTGAGGTCATGCTCGTTGGCCGCGGTGG TGACCAGGCTGTGGGTCAGGCCACTCTTGGCATCGACACCAATGTGGGCCTTCATGCCAAAGTGCCAC TGATTGCCTTTCTTGGTCTGATGCATCTCCGGATCGCGTTGCTGCTCTTTGTTCTTGGTCGAGCTGGG TGCCTCAATGATGGTGGCATCGACCAAGGTGCCTTGAGTCATCATGACGCCTGCTTCGGCCAGCCAGC GATTGATGGTCTTGAACAATTGGCGGGCCAGTTGATGCTGCTCCAGCAGGTGGCGGAAATTCATGATG GTGGTGCGGTCCGGCAAGGCGCTATCCAGGGATAACCGGGCAAACAGACGCATGGAGGCGATTTCGTA CAGAGCATCTTCCATCGCGCCATCGCTCAGGTTGTACCAATGCTGCATGCAGTGAATGCGTAGCATGG TTTCCAGCGGATAAGGTCGCCGGCCATTACCAGCCTTGGGGTAAAACGGCTCGATGACTTCCACCATG TTTTGCCATGGCAGAATCTGCTCCATGCGGGACAAGAAAATCTCTTTTCTGGTCTGACGGCGCTTACT GCTGAATTCACTGTCGGCGAAGGTAAGTTGATGACTCATGATGAACCCTGTTCTATGGCTCCAGATGA CAAACATGATCTCATATCAGGGACTTGTTCGCACCTTCC |
| 11 | IS 5 D | GGAAGGTGCGAATAAGCGGGGAAATTCTTCTCGGCTGACTCAGTCATTTCATTTCTTCATGTTTGAGC CGATTTTTTCTCCCGTAAATGCCTTGAATCAGCCTATTTAGACCGTTTCTTCGCCATTTAAGGCGTTA TCCCCAGTTTTTAGTGAGATCTCTCCCACTGACGTATCATTTGGTCCGCCCGAAACAGGTTGGCCAGC GTGAATAACATCGCCAGTTGGTTATCGTTTTTCAGCAACCCCTTGTATCTGGCTTTCACGAAGCCGAA CTGTCGCTTGATGATGCGAAATGGGTGCTCCACCCTGGCCCGGATGCTGGCTTTCATGTATTCGATGT TGATGGCCGTTTTGTTCTTGCGTGGATGCTGTTTCAAGGTTCTTACCTTGCCGGGGCGCTCGGCGATC |
| | | AGCCAGTCCACATCCACCTCGGCCAGCTCCTCGCGCTGTGGCGCCCCTTGGTAGCCGGCATCGGCTGA GACAAATTGCTCCTCTCCATGCAGCAGATTACCCAGCTGATTGAGGTCATGCTCGTTGGCCGCGGTGG TGACCAGGCTGTGGGTCAGGCCACTCTTGGCATCGACACCAATGTGGGCCTTCATGCCAAAGTGCCAC TGATTGCCTTTCTTGGTCTGATGCATCTCCGGATCGCGTTGCTGCTCTTTGTTCTTGGTCGAGCTGGG TGCCTCAATGATGGTGGCATCGACCAAGGTGCCTTGAGTCATCATGACGCCTGCTTCGGCCAGCCAGC GATTGATGGTCTTGAACAATTGGCGGGCCAGTTGATGCTGCTCCAGCAGGTGGCGGAAATTCATGATG GTGGTGCGGTCCGGCAAGGCGCTATCCAGGGATAACCGGGCAAACAGACGCATGGAGGCGATTTCGTA CAGAGCATCTTCCATCGCGCCATCGCTCAGGTTGTACCAATGCTGCATGCAGTGAATGCGTAGCATGG TTTCCAGCGGATAAGGTCGCCGGCCATTACCAGCCTTGGGGTAAAACGGCTCGATGACTTCCACCATG TTTTGCCATGGCAGAATCTGCTCCATGCGGGACAAGAAAATCTCTTTTCTGGTCTGACGGCGCTTACT GCTGAATTCACTGTCGGCGAAGGTAAGTTGATGACTCATGATGAACCCTGTTCTATGGCTCCAGATGA CAAACATGATCTCATATCAGGGACTTGTTCGCACCTTCC |
| 1 2 | IS 5F | GGAAGGTGCGAACAAGTCCCTGATATGAGATCATGTTTGTCATCTGGAGCCATAGAACAGGGTTCATC ATGAGTCATCAACTTACCTTCGCCGACAGTGAATTCAGCAGTAAGCGCCGTCAGACCAGAAAAGAGAT TTTCTTGTCCCGCATGGAGCAGATTCTGCCATGGCAAAACATGGTGGAAGTCATCGAGCCGTTTTACC CCAAGGCTGGTAATGGCCGGCGACCTTATCCGCTGGAAACCATGCTACGCATTCACTGCATGCAGCAT TGGTACAACCTGAGCGATGGCGCGATGGAAGATGCTCTGTACGAAATCGCCTCCATGCGTCTGTTTGC CCGGTTATCCCTGGATAGCGCCTTGCCGGACCGCACCACCATCATGAATTTCCGCCACCTGCTGGAGC AGCATCAACTGGCCCGCCAATTGTTCAAGACCATCAATCGCTGGCTGGCCGAAGCAGGCGTCATGATG ACTCAAGGCACCTTGGTCGATGCCACCATCATTGAGGCACCCAGCTCGACCAAGAACAAAGAGCAGCA ACGCGATCCGGAGATGCATCAGACCAAGAAAGGCAATCAGTGGCACTTTGGCATGAAGGCCCACATTG GTGTCGATGCCAAGAGTGGCCTGACCCACAGCCTGGTCACCACCGCGGCCAACGAGCATGACCTCAAT CAGCTGGGTAATCTGCTGCATGGAGAGGAGCAATTTGTCTCAGCCGATGCCGGCTACCAAGGGGCGCC ACAGCGCGAGGAGCTGGCCGAGGTGGATGTGGACTGGCTGATCGCCGAGCGCCCCGGCAAGGTAAGAA CCTTGAAACAGCATCCACGCAAGAACAAAACGGCCATCAACATCGAATACATGAAAGCCAGCATCCGG GCCAGGGTGGAGCACCCATTTCGCATCATCAAGCGACAGTTCGGCTTCGTGAAAGCCAGATACAAGGG GTTGCTGAAAAACGATAACCAACTGGCGATGTTATTCACGCTGGCCAACCTGTTTCGGGCGGACCAAA TGATACGTCAGTGGGAGAGATCTCACTAAAAACTGGGGATAACGCCTTAAATGGCGAAGAAACGGTCT AAATAGGCTGATTCAAGGCATTTACGGGAGAAAAAATCGGCTCAAACATGAAGAAATGAAATGACTGA GTCAGCCGAGAAGAATTTCCCCGCTTATTCGCACCTTCC |
| 1 3 | IS 5 H | GGAAGGTGCGAATAAGTGGGGAAATTCTTCTCGGCTGACTCAGTCATTTCATTTCTTCATGTTTGAGC CGATTTTTTCTCCCGTAAATGCCTTGAATCAGCCTATTTAGACCGTTTCTTCGCCATTTAAGGCGTTA TCCCCAGTTTTTAGTGAGATCTCTCCCACTGACGTATCATTTGGTCCGCCCGAAACAGGTTGGCCAGC GTGAATAACATCGCCAGTTGGTTATCGTTTTTCAGCAACCCCTTGTATCTGGCTTTCACGAAGCCGAA CTGTCGCTTGATGATGCGAAATGGGTGCTCCACCTTGGCCCGGATGCTGGCTTTCATGTATTCGATGT TGATGGCCGTTTTGTTCTTGCGTGGATGCTGTTTCAAGGTTCTTACCTTGCCGGGGCGCTCGGCGATC AGCCAGTCCACATCCACCTCGGCCAGCTCCTCGCGCTGTGGCGCCCCTTGGTAGCCGGCATCGGCTGA GACAAATTGCTCCTCTCCATGCAGCAGATTACCCAGCTGATTGAGGTCATGCTCGTTGGCCGCGGTGG TGACTAGGCTGTGGGTCAGGCCGCTCTTGGCATCGACACCAATGTGGGCCTTCATGCCAAAGTGCCAC |
| | | TGATTGCCTTTCTTGGTCTGATGCATCTCCGGATCGCGTTGCTGCTCTTTGTTCTTGGTCGAGCTGGG TGCCTCAATGATGGTGGCATCGACCAAGGTGCCTTGAGTCATCATGACGCCTGCTTCGGCCAGCCAGC GATTGATGGTCTTGAACAATTGGCGGGCCAGTTGATGCTGCTCCAGCAGGTGGCGGAAATTCATGATG GTGGTGCGGTCCGGCAAGGCGCTATCCAGGGATAACCGGGCAAACAGACGCATGGAGGCGATTTCGTA CAGAGCATCTTCCATCGCGCCATCGCTCAGGTTGTACCAATGCTGCATGCAGTGAATGCGTAGCATGG TTTCCAGCGGATAAGGTCGCCGGCCATTACCAGCCTTGGGGTAAAACGGCTCGATGACTTCCACCATG TTTTGCCATGGCAGAATCTGCTCCATGCGGGACAAGAAAATCTCTTTTCTGGTCTGACGGCGCTTACT GCTGAATTCACTGTCGGCGAAGGTAAGTTGATGACTCATGATGAACCCTGTTCCATGGCTCCAGATGA CAAACATGATCTCATATCAGGGACTTGTTCGCACCTTCC |
| 1 4 | IS 5I | GGAAGGTGCGAATAAGCGGGGAAATTCTTCTCGGCTGACTCAGTCATTTCATTTCTTCATGTTTGAGC CGATTTTTTCTCCCGTAAATGCCTTGAATCAGCCTATTTAGACCGTTTCTTCGCCATTTAAGGCGTTA TCCCCAGTTTTTAGTGAGATCTCTCCCACTGACGTATCATTTGGTCCGCCCGAAACAGGTTGGCCAGC GTGAATAACATCGCCAGTTGGTTATCGTTTTTCAGCAACCCCTTGTATCTGGCTTTCACGAAGCCGAA CTGTCGCTTGATGATGCGAAATGGGTGCTCCACCCTGGCCCGGATGCTGGCTTTCATGTATTCGATGT TGATGGCCGTTTTGTTCTTGCGTGGATGCTGTTTCAAGGTTCTTACCTTGCCGGGGCGCTCGGCGATC AGCCAGTCCACATCCACCTCGGCCAGCTCCTCGCGCTGTGGCGCCCCTTGGTAGCCGGCATCGGCTGA GACAAATTGCTCCTCTCCATGCAGCAGATTACCCAGCTGATTGAGGTCATGCTCGTTGGCCGCGGTGG TGACCAGGCTGTGGGTCAGGCCACTCTTGGCATCGACACCAATGTGGGCCTTCATGCCAAAGTGCCAC TGATTGCCTTTCTTGGTCTGATGCATCTCCGGATCGCGTTGCTGCTCTTTGTTCTTGGTCGAGCTGGG TGCCTCAATGATGGTGGCATCGACCAAGGTGCCTTGAGTCATCATGACGCCTGCTTCGGCCAGCCAGC GATTGATGGTCTTGAACAATTGGCGGGCCAGTTGATGCTGCTCCAGCAGGTGGCGGAAATTCATGATG GTGGTGCGGTCCGGCAAGGCGCTATCCAGGGATAACCGGGCAAACAGACGCATGGAGGCGATTTCGTA CAGAGCATCTTCCATCGCGCCATCGCTCAGGTTGTACCAATGCTGCATGCAGTGAATGCGTAGCATGG TTTCCAGCGGATAAGGTCGCCGGCCATTACCAGCCTTGGGGTAAAACGGCTCGATGACTTCCACCATG TTTTGCCATGGCAGAATCTGCTCCATGCGGGACAAGAAAATCTCTTTTCTGGTCTGACGGCGCTTACT GCTGAATTCACTGTCGGCGAAGGTAAGTTGATGACTCATGATGAACCCTGTTCTATGGCTCCAGATGA CAAACATGATCTCATATCAGGGACTTGTTCGCACCTTCC |
| 1 5 | IS 5 K | GGAAGGTGCGAATAAGCGGGGAAATTCTTCTCGGCTGACTCAGTCATTTCATTTCTTCATGTTTGAGC CGATTTTTTCTCCCGTAAATGCCTTGAATCAGCCTATTTAGACCGTTTCTTCGCCATTTAAGGCGTTA TCCCCAGTTTTTAGTGAGATCTCTCCCACTGACGTATCATTTGGTCCGCCCGAAACAGGTTGGCCAGC GTGAATAACATCGCCAGTTGGTTATCGTTTTTCAGCAACCCCTTGTATCTGGCTTTCACGAAGCCGAA CTGTCGCTTGATGATGCGAAATGGGTGCTCCACCCTGGCCCGGATGCTGGCTTTCATGTATTCGATGT TGATGGCCGTTTTGTTCTTGCGTGGATGCTGTTTCAAGGTTCTTACCTTGCCGGGGCGCTCGGCGATC AGCCAGTCCACATCCACCTCGGCCAGCTCCTCGCGCTGTGGCGCCCCTTGGTAGCCGGCATCGGCTGA GACAAATTGCTCCTCTCCATGCAGCAGATTACCCAGCTGATTGAGGTCATGCTCGTTGGCCGCGGTGG TGACCAGGCTGTGGGTCAGGCCACTCTTGGCATCGACACCAATGTGGGCCTTCATGCCAAAGTGCCAC TGATTGCCTTTCTTGGTCTGATGCATCTCCGGATCGCGTTGCTGCTCTTTGTTCTTGGTCGAGCTGGG TGCCTCAATGATGGTGGCATCGACCAAGGTGCCTTGAGTCATCATGACGCCTGCTTCGGCCAGCCAGC GATTGATGGTCTTGAACAATTGGCGGGCCAGTTGATGCTGCTCCAGCAGGTGGCGGAAATTCATGATG |
| | | GTGGTGCGGTCCGGCAAGGCGCTATCCAGGGATAACCGGGCAAACAGACGCATGGAGGCGATTTCGTA CAGAGCATCTTCCATCGCGCCATCGCTCAGGTTGTACCAATGCTGCATGCAGTGAATGCGTAGCATGG TTTCCAGCGGATAAGGTCGCCGGCCATTACCAGCCTTGGGGTAAAACGGCTCGATGACTTCCACCATG TTTTGCCATGGCAGAATCTGCTCCATGCGGACAAGAAAATCTCTTTTCTGGTCTGACGGCGCTTACT GCTGAATTCACTGTCGGCGAAGGTAAGTTGATGACTCATGATGAACCCTGTTCTATGGCTCCAGATGA CAAACATGATCTCATATCAGGGACTTGTTCGCACCTTCC |
| 1 6 | IS 5L O | GAGTCATCAACTTACCTTCGCCGACAGTGAATTCAGCAGTAAGCGCCGTCAGACCAGAAAAGAGATTT TCTTGTCCCGCATGGAGCAGATTCTGCCATGGCAAAACATGGTGGAAGTCATCGAGCCGTTTTACCCC AAGGCTGGTAATGGCCGGCGACCTTATCCGCTGGAAACCATGCTACGCATTCACTGCATGCAGCATTG GTACAACCTGAGCGATGGCGCGATGGAAGATGCTCTGTACGAAATCGCCTCCATGCGTCTGTTTGCCC GGTTATCCCTGGATAGCGCCTTGCCGGACCGCACCACCATCATGAATTTCCGCCACCTGCTGGAGCAG CATCAACTGGCCCGCCAATTGTTCAAGACCATCAATCGCTGGCTGGCCGAAGCAGGCGTCATGATGAC TCAAGGCACCTTGGTCGATGCCACCATCATTGAGGCACCCAGCTCGACCAAGAACAAAGAGCAGCAAC GCGATCCGGAGATGCATCAGACCAAGAAAGGCAATCAGTGGCACTTTGGCATGAAGGCCCACATTGGT GTCGATGCCAAGAGTGGCCTGACCCACAGCCTGGTCACCACCGCGGCCAACGAGCATGACCTCAATCA GCTGGGTAATCTGCTGCATGGAGAGGAGCAATTTGTCTCAGCCGATGCCGGCTACCAAGGGGCGCCAC AGCGCGAGGAGCTGGCCGAGGTGGATGTGGACTGGCTGATCGCCGAGCGCCCCGGCAAGGTAAGAACC TTGAAACAGCATCCACGCAAGAACAAAACGGCCATCAACATCGAATACATGAAAGCCAGCATCCGGGC CAGGGTGGAGCACCCATTTCGCATCATCAAGCGACAGTTCGGCTTCGTGAAAGCCAGATACAAGGGGT TGCTGAAAAACGATAACCAACTGGCGATGTTATTCACGCTGGCCAACCTGTTTCGGGCGGACCAAATG ATACGTCAGTGGGAGAGATCTCACTAAAAACTGGGGATAACGCCTTAAATGGCGAAGAAACGGTCTAA ATAGGCTGATTCAAGGCATTTACGGGAGAAAAAATCGGCTCAAACATGAAGAAATGAAATGACTGAGT CAGCCGAGAAGAATTTCCCCGCTTATTCGCACCTTCC |
| 1 7 | IS 5 R | GGAAGGTGCGAATAAGCGGGGAAATTCTTCTCGGCTGACTCAGTCATTTCATTTCTTCATGTTTGAGC CGATTTTTTCTCCCGTAAATGCCTTGAATCAGCCTATTTAGACCGTTTCTTCGCCATTTAAGGCGTTA TCCCCAGTTTTTAGTGAGATCTCTCCCACTGACGTATCATTTGGTCCGCCCGAAACAGGTTGGCCAGC GTGAATAACATCGCCAGTTGGTTATCGTTTTTCAGCAACCCCTTGTATCTGGCTTTCACGAAGCCGAA CTGTCGCTTGATGATGCGAAATGGGTGCTCCACCCTGGCCCGGATGCTGGCTTTCATGTATTCGATGT TGATGGCCGTTTTGTTCTTGCGTGGATGCTGTTTCAAGGTTCTTACCTTGCCGGGGCGCTCGGCGATC AGCCAGTCCACATCCACCTCGGCCAGCTCCTCGCGCTGTGGCGCCCCTTGGTAGCCGGCATCGGCTGA GACAAATTGCTCCTCTCCATGCAGCAGATTACCCAGCTGATTGAGGTCATGCTCGTTGGCCGCGGTGG TGACCAGGCTGTGGGTCAGGCCACTCTTGGCATCGACACCAATGTGGGCCTTCATGCCAAAGTGCCAC TGATTGCCTTTCTTGGTCTGATGCATCTCCGGATCGCGTTGCTGCTCTTTGTTCTTGGTCGAGCTGGG TGCCTCAATGATGGTGGCATCGACCAAGGTGCCTTGAGTCATCATGACGCCTGCTTCGGCCAGCCAGC GATTGATGGTCTTGAACAATTGGCGGGCCAGTTGATGCTGCTCCAGCAGGTGGCGGAAATTCATGATG GTGGTGCGGTCCGGCAAGGCGCTATCCAGGGATAACCGGGCAAACAGACGCATGGAGGCGATTTCGTA CAGAGCATCTTCCATCGCGCCATCGCTCAGGTTGTACCAATGCTGCATGCAGTGAATGCGTAGCATGG TTTCCAGCGGATAAGGTCGCCGGCCATTACCAGCCTTGGGGTAAAACGGCTCGATGACTTCCACCATG TTTTGCCATGGCAGAATCTGCTCCATGCGGGACAAGAAAATCTCTTTTCTGGTCTGACGGCGCTTACT |
| | | GCTGAATTCACTGTCGGCGAAGGTAAGTTGATGACTCATGATGAACCCTGTTCTATGGCTCCAGATGA CAAACATGATCTCATATCAGGGACTTGTTCGCACCTTCC |
| 1 8 | IS 5T | GGAAGGTGCGAATAAGCGGGGAAATTCTTCTCGGCTGACTCAGTCATTTCATTTCTTCATGTTTGAGC CGATTTTTTCTCCCGTAAATGCCTTGAATCAGCCTATTTAGACCGTTTCTTCGCCATTTAAGGCGTTA TCCCCAGTTTTTAGTGAGATCTCTCCCACTGACGTATCATTTGGTCCGCCCGAAACAGGTTGGCCAGC GTGAATAACATCGCCAGTTGGTTATCGTTTTTCAGCAACCCCTTGTATCTGGCTTTCACGAAGCCGAA CTGTCGCTTGATGATGCGAAATGGGTGCTCCACCCTGGCCCGGATGCTGGCTTTCATGTATTCGATGT TGATGGCCGTTTTGTTCTTGCGTGGATGCTGTTTCAAGGTTCTTACCTTGCCGGGGCGCTCGGCGATC AGCCAGTCCACATCCACCTCGGCCAGCTCCTCGCGCTGTGGCGCCCCTTGGTAGCCGGCATCGGCTGA GACAAATTGCTCCTCTCCATGCAGCAGATTACCCAGCTGATTGAGGTCATGCTCGTTGGCCGCGGTGG TGACCAGGCTGTGGGTCAGGCCACTCTTGGCATCGACACCAATGTGGGCCTTCATGCCAAAGTGCCAC TGATTGCCTTTCTTGGTCTGATGCATCTCCGGATCGCGTTGCTGCTCTTTGTTCTTGGTCGAGCTGGG TGCCTCAATGATGGTGGCATCGACCAAGGTGCCTTGAGTCATCATGACGCCTGCTTCGGCCAGCCAGC GATTGATGGTCTTGAACAATTGGCGGGCCAGTTGATGCTGCTCCAGCAGGTGGCGGAAATTCATGATG GTGGTGCGGTCCGGCAAGGCGCTATCCAGGGATAACCGGGCAAACAGACGCATGGAGGCGATTTCGTA CAGAGCATCTTCCATCGCGCCATCGCTCAGGTTGTACCAATGCTGCATGCAGTGAATGCGTAGCATGG TTTCCAGCGGATAAGGTCGCCGGCCATTACCAGCCTTGGGGTAAAACGGCTCGATGACTTCCACCATG TTTTGCCATGGCAGAATCTGCTCCATGCGGGACAAGAAAATCTCTTTTCTGGTCTGACGGCGCTTACT GCTGAATTCACTGTCGGCGAAGGTAAGTTGATGACTCATGATGAACCCTGTTCTATGGCTCCAGATGA CAAACATGATCTCATATCAGGGACTTGTTCGCACCTTCC |
| 1 9 | IS 5 Y | GGAAGGTGCGAATAAGCAGGTCATTTCTTCCCAAGCTGACTCGCTGATTAAAATTTCGCGGATCTGGG CCGATTTTTTCCCGCAAACACATCGAATCAGCCTATTTAGGCTATTTTTTCCACCATTTCTGGCGTT ATTTCCGGTTTTTACTGAGATCTCTCCCACTGACGTATCATTTGGTCCACCCGAAACAGGTTGGCCAG GGTGAATAACATCGCCAGTTGGTTATCGTTTTTCAGCAGCCCCTTGTATCTGGCTTTCACGAAGCCGA ACTGCCGCTTGATGATGCGAAACGGGTGCTCCACCCTGGCACGGATGCTGGCTTTCATGTATTCGATG TTGATGGCCGTTTTGTTCTTGCGCGGATTCTGCTTCAAGGTTTTTACCTTGCCGGGACGCTCGGCGAT CAGCCAGTCCACATCCACCTCGGCCAGCTCCTCGCGCTGTGGCGCTCCTTGGTAGCCGGCATCGGCTG AGACAAATTGCTCCTCTCCATGAAGCAGATTACCCAGCTGATTGAGGTCATGCTCGTTGGCCGCGGTG GTGACCAGGCTGTGGGTCAGGCCACTCTTGGCATCGACACCAATGTGGGCCTTCATGCCAAAGTGCCA CTGATTGCCTTTCTTGGTCTGATGCATCTCCGGATCGCGTTGCTGCTCTTTGTTCTTGGTAGAGCTGG GTGCCTCAATGATGGTGGCATCCACCAAAGTGCCTTGGGTCATCATGACGCCTGCTTCGGCCAGCCAG CGATTGATGGTCTTGAACAATTGACGGGCCAGTTGATGCTGCTCGAGCAGGTGGCGGAAATTCATGAT GGTGGTGCGATCCGGCAGGGCGCTATCCAGGGATAATCGGGCAAACAGGCGCATGGAGGCGATTTCGT ACAGGGCATCTTCCATGGCACCGTCGCTCAGGTTGTACCAATGCTGCATGCAGTGAATACGCAGCATG GTCTCCAGCGGATAGGGCCGTCGGCCATTGCCCGCCTTGGGATAAAACGGCTCGATGACAGCGGTCAT ATTCTGCCATGGCAGAATCTGCTCCATGCGGGAGAGGAAAATCTCTTTTCGGGTCTGACGGCGCTTAG TGCTGAATTCACTATCGGCGAAGGTGAGTTGATGGCTCATGATGTCCCTCTGGGATGCGCTCCGGATG AATATGATGATCTCATATCAGGAACTTGTTCGCACCTTCC |
| 2 0 | IS 4 | TAATGCCAGTCAGTTAAGCAACTGACTGGCTCTTTTTCGGGGCTGTGGGGTATTTCCAGGGCCTCTCC TTTACCACTCTCGGGAAGGCCCTTTCCCTTCTTGTCGGTAATTTCACAAGTTGTCCCATACTTGCAAG ATCGCGCATCAGCTCCGGTATACGTCCCGGTGAAGCGCCCTGCAATGTCATCAGCATTCTCATCACCA TTCCGCATGATTCTGAGAAACTCAGTTGATTCGGCCAGTAACCTTTCAGATGTTCCGCCATTTTAATC ATCTGATATCTCACCAGATTATAAGCCAGTAAGACACCCCACAGCTCTTGCTCCACAAGCTCCGGCTT TTTACTTCTCAGCGTCAGCCTGCTCCGTTGCATCGTCTGTTTTATCTCCCTGTATCCCAGTTCGATTT CCCAGCGATGACTGTACAGATCCCCCATTTCTCCTCCGGGGAAGCGCATGGCGTCCGTCATCGACGTC AGCAGATGGCAGACTTTTCCTTTGCGCGTCACGGTCAGCAGGCGGGCAGTCACTTCATTTCCCAGTCC CGGCCACTTTTTTCGTGCCTGCGGGCTGGTTTTCAGCTTCACCAGATGATCGCCTTTACCCAGTTTTC TGATCTCTTCATATTGCGCTCCCTTTCTGAGAGGTATCATCCAGTGGCGGTGTTCTCCCGCCAGGCTC CAGGCATTTAACAGTCCCAGTGAGTAATAACCTTTATCCATTAACGTCAGAGTGTTATCGCCGGTTTG TTCTATAAGTTGCTCAGCAAGCTCATTTTCGCTGTTCTTCATCGTGCCGAAGGCTGCAGCCGTCAGCA GATGGCTGGTCAGTTCCATCTGGCAGACCATTTTGACCTGCGGGTAGAGCGCCGGGTTCCCGGCATGT GTCTGGCGGGGGAAGGCTGCATCGTTCTCTGGTGTATCCGGTGTGCGCCAGAACACACCATCGATGGC CAGCAGGGTCAGGCCGCACCAGTGCGGATGCGGCGTGGCGTTATGCCAGAGCTGCGCTGTTTTCGTGA ACACGCGGCGGACAGCCTCACTTCCCAGGCGCTGGCGGGCCTGAATAACGGCACTGGGGGCAACGAAG GGGCGATTGCCCGGCAGCATGATGTCCAGGCGATTCACAATCTGGTGAAGAGGTTCTTTACGCTCAAG CGCCATGCCAACAATACACCAGACCATCATTTCGAGGGGAAGACGGCGCTTGCGTAGCGTTACAGTAC CTGATTCGGCAAGGCAACGAGAGATGAGTTCGGGGTCGAGGTAATCCCCCAGAGAAGTCAGTGGGTTA CGCAGAGAATCGTAACGGGATACCAGATCAAGAGCCTGTCCAATGTGCATAAAAAAATCCGGAAACAA GTGAGCGTTTCCGGATTCTTACACAGCCACTGGATCGGTCAACTGATCCTTAACTGATCGGCATTA |
| 2 1 | IS 2 A | TAGACTGGCCCCCTGAATCTCCAGACAACCAATATCACTTAAATAAGTGATAGTCTTAATACTAGTTT TTAGACTAGTCATTGGAGAACAGATGATTGATGTCTTAGGGCCGGAGAAACGCAGACGGCGTACCACA CAGGAAAAGATCGCAATTGTTCAGCAGAGCTTTGAACCGGGGATGACGGTCTCCCTCGTTGCCCGGCA ACATGGTGTAGCAGCCAGCCAGTTATTTCTCTGGCGTAAGCAATACCAGGAAGGAAGTCTTACTGCTG TCGCCGCCGGAGAACAGGTTGTTCCTGCCTCTGAACTTGCTGCCGCCATGAAGCAGATTAAAGAACTC CAGCGCCTGCTCGGCAAGAAAACGATGGAAAATGAACTCCTCAAAGAAGCCGTTGAATATGGACGGGC AAAAAAGTGGATAGCGCACGCGCCCTTATTGCCCGGGGATGGGGAGTAAGCTTAGTCAGCCGTTGTCT CCGGGTGTCGCGTGCGCAGTTGCACGTCATTCTCAGACGAACCGATGACTGGATGGATGGCCGCCGCA GTCGTCACACTGATGATACGGATGTGCTTCTCCGTATACACCATGTTATCGGAGAGCTGCCAACGTAT GGTTATCGTCGGGTATGGGCGCTGCTTCGCAGACAGGCAGAACTTGATGGTATGCCTGCGATCAATGC CAAACGTGTTTACCGGATCATGCGCCAGAATGCGCTGTTGCTTGAGCGAAAACCTGCTGTACCGCCAT CGAAACGGGCACATACAGGCAGAGTGGCCGTGAAAGAAAGCAATCAGCGATGGTGCTCTGACGGGTTC GAGTTCTGCTGTGATAACGGAGAGAGACTGCGTGTCACGTTCGCGCTGGACTGCTGTGATCGTGAGGC ACTGCACTGGGCGGTCACTACCGGCGGCTTCAACAGTGAAACAGTACAGGACGTCATGCTGGGAGCGG TGGAACGCCGCTTCGGCAACGATCTTCCGTCGTCTCCAGTGGAGTGGCTGACGGATAATGGTTCATGC TACCGGGCTAATGAAACACGCCAGTTCGCCCGGATGTTGGGACTTGAACCGAAGAACACGGCGGTGCG GAGTCCGGAGAGTAACGGAATAGCAGAGAGCTTCGTGAAAACGATAAAGCGTGACTACATCAGTATCA TGCCCAAACCAGACGGGTTAACGGCAGCAAAGAACCTTGCAGAGGCGTTCGAGCATTATAACGAATGG |
| | | CATCCGCATAGTGCGCTGGGTTATCGCTCGCCACGGGAATATCTGCGGCAGCGGGCTTGTAATGGGTT AAGTGATAACAGATGTCTGGAAATATAGGGGCAAATCCA |
| 22 | IS 2 D | TGGATTTGCCCCTATATTTCCAGACATCTGTTATCACTTAACCCATTACAAGCCCGCTGCCGCAGATA TTCCCGTGGCGAGCGATAACCCAGCGCACTATGCGGATGCCATTCGTTATAATGCTCGAACGCCTCTG CAAGGTTCTTTGCTGCCGTTAACCCGTCTGGTTTGGGCATGATACTGATGTAGTCACGCTTTATCGTT TTCACGAAGCTCTCTGCTATTCCGTTACTCTCCGGACTCCGCACCGCCGTGTTCTTCGGTTCAAGTCC CAACATCCGGGCGAACTGGCGTGTTTCATTAGCCCGGTAGCATGAACCATTATCCGTCAGCCACTCCA CTGGAGACGACGGAAGATCGTTGCCGAAGCGGCGTTCCACCGCTCCCAGCATGACGTCCTGTACTGTT TCACTGTTGAAGCCGCCGGTAGTGACCGCCCAGTGCAGTGCCTCACGATCACAGCAGTCCAGCGCGAA CGTGACACGCAGTCTCTCTCCGTTATCACAGCAGAACTCGAACCCGTCAGAGCACCATCGCTGATTGC TTTCTTTCACGGCCACTCTGCCTGTATGTGCCCGTTTCGATGGCGGTACAGCAGGTTTTCGCTCAAGC AACAGCGCATTCTGGCGCATGATCCGGTAAACACGTTTGGCATTGATCGCAGGCATACCATCAAGTTC TGCCTGTCTGCGAAGCAGCGCCCATACCCGACGATAACCATACGTTGGCAGCTCTCCGATAACATGGT GTATACGGAGAAGCACATCCGTATCATCAGTGTGACGACTGCGGCGGCCATCCATCCAGTCATCGGTT CGTCTGAGAATGACGTGCAACTGCGCACGCGACACCCGGAGACAACGGCTGACTAAGCTTACTCCCCA TCCCCGGGCAATAAGGGCGCGTGCGCTATCCACTTTTTTGCCCGTCCATATTCAACGGCTTCTTTGAG GAGTTCATTTTCCATCGTTTTCTTGCCGAGCAGGCGCTGGAGTTCTTTAATCTGCTTCATGGCGGCAG CAAGTTCAGAGGCAGGAACAACCTGTTCTCCGGCGGCGACAGCAGTAAGACTTCCTTCCTGGTATTGC TTACGCCAGAGAAATAACTGGCTGGCTGCTACACCATGTTGCCGGGCAACGAGGGAGACCGTCATCCC CGGTTCAAAGCTCTGCTGAACAATTGCGATCTTTTCCTGTGTGGTACGCCGTCTGCGTTTCTCCGGCC CTAAGACATCAATCATCTGTTCTCCAATGACTAGTCTAAAAACTAGTATTAAGACTATCACTTATTTA AGTGATATTGGTTGTCTGGAGATTCAGGGGGCCAGTCTA |
| 2 3 | IS 2 E | AAGAAAGCAATCAGCGATGGTGCTCTGACGGGTTCGAGTTCTGCTGTGATAACGGAGAGAGACTGCGT GTCACGTTCGCGCTGGACTGCTGTGATCGTGAGGCACTGCACTGGGCGGTCACTACCGGCGGCTTCAA CAGTGAAACAGTACAGGACGTCATGCTGGGAGCGGTGGAACGCCGCTTCGGCAACGATCTTCCGTCGT CTCCAGTGGAGTGGCTGACGGATAATGGTTCATGCTACCGGGCTAATGAAACACGCCAGTTCGCCCGG ATGTTGGGACTTGAACCGAAGAACACGGCGGTGCGGAGTCCGGAGAGTAACGGAATAGCAGAGAGCTT CGTGAAAACGATAAAGCGTGACTACATCAGTATCATGCCCAAACCAGACGGGTTAACGGCAGCAAAGA ACCTTGCAGAGGCGTTCGAGCATTATAACGAATGGCATCCGCATAGTGCGCTGGGTTATCGCTCGCCA CGGGAATATCTGCGGCAGCGGGCTTGTAATGGGTTAA |
| 2 4 | IS 2F | TGGATTTGCCCCTATATTTCCAGACATCTGTTATCACTTAACCCATTACAAGCCCGCTGCCGCAGATA TTCCCGTGGCGAGCGATAACCCAGCGCACTATGCGGATGCCATTCGTTATAATGCTCGAACGCCTCTG CAAGGTTCTTTGCTGCCGTTAACCCGTCTGGTTTGGGCATGATACTGATGTAGTCACGCTTTATCGTT TTCACGAAGCTCTCTGCTATTCCGTTACTCTCCGGACTCCGCACCGCCGTGTTCTTCGGTTCAAGTCC CAACATCCGGGCGAACTGGCGTGTTTCATTAGCCCGGTAGCATGAACCATTATCCGTCAGCCACTCCA CTGGAGACGACGGAAGATCGTTGCCGAAGCGGCGTTCCACCGCTCCCAGCATGACGTCCTGTACTGTT TCACTGTTGAAGCCGCCGGTAGTGACCGCCCAGTGCAGTGCCTCACGATCACAGCAGTCCAGCGCGAA CGTGACACGCAGTCTCTCTCCGTTATCACAGCAGAACTCGAACCCGTCAGAGCACCATCGCTGATTGC TTTCTTTCACGGCCACTCTGCCTGTATGTGCCCGTTTCGATGGCGGTACAGCAGGTTTTCGCTCAAGC |
| | | AACAGCGCATTCTGGCGCATGATCCGGTAAACACGTTTGGCATTGATCGCAGGCATACCATCAAGTTC TGCCTGTCTGCGAAGCAGCGCCCATACCCGACGATAACCATACGTTGGCAGCTCTCCGATAACATGGT GTATACGGAGAAGCACATCCGTATCATCAGTGTGACGACTGCGGCGGCCATCCATCCAGTCATCGGTT CGTCTGAGAATGACGTGCAACTGCGCACGCGACACCCGGAGACAACGGCTGACTAAGCTTACTCCCCA TCCCCGGGCAATAAGGGCGCGTGCGCTATCCACTTTTTTGCCCGTCCATATTCAACGGCTTCTTTGAG GAGTTCATTTTCCATCGTTTTCTTGCCGAGCAGGCGCTGGAGTTCTTTAATCTGCTTCATGGCGGCAG CAAGTTCAGAGGCAGGAACAACCTGTTCTCCGGCGGCGACAGCAGTAAGACTTCCTTCCTGGTATTGC TTACGCCAGAGAAATAACTGGCTGGCTGCTACACCATGTTGCCGGGCAACGAGGGAGACCGTCATCCC CGGTTCAAAGCTCTGCTGAACAATTGCGATCTTTTCCTGTGTGGTACGCCGTCTGCGTTTCTCCGGCC CTAAGACATCAATCATCTGTTCTCCAATGACTAGTCTAAAAACTAGTATTAAGACTATCACTTATTTA AGTGATATTGGTTGTCTGGAGATTCAGGGGGCCAGTCTA |
| 2 5 | IS 2 H | TGGATTTGCCCCTATATTTCCAGACATCTGTTATCACTTAACCCATTACAAGCCCGCTGCCGCAGATA TTCCCGTGGCGAGCGATAACCCAGCGCACTATGCGGATGCCATTCGTTATAATGCTCGAACGCCTCTG CAAGGTTCTTTGCTGCCGTTAACCCGTCTGGTTTGGGCATGATACTGATGTAGTCACGCTTTATCGTT TTCACGAAGCTCTCTGCTATTCCGTTACTCTCCGGACTCCGCACCGCCGTGTTCTTCGGTTCAAGTCC CAACATCCGGGCGAACTGGCGTGTTTCATTAGCCCGGTAGCATGAACCATTATCCGTCAGCCACTCCA CTGGAGACGACGGAAGATCGTTGCCGAAGCGGCGTTCCACCGCTCCCAGCATGACGTCCTGTACTGTT TCACTGTTGAAGCCGCCGGTAGTGACCGCCCAGTGCAGTGCCTCACGATCACAGCAGTCCAGCGCGAA CGTGACACGCAGTCTCTCTCCGTTATCACAGCAGAACTCGAACCCGTCAGAGCACCATCGCTGATTGC TTTCTTTCACGGCCACTCTGCCTGTATGTGCCCGTTTCGATGGCGGTACAGCAGGTTTTCGCTCAAGC AACAGCGCATTCTGGCGCATGATCCGGTAAACACGTTTGGCATTGATCGCAGGCATACCATCAAGTTC TGCCTGTCTGCGAAGCAGCGCCCATACCCGACGATAACCATACGTTGGCAGCTCTCCGATAACATGGT GTATACGGAGAAGCACATCCGTATCATCAGTGTGACGACTGCGGCGGCCATCCATCCAGTCATCGGTT CGTCTGAGAATGACGTGCAACTGCGCACGCGACACCCGGAGACAACGGCTGACTAAGCTTACTCCCCA TCCCCGGGCAATAAGGGCGCGTGCGCTATCCACTTTTTTGCCCGTCCATATTCAACGGCTTCTTTGAG GAGTTCATTTTCCATCGTTTTCTTGCCGAGCAGGCGCTGGAGTTCTTTAATCTGCTTCATGGCGGCAG CAAGTTCAGAGGCAGGAACAACCTGTTCTCCGGCGGCGACAGCAGTAAGACTTCCTTCCTGGTATTGC TTACGCCAGAGAAATAACTGGCTGGCTGCTACACCATGTTGCCGGGCAACGAGGGAGACCGTCATCCC CGGTTCAAAGCTCTGCTGAACAATTGCGATCTTTTCCTGTGTGGTACGCCGTCTGCGTTTCTCCGGCC CTAAGACATCAATCATCTGTTCTCCAATGACTAGTCTAAAAACTAGTATTAAGACTATCACTTATTTA AGTGATATTGGTTGTCTGGAGATTCAGGGGGCCAGT |
| 2 6 | IS 2I | TAGACTGGCCCCCTGAATCTCCAGACAACCAATATCACTTAAATAAGTGATAGTCTTAATACTAGTTT TTAGACTAGTCATTGGAGAACAGATGATTGATGTCTTAGGGCCGGAGAAACGCAGACGGCGTACCACA CAGGAAAAGATCGCAATTGTTCAGCAGAGCTTTGAACCGGGGATGACGGTCTCCCTCGTTGCCCGGCA ACATGGTGTAGCAGCCAGCCAGTTATTTCTCTGGCGTAAGCAATACCAGGAAGGAAGTCTTACTGCTG TCGCCGCCGGAGAACAGGTTGTTCCTGCCTCTGAACTTGCTGCCGCCATGAAGCAGATTAAAGAACTC CAGCGCCTGCTCGGCAAGAAAACGATGGAAAATGAACTCCTCAAAGAAGCCGTTGAATATGGACGGGC AAAAAAGTGGATAGCGCACGCGCCCTTATTGCCCGGGGATGGGGAGTAAGCTTAGTCAGCCGTTGTCT CCGGGTGTCGCGTGCGCAGTTGCACGTCATTCTCAGACGAACCGATGACTGGATGGATGGCCGCCGCA |
| | | GTCGTCACACTGATGATACGGATGTGCTTCTCCGTATACACCATGTTATCGGAGAGCTGCCAACGTAT GGTTATCGTCGGGTATGGCGCTGCTTCGCAGACAGGCAGAACTTGATGGTATGCCTGCGATCAATGC CAAACGTGTTTACCGGATCATGCGCCAGAATGCGCTGTTGCTTGAGCGAAAACCTGCTGTACCGCCAT CGAAACGGGCACATACAGGCAGAGTGGCCGTGAAAGAAAGCAATCAGCGATGGTGCTCTGACGGGTTC GAGTTCTGCTGTGATAACGGAGAGAGACTGCGTGTCACGTTCGCGCTGGACTGCTGTGATCGTGAGGC ACTGCACTGGGCGGTCACTACCGGCGGCTTCAACAGTGAAACAGTACAGGACGTCATGCTGGGAGCGG TGGAACGCCGCTTCGGCAACGATCTTCCGTCGTCTCCAGTGGAGTGGCTGACGGATAATGGTTCATGC TACCGGGCTAATGAAACACGCCAGTTCGCCCGGATGTTGGGACTTGAACCGAAGAACACGGCGGTGCG GAGTCCGGAGAGTAACGGAATAGCAGAGAGCTTCGTGAAAACGATAAAGCGTGACTACATCAGTATCA TGCCCAAACCAGACGGGTTAACGGCAGCAAAGAACCTTGCAGAGGCGTTCGAGCATTATAACGAATGG CATCCGCATAGTGCGCTGGGTTATCGCTCGCCACGGGAATATCTGCGGCAGCGGGCTTGTAATGGGTT AAGTGATAACAGATGTCTGGAAATATAGGGGCAAATCCA |
| 2 7 | IS 2 K | TAGACTGGCCCCCTGAATCTCCAGACAACCAATATCACTTAAATAAGTGATAGTCTTAATACTAGTTT TTAGACTAGTCATTGGAGAACAGATGATTGATGTCTTAGGGCCGGAGAAACGCAGACGGCGTACCACA CAGGAAAAGATCGCAATTGTTCAGCAGAGCTTTGAACCGGGGATGACGGTCTCCCTCGTTGCCCGGCA ACATGGTGTAGCAGCCAGCCAGTTATTTCTCTGGCGTAAGCAATACCAGGAAGGAAGTCTTACTGCTG TCGCCGCCGGAGAACAGGTTGTTCCTGCCTCTGAACTTGCTGCCGCCATGAAGCAGATTAAAGAACTC CAGCGCCTGCTCGGCAAGAAAACGATGGAAAATGAACTCCTCAAAGAAGCCGTTGAATATGGACGGGC AAAAAAGTGGATAGCGCACGCGCCCTTATTGCCCGGGGATGGGGAGTAAGCTTAGTCAGCCGTTGTCT CCGGGTGTCGCGTGCGCAGTTGCACGTCATTCTCAGACGAACCGATGACTGGATGGATGGCCGCCGCA GTCGTCACACTGATGATACGGATGTGCTTCTCCGTATACACCATGTTATCGGAGAGCTGCCAACGTAT GGTTATCGTCGGGTATGGGCGCTGCTTCGCAGACAGGCAGAACTTGATGGTATGCCTGCGATCAATGC CAAACGTGTTTACCGGATCATGCGCCAGAATGCGCTGTTGCTTGAGCGAAAACCTGCTGTACCGCCAT CGAAACGGGCACATACAGGCAGAGTGGCCGTGAAAGAAAGCAATCAGCGATGGTGCTCTGACGGGTTC GAGTTCTGCTGTGATAACGGAGAGAGACTGCGTGTCACGTTCGCGCTGGACTGCTGTGATCGTGAGGC ACTGCACTGGGCGGTCACTACCGGCGGCTTCAACAGTGAAACAGTACAGGACGTCATGCTGGGAGCGG TGGAACGCCGCTTCGGCAACGATCTTCCGTCGTCTCCAGTGGAGTGGCTGACGGATAATGGTTCATGC TACCGGGCTAATGAAACACGCCAGTTCGCCCGGATGTTGGGACTTGAACCGAAGAACACGGCGGTGCG GAGTCCGGAGAGTAACGGAATAGCAGAGAGCTTCGTGAAAACGATAAAGCGTGACTACATCAGTATCA TGCCCAAACCAGACGGGTTAACGGCAGCAAAGAACCTTGCAGAGGCGTTCGAGCATTATAACGAATGG CATCCGCATAGTGCGCTGGGTTATCGCTCGCCACGGGAATATCTGCGGCAGCGGGCTTGTAATGGGTT AAGTGATAACAGATGTCTGGAAATATAGGGGCAAATCCA |
| 2 8 | IS 3 A | TGGACACGCGGCTAAGTGAGTAAACTCTCAGTCAGAGGTGACTCACATGACAAAAACAGTATCAACCA GTAAAAAACCCCGTAAACAGCATTCGCCTGAATTTCGCAGTGAAGCCCTGAAGCTTGCTGAACGCATC GGTGTTACTGCCGCAGCCCGTGAACTCAGCCTGTATGAATCACAACTCTACAACTGGCGCAGTAAACA GCAAAATCAGCAGACGTCTTCTGAACGTGAACTGGAGATGTCTACCGAGATTGCACGTCTCAAACGCC AGCTGGCAGAACGGGATGAAGAGCTGGCTATCCTCCAAAAGGCCGCGACATACTTCGCGAAGCGCCTG AAATGAAGTATGTCTTTATTGAAAAACATCAGGCTGAGTTCAGCATCAAAGCAATGTGCCGCGTGCTC CGGGTGGCCCGCAGCGGCTGGTATACGTGGTGTCAGCGGCGGACAAGGATAAGCACGCGTCAGCAGTT |
| | | CCGCCAACACTGCGACAGCGTTGTCCTCGCGGCTTTTACCCGGTCAAAACAGCGTTACGGTGCCCCAC GCCTGACGGATGAACTGCGTGCTCAGGGTTACCCCTTTAACGTAAAAACCGTGGCGGCAAGCCTGCGC CGTCAGGGACTGAGGGCAAAGGCCTCCCGGAAGTTCAGCCCGGTCAGCTACCGCGCACACGGCCTGCC TGTGTCAGAAAATCTGTTGGAGCAGGATTTTTACGCCAGTGGCCCGAACCAGAAGTGGGCAGGAGACA TCACGTACTTACGTACAGATGAAGGCTGGCTGTATCTGGCAGTGGTCATTGACCTGTGGTCACGTGCC GTTATTGGCTGGTCAATGTCGCCACGCATGACGGCGCAACTGGCCTGCGATGCCCTGCAGATGGCGCT GTGGCGGCGTAAGAGGCCCCGGAACGTTATCGTTCACACGGACCGTGGAGGCCAGTACTGTTCAGCAG ATTATCAGGCGCAACTGAAGCGGCATAATCTGCGTGGAAGTATGAGCGCAAAAGGTTGCTGCTACGAT AATGCCTGCGTGGAAAGCTTCTTTCATTCGCTGAAAGTGGAATGTATCCATGGAGAACACTTTATCAG CCGGGAAATAATGCGGGCAACGGTGTTTAATTATATCGAATGTGATTACAATCGGTGGCGGCGGCACA GTTGGTGTGGCGGCCTCAGTCCGGAACAATTTGAAAACAAGAACCTCGC |
| 2 9 | IS 3 B | TGATCCTACCCACGTAATATGGACACAGGCCTAAGCGAGGTTCTTGTTTTCAAATTGTTCCGGACTGA GGCCGCCACACCAACTGTGCCGCCGCCACCGATTGTAATCACATTCGATATAATTAAACACCGTTGCC CGCATTATTTCCCGGCTGATAAAGTGTTCTCCATGGATACATTCCACTTTCAGCGAATGAAAGAAGCT TTCCACGCAGGCATTATCGTAGCAGCAACCTTTTGCGCTCATACTTCCACGCAGATTATGCCGCTTCA GTTGCGCCTGATAATCTGCTGAACAGTACTGGCCTCCACGGTCCGTGTGAACGATAACGTTCCGGGGC CTCTTACGCCGCCACAGCGCCATCTGCAGGGCATCGCAGGCCAGTTGCGCCGTCATGCGTGGCGACAT TGACCAGCCAATAACGGCACGTGACCACAGGTCAATGACCACTGCCAGATACAGCCAGCCTTCATCTG TACGTAAGTACGTGATGTCTCCTGCCCACTTCTGGTTCGGGCCACTGGCGTAAAAATCCTGCTCCAAC AGATTTTCTGACACAGGCAGGCCGTGTGCGCGGTAGCTGACCGGGCTGAACTTCCGGGAGGCCTTTGC CCTCAGTCCCTGACGGCGCAGGCTTGCCGCCACGGTTTTTACGTTAAAGGGGTAACCCTGAGCACGCA GTTCATCCGTCAGGCGTGGGGCACCGTAACGCTGTTTTGACCGGGTAAAAGCCGCGAGGACAACGCTG TCGCAGTGTTGGCGGAACTGCTGACGCGTGCTTATCCTTGTCCGCCGCTGACACCACGTATACCAGCC GCTGCGGGCCACCCGGAGCACGCGGCACATTGCTTTGATGCTGAACTCAGCCTGATGTTTTTCAATAA AGACATACTTCATTTCAGGCGCTTCGCGAAGTATGTCGCGGCCTTTTGGAGGATAGCCAGCTCTTCAT CCCGTTCTGCCAGCTGGCGTTTGAGACGTGCAATCTCGGTAGACATCTCCAGTTCACGTTCAGAAGAC GTCTGCTGATTTTGCTGTTTACTGCGCCAGTTGTAGAGTTGTGATTCATACAGGCTGAGTTCACGGGC TGCGGCAGTAACACCGATGCGTTCAGCAAGCTTCAGGGCTTCACTGCGAAATTCAGGCGAATGCTGTT TACGGGGTTTTTTACTGGTTGATACTGTTTTTGTCATGTGAGTCACCTCTGACTGAGAGTTTACTCAC TTAGCCGCGTGTCCACTATTGCTGGGTAAGATCA |
| 3 0 | IS 3 C | TGATCTTACCCAGCAATAGTGGACACGCGGCTAAGTGAGTAAACTCTCAGTCAGAGGTGACTCACATG ACAAAAACAGTATCAACCAGTAAAAAACCCCGTAAACAGCATTCGCCTGAATTTCGCAGTGAAGCCCT GAAGCTTGCTGAACGCATCGGTGTTACTGCCGCAGCCCGTGAACTCAGCCTGTATGAATCACAACTCT ACAACTGGCGCAGTAAACAGCAAAATCAGCAGACGTCTTCTGAACGTGAACTGGAGATGTCTACCGAG ATTGCACGTCTCAAACGCCAGCTGGCAGAACGGGATGAAGAGCTGGCTATCCTCCAAAAGGCCGCGAC ATACTTCGCGAAGCGCCTGAAATGAAGTATGTCTTTATTGAAAAACATCAGGCTGAGTTCAGCATCAA AGCAATGTGCCGCGTGCTCCGGGTGGCCCGCAGCGGCTGGTATACGTGGTGTCAGCGGCGGACAAGGA TAAGCACGCGTCAGCAGTTCCGCCAACACTGCGACAGCGTTGTCCTCGCGGCTTTTACCCGGTCAAAA CAGCGTTACGGTGCCCCACGCCTGACGGATGAACTGCGTGCTCAGGGTTACCCCTTTAACGTAAAAAC |
| | | CGTGGCGGCAAGCCTGCGCCGTCAGGGACTGAGGGCAAAGGCCTCCCGGAAGTTCAGCCCGGTCAGCT ACCGCGCACACGGCCTGCCTGTGTCAGAAAATCTGTTGGAGCAGGATTTTTACGCCAGTGGCCCGAAC CAGAAGTGGGCAGGAGACATCACGTACTTACGTACAGATGAAGGCTGGCTGTATCTGGCAGTGGTCAT TGACCTGTGGTCACGTGCCGTTATTGGCTGGTCAATGTCGCCACGCATGACGGCGCAACTGGCCTGCG ATGCCCTGCAGATGGCGCTGTGGCGGCGTAAGAGGCCCCGGAACGTTATCGTTCACACGGACCGTGGA GGCCAGTACTGTTCAGCAGATTATCAGGCGCAACTGAAGCGGCATAATCTGCGTGGAAGTATGAGCGC AAAAGGTTGCTGCTACGATAATGCCTGCGTGGAAAGCTTCTTTCATTCGCTGAAAGTGGAATGTATCC ATGGAGAACACTTTATCAGCCGGGAAATAATGCGGGCAACGGTGTTTAATTATATCGAATGTGATTAC AATCGGTGGCGGCGGCACAGTTGGTGTGGCGGCCTCAGTCCGGAACAATTTGAAAACAAGAACCTCGC TTAGGCCTGTGTCCATATTACGTGGGTAGGATCA |
| 3 1 | IS 3 D | TGATCTTACCCAGCAATAGTGGACACGCGGCTAAGTGAGTAAACTCTCAGTCAGAGGTGACTCACATG ACAAAAACAGTATCAACCAGTAAAAAACCCCGTAAACAGCATTCGCCTGAATTTCGCAGTGAAGCCCT GAAGCTTGCTGAACGCATCGGTGTTACTGCCGCAGCCCGTGAACTCAGCCTGTATGAATCACAACTCT ACAACTGGCGCAGTAAACAGCAAAATCAGCAGACGTCTTCTGAACGTGAACTGGAGATGTCTACCGAG ATTGCACGTCTCAAACGCCAGCTGGCAGAACGGGATGAAGAGCTGGCTATCCTCCAAAAGGCCGCGAC ATACTTCGCGAAGCGCCTGAAATGAAGTATGTCTTTATTGAAAAACATCAGGCTGAGTTCAGCATCAA AGCAATGTGCCGCGTGCTCCGGGTGGCCCGCAGCGGCTGGTATACGTGGTGTCAGCGGCGGACAAGGA TAAGCACGCGTCAGCAGTTCCGCCAACACTGCGACAGCGTTGTCCTCGCGGCTTTTACCCGGTCAAAA CAGCGTTACGGTGCCCCACGCCTGACGGATGAACTGCGTGCTCAGGGTTACCCCTTTAACGTAAAAAC CGTGGCGGCAAGCCTGCGCCGTCAGGGACTGAGGGCAAAGGCCTCCCGGAAGTTCAGCCCGGTCAGCT ACCGCGCACACGGCCTGCCTGTGTCAGAAAATCTGTTGGAGCAGGATTTTTACGCCAGTGGCCCGAAC CAGAAGTGGGCAGGAGACATCACGTACTTACGTACAGATGAAGGCTGGCTGTATCTGGCAGTGGTCAT TGACCTGTGGTCACGTGCCGTTATTGGCTGGTCAATGTCGCCACGCATGACGGCGCAACTGGCCTGCG ATGCCCTGCAGATGGCGCTGTGGCGGCGTAAGAGGCCCCGGAACGTTATCGTTCACACGGACCGTGGA GGCCAGTACTGTTCAGCAGATTATCAGGCGCAACTGAAGCGGCATAATCTGCGTGGAAGTATGAGCGC AAAAGGTTGCTGCTACGATAATGCCTGCGTGGAAAGCTTCTTTCATTCGCTGAAAGTGGAATGTATCC ATGGAGAACACTTTATCAGCCGGGAAATAATGCGGGCAACGGTGTTTAATTATATCGAATGTGATTAC AATCGGTGGCGGCGGCACAGTTGGTGTGGCGGCCTCAGTCCGGAACAATTTGAAAACAAGAACCTCGC TTAGGCCTGTGTCCATATTACGTGGGTAGGATCA |
| 3 2 | IS 3 E | TGATCTTACCCAGCAATAGTGGACACGCGGCTAAGTGAGTAAACTCTCAGTCAGAGGTGACTCACATG ACAAAAACAGTATCAACCAGTAAAAAACCCCGTAAACAGCATTCGCCTGAATTTCGCAGTGAAGCCCT GAAGCTTGCTGAACGCATCGGTGTTACTGCCGCAGCCCGTGAACTCAGCCTGTATGAATCACAACTCT ACAACTGGCGCAGTAAACAGCAAAATCAGCAGACGTCTTCTGAACGTGAACTGGAGATGTCTACCGAG ATTGCACGTCTCAAACGCCAGCTGGCAGAACGGGATGAAGAGCTGGCTATCCTCCAAAAGGCCGCGAC ATACTTCGCGAAGCGCCTGAAATGAAGTATGTCTTTATTGAAAAACATCAGGCTGAGTTCAGCATCAA AGCAATGTGCCGCGTGCTCCGGGTGGCCCGCAGCGGCTGGTATACGTGGTGTCAGCGGCGGACAAGGA TAAGCACGCGTCAGCAGTTCCGCCAACACTGCGACAGCGTTGTCCTCGCGGCTTTTACCCGGTCAAAA CAGCGTTACGGTGCCCCACGCCTGACGGATGAACTGCGTGCTCAGGGTTACCCCTTTAACGTAAAAAC CGTGGCGGCAAGCCTGCGCCGTCAGGGACTGAGGGCAAAGGCCTCCCGGAAGTTCAGCCCGGTCAGCT |
| | | ACCGCGCACACGGCCTGCCTGTGTCAGAAAATCTGTTGGAGCAGGATTTTTACGCCAGTGGCCCGAAC CAGAAGTGGGCAGGAGACATCACGTACTTACGTACAGATGAAGGCTGGCTGTATCTGGCAGTGGTCAT TGACCTGTGGTCACGTGCCGTTATTGGCTGGTCAATGTCGCCACGCATGACGGCGCAACTGGCCTGCG ATGCCCTGCAGATGGCGCTGTGGCGGCGTAAGAGGCCCCGGAACGTTATCGTTCACACGGACCGTGGA GGCCAGTACTGTTCAGCAGATTATCAGGCGCAACTGAAGCGGCATAATCTGCGTGGAAGTATGAGCGC AAAAGGTTGCTGCTACGATAATGCCTGCGTGGAAAGCTTCTTTCATTCGCTGAAAGTGGAATGTATCC ATGGAGAACACTTTATCAGCCGGGAAATAATGCGGGCAACGGTGTTTAATTATATCGAATGTGATTAC AATCGGTGGCGGCGGCACAGTTGGTGTGGCGGCCTCAGTCCGGAACAATTTGAAAACAAGAACCTCGC TTAGGCCTGTGTCCATATTACGTGGGTAGGATCA |
| 33 | IS 30 A | TGTAGATTCAATTGGTCAACGCAACAGTTATGTGAAAACATGGGGTTGCGGAGGTTTTTGAATGAGA CGAACATTTACAGCAGAGGAAAAAGCCTCTGTTTTTGAACTATGGAAGAACGGAACAGGCTTCAGTGA AATAGCGAATATCCTGGGTTCAAAACCCGGAACGATCTTCACTATGTTAAGGATACTGGCGGCATAA AACCCCATGAGCGTAAGCGGGCTGTAGCTCACCTGACACTGTCTGAGCGCGAGGAGATACGAGCTGGT TTGTCAGCCAAAATGAGCATTCGTGCGATAGCTACTGCGCTGAATCGCAGTCCTTCGACGATCTCACG TGAAGTTCAGCGTAATCGGGGCAGACGCTATTACAAAGCTGTTGATGCTAATAACCGAGCCAACAGAA TGGCGAAAAGGCCAAAACCGTGCTTACTGGATCAAAATTTACCATTGCGAAAGCTTGTTCTGGAAAAG CTGGAGATGAAATGGTCTCCAGAGCAAATATCAGGATGGTTAAGGCGAACAAAACCACGTCAAAAAAC GCTGCGAATATCACCTGAGACAATTTATAAAACGCTGTACTTTCGTAGCCGTGAAGCGCTACACCACC TGAATATACAGCATCTGCGACGGTCGCATAGCCTTCGCCATGGCAGGCGTCATACCCGCAAAGGCGAA AGAGGTACGATTAACATAGTGAACGGAACACCAATTCACGAACGTTCCCGAAATATCGATAACAGACG CTCTCTGGGGCATTGGGAGGGCGATTTAGTCTCAGGTACAAAAAACTCTCATATAGCCACACTTGTAG ACCGAAAATCACGTTATACGATCATCGTTAGACTCAGGGGCAAAGATTCTGTCTCAGTAAATCAGGCT CTTACCGACAAATTCCTGAGTTTACCGTCAGAACTCAGAAAATCACTGACATGGACAGAGGAATGGA ACTGGCCAGACATCTAGAATTTACTGTCAGCACCGGCGTTAAAGTTTACTTCTGCGATCCTCAGAGTC CTTGGCAGCGGGGAACAAATGAGAATACAAATGGGCTAATTCGGCAGTACTTTCCTAAAAAGACATGT CTTGCCCAATATACTCAACATGAACTAGATCTGGTTGCTGCTCAGCTAAACAACAGACCGAGAAAGAC ACTGAAGTTCAAAACACCGAAAGAGATAATTGAAAGGGGTGTTGCATTGACAGATTGAATCTACA |
| 3 4 | IS 30 B | GAGAACACAAATGGGCTAATTCGGCAGTACTTTCCTAAAAAGACATGTCTTGCCCAATATACTCAACA TGAACTAGATCTGGTTGCTGCTCAGCTAAACAACAGACCGAGAAAGACACTGAAGTTCAAAACACCGA AAGAGATAATTGAAAGGGGTGTTGCATTGACAGATTGAATCTACAG |
| 3 5 | IS 30 C | TGTAGATTCAATTGGTCAACGCAACAGTTATGTGAAAACATGGGGTTGCGGAGGTTTTTTGAATGAGA CGAACATTTACAGCAGAGGAAAAAGCCTCTGTTTTTGAACTATGGAAGAACGGAACAGGCTTCAGTGA AATAGCGAATATCCTGGGTTCAAAACCCGGAACGATCTTCACTATGTTAAGGATACTGGCGGCATAA AACCCCATGAGCGTAAGCGGGCTGTAGCTCACCTGACACTGTCTGAGCGCGAGGAGATACGAGCTGGT TTGTCAGCCAAAATGAGCATTCGTGCGATAGCTACTGCGCTGAATCGCAGTCCTTCGACGATCTCACG TGAAGTTCAGCGTAATCGGGGCAGACGCTATTACAAAGCTGTTGATGCTAATAACCGAGCCAACAGAA TGGCGAAAAGGCCAAAACCGTGCTTACTGGATCAAAATTTACCATTGCGAAAGCTTGTTCTGGAAAAG CTGGAGATGAAATGGTCTCCAGAGCAAATATCAGGATGGTTAAGGCGAACAAAACCACGTCAAAAAAC GCTGCGAATATCACCTGAGACAATTTATAAAACGCTGTACTTTCGTAGCCGTGAAGCGCTACACCACC |
| | | TGAATATACAGCATCTGCGACGGTCGCATAGCCTTCGCCATGGCAGGCGTCATACCCGCAAAGGCGAA AGAGGTACGATTAACATAGTGAACGGAACACCAATTCACGAACGTTCCCGAAATATCGATAACAGACG CTCTCTAGGGCATTGGGAGGGCGATTTAGTCTCAGGTACAAAAAACTCTCATATAGCCACACTTGTAG ACCGAAAATCACGTTATACGATCATCCTTAGACTCAGGGGCAAAGATTCTGTCTCAGTAAATCAGGCT CTTACCGACAAATTCCTGAGTTTACCGTCAGAACTCAGAAAATCACTGACATGGGACAGAGGAATGGA ACTGGCCAGACATCTAGAATTTACTGTCAGCACCGGCGTTAAAGTTTACTTCTGCGATCCTCAGAGTC CTTGGCAGCGGGGAACAAATGAGAACACAAATGGGCTAATTCGGCAGTACTTTCCTAAAAAGACATGT CTTGCCCAATATACTCAACATGAACTAGATCTGGTTGCTGCTCAGCTAAACAACAGACCGAGAAAGAC ACTGAAGTTCAAAACACCGAAAGAGATAATTGAAAGGGGTGTTGCATTGACAGATTGAATCTACA |
| 3 6 | IS 30 D | TGTAGATTCAATTGGTCAACGCAACAGTTATGTGAAAACATGGGGTTGCGGAGGTTTTTTGAATGAGA CGAACATTTACAGCAGAGGAAAAAGCCTCTGTTTTTGAACTATGGAAGAACGGAACAGGCTTCAGTGA AATAGCGAATATCCTGGGTTCAAAACCCGGAACGATCTTCACTATGTTAAGGATACTGGCGGCATAA AACCCCATGAGCGTAAGCGGGCTGTAGCTCACCTGACACTGTCTGAGCGCGAGGAGATACGAGCTGGT TTGTCAGCCAAAATGAGCATTCGTGCGATAGCTACTGCGCTGAATCGCAGTCCTTCGACGATCTCACG TGAAGTTCAGCGTAATCGGGGCAGACGCTATTACAAAGCTGTTGATGCTAATAACCGAGCCAACAGAA TGGCGAAAAGGCCAAAACCGTGCTTACTGGATCAAAATTTACCATTGCGAAAGCTTGTTCTGGAAAAG CTGGAGATGAAATGGTCTCCAGAGCAAATATCAGGATGGTTAAGGCGAACAAAACCACGTCAAAAAAC GCTGCGAATATCACCTGAGACAATTTATAAAACGCTGTACTTTCGTAGCCGTGAAGCGCTACACCACC TGAATATACAGCATCTGCGACGGTCGCATAGCCTTCGCCATGGCAGGCGTCATACCCGCAAAGGCGAA AGAGGTACGATTAACATAGTGAACGGAACACCAATTCACGAACGTTCCCGAAATATCGATAACAGACG CTCTCTAGGGCATTGGGAGGGCGATTTAGTCTCAGGTACAAAAAACTCTCATATAGCCACACTTGTAG ACCGAAAATCACGTTATACGATCATCCTTAGACTCAGGGGCAAAGATTCTGTCTCAGTAAATCAGGCT CTTACCGACAAATTCCTGAGTTTACCGTCAGAACTCAGAAAATCACTGACATGGACAGAGGAATGGA ACTGGCCAGACATCTAGAATTTACTGTCAGCACCGGCGTTAAAGTTTACTTCTGCGATCCTCAGAGTC CTTGGCAGCGGGGAACAAATGAGAACACAAATGGGCTAATTCGGCAGTACTTTCCTAAAAAGACATGT CTTGCCCAATATACTCAACATGAACTAGATCTGGTTGCTGCTCAGCTAAACAACAGACCGAGAAAGAC ACTGAAGTTCAAAACACCGAAAGAGATAATTGAAAGGGGTGTTGCATTGACAGATTGAATCTACA |
| 3 7 | IS 15 0 | TGTACTGCACCCATTTTGTTGGACGATGAAATGGAATAGCCCCTAATATGTCAAAGCCAAAATACCCT TTTGAAAAGCGCCTTGAAGTCGTGAATCACTACTTCACAACTGATGATGGTTACAGGATCATCTCGGC ACGTTTTGGTGTCCCCCGAACCCAGGTCAGGACATGGGTTGCCCTCTATGAAAAACATGGAGAAAAAG GTTTAATTCCCAAACCTAAAGGCGTTAGTGCTGATCCAGAGTTGCGTATTAAGGTCGTGAAAGCTGTG ATCGAGCAGCACATGTCCCTTAATCAGGCTGCTGCTCACTTTATGCTTGCTGGTAGTGGTTCTGTAGC CAGGTGGCTGAAGGTCTATGAAGAGCGCGGAGAAGCTGGTTTACGCGCGCTCAAGATTGGCACCAAAA GAAACATTGCAATATCAGTTGATCCAGAAAAAGCGGCATCAGCATTGGAGCTGTCAAAAGACCGACGC ATTGAGGATCTTGAAAGGCAAGTTCGATTTCTTGAAACGCGGCTTATGTATCTAAAAAAGCTGAAAGC CTTAGCTCATCCCACGAAAAAGTGAAAGTACTCAACGAGCTAAGGCAGTTTTATCCTCTTGATGAGCT TCTCAGGGCTGCGGAGATACCGCGCAGTACGTTTTATTATCATCTAAAGGCTCTCAGCAAGCCTGACA AGTATGCGGACGTTAAAAAGCGTATTAGTGAGATTTATCACGAGAATAGAGGCCGATACGGATACCGT AGGGTAACGCTGTCTCTTCATCGAGAAGGAAACAGATTAACCATAAAGCTGTTCAGCGCCTGATGGG |
| | | AACCCTCTCACTTAAAGCAGCGATTAAGGTCAAGCGATACCGCTCTTACAGAGGAGAGGTAGGGCAAA CCGCCCCTAATGTTCTCCAAAGAGATTTCAAGGCTACGCGGCCAAACGAGAAGTGGGTTACCGATGTT ACTGAATTTGCAGTCAATGGGCGCAAGCTGTATTTGTCTCCAGTAATAGATCTCTTCAACAACGAAGT TATTTCTTACAGCCTTTCGGAAAGACCAGTGATGAACATGGTTGAGAATATGCTCGATCAGGCATTCA AAAAGCTTAATCCTCACGAGCATCCTGTTCTGCACTCTGACCAGGGATGGCAGTATCGTATGAGAAGA TATCAAAATATCCTTAAAGAACATGGTATTAAACAAAGCATGTCCAGAAAAGGCAATTGTCTGGATAA TGCTGTGGTGGAGTGTTTCTTTGGAACCTTAAAGTCGGAGTGTTTTATCTTGATGAGTTCAGTAATA TAAGCGAACTGAAGGATGCTGTTACGGAATATATTGAATACTACAACAGCAGAAGAATTAGCCTGAAA TTAAAAGGTCTGACTCCAATTGAATATCGGAATCAGACCTATATGCCTCGTGTTTAACTGTCCAACTT TTTGGGGTCAGTACA |
| 3 8 | IS 18 6 A | CCCATAAGCGCTAACTTAAGGGTTGTGGTATTACGCCTGATATGATTTAACGTGCCGATGAATTACTC TCACGATAACTGGTCAGCAATTCTGGCCCATATTGGTAAGCCCGAAGAACTGGATACTTCGGCACGTA ATGCCGGGGCTCTAACCCGCCGCCGCGAAATTCGTGATGCTGCAACTCTGCTACGTCTGGGGCTGGCT TACGGCCCCGGGGGGATGTCATTACGTGAAGTCACTGCATGGGCTCAGCTCCATGACGTTGCAACATT ATCTGACGTGGCTCTCCTGAAGCGGCTGCGGAATGCCGCCGACTGGTTTGGCATACTTGCCGCACAAA CACTTGCTGTACGCGCCGCAGTTACGGGTTGTACAAGCGGAAAGAGATTGCGTCTTGTCGATGGAACA GCAATCAGTGCGCCCGGGGGCGGCAGCGCTGAATGGCGACTACATATGGGATATGATCCTCATACCTG TCAGTTCACTGATTTTGAGCTAACCGACAGCAGAGACGCTGAACGGCTGGACCGATTTGCGCAAACGG CAGACGAGATACGCATTGCTGACCGGGGATTCGGTTCGCGTCCCGAATGTATCCGCTCACTTGCTTTT GGAGAAGCTGATTATATCGTCCGGGTTCACTGGCGAGGATTGCGCTGGTTAACTGCAGAAGGAATGCG CTTTGACATGATGGGTTTTCTGCGCGGGCTGGATTGCGGTAAGAACGGTGAAACCACTGTAATGATAG GCAATTCAGGTAATAAAAAAGCCGGAGCTCCCTTTCCGGCACGTCTCATTGCCGTATCACTTCCTCCC GAAAAAGCATTAATCAGTAAAACCCGACTGCTCAGCGAGAATCGTCGAAAAGGACGAGTAGTTCAGGC GGAAACGCTGGAAGCAGCGGGCCATGTGCTATTGCTAACATCATTACCGGAAGATGAATATTCAGCAG AGCAAGTGGCTGATTGTTACCGTCTGCGATGGCAAATTGAACTGGCTTTTAAGCGGCTCAAAAGTTTG CTGCACCTGGATGCTTTGCGTGCAAAGGAACCTGAACTCGCGAAAGCGTGGATATTTGCTAATCTACT CGCCGCATTTTTAATTGACGACATAATCCAGCCATCGCTGGATTTCCCCCCCAGAAGTGCCGGATCCG AAAAGAAGAACTAACTCGTTGTGGAGAATAACAAAAATGGTCATCTGGAGCTTACAGGTGGCCATTCG TGGGACAGTATCCCTGACAGCCTACAAAACGCAATTGAAGAACGCGAGGCATCGTCTTAACGAGGCAC CGAGGCGTCGCATTCTTCAGATGGTTCAACCCTTAAGTTAGCGCTTATGGG |
| 3 9 | IS 18 6 B | CCCATAAGCGCTAACTTAAGGGTTGTGGTATTACGCCTGATATGATTTAACGTGCCGATGAATTACTC TCACGATAACTGGTCAGCAATTCTGGCCCATATTGGTAAGCCCGAAGAACTGGATACTTCGGCACGTA ATGCCGGGGCTCTAACCCGCCGCCGCGAAATTCGTGATGCTGCAACTCTGCTACGTCTGGGGCTGGCT TACGGCCCCGGGGGATGTCATTACGTGAAGTCACTGCATGGGCTCAGCTCCATGACGTTGCAACATT ATCTGACGTGGCTCTCCTGAAGCGGCTGCGGAATGCCGCCGACTGGTTTGGCATACTTGCCGCACAAA CACTTGCTGTACGCGCCGCAGTTACGGGTTGTACAAGCGGAAAGAGATTGCGTCTTGTCGATGGAACA GCAATCAGTGCGCCCGGGGGCGGCAGCGCTGAATGGCGACTACATATGGGATATGATCCTCATACCTG TCAGTTCACTGATTTTGAGCTAACCGACAGCAGAGACGCTGAACGGCTGGACCGATTTGCGCAAACGG CAGACGAGATACGCATTGCTGACCGGGGATTCGGTTCGCGTCCCGAATGTATCCGCTCACTTGCTTTT |
| | | GGAGAAGCTGATTATATCGTCCGGGTTCACTGGCGAGGATTGCGCTGGTTAACTGCAGAAGGAATGCG CTTTGACATGATGGGTTTTCTGCGCGGCTGGATTGCGGTAAGAACGGTGAAACCACTGTAATGATAG GCAATTCAGGTAATAAAAAAGCCGGAGCTCCCTTTCCGGCACGTCTCATTGCCGTATCACTTCCTCCC GAAAAAGCATTAATCAGTAAAACCCGACTGCTCAGCGAGAATCGTCGAAAAGGACGAGTAGTTCAGGC GGAAACGCTGGAAGCAGCGGGCCATGTGCTATTGCTAACATCATTACCGGAAGATGAATATTCAGCAG AGCAAGTGGCTGATTGTTACCGTCTGCGATGGCAAATTGAACTGGCTTTTAAGCGGCTCAAAAGTTTG CTGCACCTGGATGCTTTGCGTGCAAAGGAACCTGAACTCGCGAAAGCGTGGATATTTGCTAATCTACT CGCCGCATTTTTAATTGACGACATAATCCAGCCATCGCTGGATTTCCCCCCCAGAAGTGCCGGATCCG AAAAGAAGAACTAACTCGTTGTGGAGAATAACAAAAATGGTCATCTGGAGCTTACAGGTGGCCATTCG TGGGACAGTATCCCTGACAGCCTACAAAACGCAATTGAAGAACGCGAGGCATCGTCTTAACGAGGCAC CGAGGCGTCGCATTCTTCAGATGGTTCAACCCTTAAGTTAGCGCTTATGGG |
| 4 0 | IS 18 6 C | CCCATAAGCGCTAACTTAAGGGTTGTGGTATTACGCCTGATATGATTTAACGTGCCGATGAATTACTC TCACGATAACTGGTCAGCAATTCTGGCCCATATTGGTAAGCCCGAAGAACTGGATACTTCGGCACGTA ATGCCGGGGCTCTAACCCGCCGCCGCGAAATTCGTGATGCTGCAACTCTGCTACGTCTGGGGCTGGCT TACGGCCCCGGGGGATGTCATTACGTGAAGTCACTGCATGGGCTCAGCTCCATGACGTTGCAACATT ATCTGACGTGGCTCTCCTGAAGCGGCTGCGGAATGCCGCCGACTGGTTTGGCATACTTGCCGCACAAA CACTTGCTGTACGCGCCGCAGTTACGGGTTGTACAAGCGGAAAGAGATTGCGTCTTGTCGATGGAACA GCAATCAGTGCGCCCGGGGGCGGCAGCGCTGAATGGCGACTACATATGGGATATGATCCTCATACCTG TCAGTTCACTGATTTTGAGCTAACCGACAGCAGAGACGCTGAACGGCTGGACCGATTTGCGCAAACGG CAGACGAGATACGCATTGCTGACCGGGGATTCGGTTCGCGTCCCGAATGTATCCGCTCACTTGCTTTT GGAGAAGCTGATTATATCGTCCGGGTTCACTGGCGAGGATTGCGCTGGTTAACTGCAGAAGGAATGCG CTTTGACATGATGGGTTTTCTGCGCGGGCTGGATTGCGGTAAGAACGGTGAAACCACTGTAATGATAG GCAATTCAGGTAATAAAAAAGCCGGAGCTCCCTTTCCGGCACGTCTCATTGCCGTATCACTTCCTCCC GAAAAAGCATTAATCAGTAAAACCCGACTGCTCAGCGAGAATCGTCGAAAAGGACGAGTAGTTCAGGC GGAAACGCTGGAAGCAGCGGGCCATGTGCTATTGCTAACATCATTACCGGAAGATGAATATTCAGCAG AGCAAGTGGCTGATTGTTACCGTCTGCGATGGCAAATTGAACTGGCTTTTAAGCGGCTCAAAAGTTTG CTGCACCTGGATGCTTTGCGTGCAAAGGAACCTGAACTCGCGAAAGCGTGGATATTTGCTAATCTACT CGCCGCATTTTTAATTGACGACATAATCCAGCCATCGCTGGATTTCCCCCCCAGAAGTGCCGGATCCG AAAAGAAGAACTAACTCGTTGTGGAGAATAACAAAAATGGTCATCTGGAGCTTACAGGTGGCCATTCG TGGGACAGTATCCCTGACAGCCTACAAAACGCAATTGAAGAACGCGAGGCATCGTCTTAACGAGGCAC CGAGGCGTCGCATTCTTCAGATGGTTCAACCCTTAAGTTAGCGCTTATGGG |
| 4 1 | IS 60 0 | TGAGGTAGCCTGAGTTTAACGGACACTCCTTCCTGAAATAGAATGGCATCAGAAGGAGCTAATAATGA GCAGAAAAACCCAACGTTACTCTAAAAAGTTCAAAGCCGAAGCTGTCAGAACGGTTCTTGAAAATCAA CTTTCGATCAGTGAAGGCGCTTCCCGATTATCTCTTCCTGAAGGCACTTTAGGACAATGGGTTACCGC CGCCAGAAAAGGGCTCGGTACTCCTGGTTCCCGCACGGTGGCTGAACTGGAATCTGAAATTCTGCAAC TGCGTAAGGCGTTAAATGAAGCTCGCCTTGAGCGAGATATATTAAAAAAAGCAAC |
| 4 2 | IS 60 9 | CCGGTGCGCCGTAAAACCCCATCCTTCAGGGCGGGGATATAAGGCGCGGTTTTCCACCTGACCAGGTG TTTGCTGTTGTTCAATGTATTGGCGGATGACGGATATTGGCGCACCTCCGCAGCTACTGGCAAAATAG CCAGGACTCCACAAAACGCCTTTGTAGTAATACCTGAATGCAATATCTGGTCGATCTCGTCGCAGTCA |
| | | CCTACCCGATACGCCTTTGAGGCTGTTTACCAGACTGGATATCGCCAGTTTGGGAGGATAGTTAATTA ACAAATGGACGTGATCTGGTTCGCCATCCATTTCAACCAGTTCAGCTTCAAAATCAGCACATACATTT GAAAAGTAAGTGCGTAGTTTTTCTGTCGCGTCATGGTCAAAAATCTGGCGTCGGTATCTGGTGACAAA GAGCAGGTGAACATGCATCAGGAAAACACAATGCCTTCCACGCCGGATATCGGTTTCTTTTTTCACAG ACCAAAGTATCATTTGACCTGTGAAACGATTACAGGCATTTAAATTCCAGTTAAGACCCGGTGGTCAA CAGGAGTGTGAAATGAGGCGCTTCGCAGGCGCATGTCGTTTCGTTTTCAATCGTGCTCTGGCACGTCA GAATGAGAATCATGAGGCCGGTAATAAATACATCCCTTACGGAAAATGGCTTCCTGGCTGGTTGAGT GGAAAAATGCCACTGAAACGCAATGGCTTAAAGATTCTCCCTCACAGCCATTGCAACAGTCACTGAAA GACCTTGAGCGGGCTTACAAAAACTTCTTCCGGAAGCGGGCTGCTTTTCCCCGATTCAAAAAGCGGGG ACAGAATGATGCATTCCGCTACCCGCAGGGCGTTAAGCTCGATCAGGAAAACAGCCGTATTTTTCTGC CGAAACTGGGCTGGATGCGCTACCGGAACAGCCGTCAGGTCACGGGTGTTGTGAAAAATGTCACTGTC AGCCAGTCCTGCGGTAAGTGGTACATCAGTATTCAGACAGAAAGTGAAGTATCAACTCCGGTTCACCC TTCAGCATCAATGGTCGGGCTGGATGCTGGCGTGGCTAAACTCGCCACGCTGTCAGATGGCACAGTCT TTGAGCCTGTAAACAGTTTTCAGAAAAACCAGAAGAAGCTGGCGAGACTTCAGCGACAGTTAAGCCGC AAGGTCAAATTCAGCAACAACTGGCAAAAGCAGAAACGCAAAATACAGCGACTGCATTCCTGTATCGC AAATATCCGCAGGGACTACCTTCACAAAGTCACAACGGCCGTCAGCAAAAACCACGCAATGATAGTCA TTGAGGATTTGAAGGTCAGCAACATGTCAAAGTCAGCAGCGGGTACGGTCAGCCAGCCGGGGCGCAAT GTCCGGGCAAAATCAGGTTTAAACCGTTCGATACTGGATCAGGGCTGGTATGAAATGCGCCGCCAGCT TGCGTATAAGCAGCTCTGGCGTGGCGGTCAGGTGCTTGCTGTTCCGCCAGCGTATACAAGCCAGCGTT GCGCGTACTGTGGTCATACAGCGAAAGAGAACCGCCTGTCACAAAGTAAATTCAGATGCCAGGTATGT GGATATACAGCGAACGCCGATGTAAATGGCGCTCGCAACATTTTAGCGGCGGGCACGCCGTTCTTGC CTGTGGAGAGATGGTGCAGTCAGGCCGCCCGTTGAAGCAGGAACCCACCGAAATGATTCAGGCGACAG CCTGAACGTAGCAGGGATCCACGTCCTTCAGGGCGTGGAGGATG |
| 4 3 | IS 91 1 A | Bold = IS911-A site 1, underlined = IS911A-site 2 |
| | | TGAAGTGGTTTACTGAATTTGGCCACCTGAACAGAGGTGATATGCTCACCTCAGAACAACACAGGTGT CATAATGAAAAAAAGAAATTTCAGCGCAGAGTTTAAACGCGAATCCGCTCAACTGGTCGTTGACCAGA ACTACACCGTGGCAGATGCAGCCAGCGCTATGGATGTCGGCCTTTCCACAATGACGCGATGGGTGAAA CAATTACGTGATGAACGGCAGGGCAAAACACCAAAAGCCTCCCCCATTACCCCGGAACAAATTGAAAT CCGTGAGCTCAGGAAAAAGCTACAACGTATTGAAATGGAAAATGAAATATTAAAAAGGCTACACCGC GCTCTTGATGTCAGACTCCCTGAACAGTTCTCGATAATCGGGAAACTCAGGGCGCGTTATCCTGTGGC CACTCTCTGCCATGTGTTCGGGGTTCATCGCAGCAGCTACAAATACTGGAAAAACCGTCCTGAAAAGC CAGACGGCAGACGGGCTGTATTACGCAGTCAGGTACTTGAACTGCATGGCATCAGCCACGGCTCTGCC GGAGCAAGAAGCATCGCCACAATGGCAACCCAGAGAGGATACCAGATGGGGCGCTGGCTTGCTGGCAG ACTCATGAAAGAGCTGGGGCTGGTCAGTTGCCAGCAGCCGACTCACCGGTATAAGCGTGGCGGTCATG AGCACGTTGCTATCCCGAATCATCTTGAGCGACAGTTCGCCGTAACGGAACCAAATCAGGTGTGGTGC GGTGATGTGACCTATA |
| 44 | IS 91 | Bold = IS911-B site 1, underlined = IS911B-site 2 |
| | 1 B | TGAAGTGGCACACTGAATTTGGCCACCTGAACAGAGGTGATATGCTCACCTCAGAACAACACAGGTGC TCCAATGAAAAAAAGAAATTTCAGCGCAGAGTTTAAACGCGAATCCGCTCAACTGGTTGTTGACCAGA AATACACGGTGGCAGATGCCGCGAAAGCTATGGATGTTGGCCTTTCCACAATGACAAGATGGGTCAAA CAACTGCGTGATGAGCGTCAGGGCAAAACACCAAAAGCCTCTCCGATAACACCAGAACAAATCGAAAT ACGTAAGCTGAGGAAAAAGCTACAACGGATTGAAATGGAGAATGAAATATTAAAAAGGCTACGGAAAA ACCGTCCTGAAAAACCAGACGGCAGACGGGCTGTATTACGCAGTCAGGTACTTGAGCTACATGGCATC AGCCACGGTTCGGCCGGAGCAAGAAGCATCGCCACAATGGCAACCCGGAGAGGCTACCAGATGGGACG CTGGCTTGCTGGCAGGCTCATGAAAGAGCTGGGGCTGGTCAGCTGTCAGCAGCCGACTCACCGGTATA AACGTGGTGGTCATGAACATGTTGCTATCCCTAACTACCTTGAAAGGCAGTTCGCCGTGACCGAGCCA AATCAGGTGTGGTGCGGTGATGTGACCTATATCTGGACGGGTAAGCGCTGGGCGTACCTCGCCGTTGT TCTCGACCTGTTCGCAAGAAAACCAGTGGGCTGGGCCATGTCGTTCTCGCCGGACAGCAGGCTTACCA TGAAAGCACTGGAAATGGCATGGGAAACCCGTGGTAAGCCCGTCGGGGTGATGTTCCAAGCGATCAAG GCAGTCATTATACGAGCAGGCAGTTCCGGCAGTTACTGTGGCGATACCGGATCAGGCAGAGTATGAGT CGGCGTGGAAACTGCTGGGATAACAGCCCAATGGAGCGCTTCTTCAGGAGTCTGAAGAACGAATGGGT GCCAGCGACGGGCTATGTAAGCTTCAGCGATGCAGCTCACGCAATAACGGACTATATCGTTGGATATT ACAGCGCACTAAGACCGCACGAATATAATGGTGGGTTACCACCAAACGAATCAGAAAACCGATACTGG AAAAACTCTAACGCGGAGGCCAGTTTTAGTTGACCACAACA |
| 4 5 | IS X | CAGCTTTTTTTAATATGTCCCGTTCGTCGGTAACCCGTTTCAGCTCTTTCTGGAGACGGCGGATCTCG GCCTGAGCATCTGACTGTTCTTTATTAGTGGAAGAATCCGGACCGTACTTCTTTATCCAGGCATAAAG GCTGTGGGTGGTGATATCGAGACGTGTTGCAACGCTGGCAACAGAATAACCGAGATCAACAACCTGTT TGACTGCTTCAGTTTTAAACTCTTCGGGATAACGCTTACCGCTCATGGGCACCTCTCTTTAAGCCATT |
| 4 6 | IS Z | CAATGCCGTTCACTTAAGAGGAGCGGCACTATGTTTCACAGGATAACGGGTTTTTGATATCTTAACCG ACCTCGGCCTTGATGGTCGGGGGCGTTTTGTTATGAACACCACTTCCAGAGCACCCCGAAGATGCTCC AGTCGTTTCGGGATGGTCCCTGGTGACGCCGTGTTTCCCAGCTCTATCATTTCTGATGCGATATTCTT CCACGCAGGCAGTAGCCAGTGGCGGTTACAACCCTTCTGGTTCAGCGTCAGCAGCAGGTCTTCGCTGT AAAAAAGTTTATCAAACAACGTAATAGAGTTATCCGGGATGGTGGCGAGCATGGAGTGGGCCAGCACA GTTTCGCTCTGCCGGTAAGGTGCGGTCACGGCATTCAGCAGAATGTGACTTCCCAGGTTCATTAAGGC CACCAGACGCATAACCGGGTAGGCGTTCTGCCGCTTAGTGGATGTGTTGGCAGACCCATAATATTCAC GCAGCTCGGGTTTATCAGGTGTCCTGAACTGTGCGCCATCAATGGCAAAAAGTTGCAGGCCGTGCCAG TCATCCTTCAGGTAACGTTCCGCGCCCCTGTCTGTGCGGTCTGGCGGAAGAGCCATTCCACTGGGGCG GCCCCCACGCGCTGACGCGCCTGGGTGACAGCGCTGCGGGCCAGCAGGTTCATCCCCGCTTCGCCATC CGCGCTCAGGTTCAGACGGCGAACAACATCGGTAATTGGCTCATTGCACCACCATCCAGATAACCATG TCCCCCGGTAAACGACGGCGGCGAACGGTCGCATGAGCAGAAAGCGTCAGGCAGTGTTGTATCCACTC GGTGGGAAGGTGTTCTGCAAATAGTTGTGCAGAGGGCGGAGGCATAAGCGGATGGTCACTGAAATCGA GCAGATCATTGAGAAGTGGCATAAGAAAACGGCTCCCTGTTGTGGAAGCCGTTATAGTGCCTCAGTTT AAGGATCGGTCAACTAATCCTTAACTGATCGGCATTG |
| 1 6 1 | IS 62 9 | TGAACCGCCCCGGGAATCCTGGAGACTAAACTTCCTGAGAAAGAGGTAAACAGGATGACTAAAAATAC TCGTTTTTCCCCCGAAGTCCGTCAACGGGCAGTCCGTATGGTTCTGGAAAGTCAGGGCGAATATGACT CACAATGGGCGACAATTTGTTCCATTGCTCCAAAGATTGGCTGTACGCCGGAGACTCTGCGTGTCCGG |
| | | GTTCGCCAGCATGAGCGGATACCGGGGGCGGTGATGGAGGGCTCACCACCGCTGAACGTCAGCGTCT GAAAGAGCTGGAGCGTGAAAATCGTGAACTGCGCCGCAGTAACGATATCCTTCGCCAGGCTTCCGCTT ATTTTGCGAAGGCGGAGTTTGACCGCCTCTGGAAAAAATGATGCCACTGCTGGATAAGCTGCGTGAGC AGTACGGGGTCGGACCGCTATGCAGCGAACTGCATATTGCCCCGTCAACGTATTACCACTGTCAGCAA CAGCGACATCATCCGGATAAACGCAGTGCCCGTGCGCAGCGCGATGACTGGCTGAAGAAAGAGATACA GCGCGTATACGATGAAAATCACAAGGTATACGGTGTGCGTAAAGTCTGGCGTCAGTTGTTACGGGAAG GTATCAGAGTGGCCAGATGCACTGTGGCACGTCTCATGGCGGTTATGGGACTTGCCGGTGTTCTCCGG GGTAAAAAGGTCCGTACGACCATCAGCCGGAAAGCCGTTGCCGCAGGCGACCGCGTAAACCGTCAGTT CGTGGCAGAACGACCTGACCAGCTGTGGGTGGCTGATTTTACTTACGTCAGCACATGGCAGGGCTTCG TCTATGTGGCGTTCATCATTGATGTGTTTGCCGGATACATCGTGGGGTGGCGGGTCTCATCGTCCATG GAAACGACATTCGTGCTGGATGCACTGGAGCAGGCGTTATGGGCCCGTCGACCGTCCGGCACGGTCCA TCACAGTGATAAAGGTTCTCAGTATGTATCGCTGGCCTACACACAGCGGCTTAAGGAAGCCGGATTAC TGGCATCAACAGGAAGTACAGGCGACTCGTATGACAACGCGATGGCGGAGAGCATCAATGGTCTTTAC AAAGCGAAGGTAATACACCGTAAGAGCTGGAAAAACCGTGCAGAAGTGGAACTGGCCACACTCACGTG GGTGGACTGGTATAACAATCGACGATTGCTGGAAAGGCTGGGCCATACTCCTCCGGCAGAAGCAGAAA AAGCTTATTATGCTTCCATCGGAAACGATGATCTGGCAGCCTGAGTTCACAGATAAAACACTCTCCAG GAAACCCGGGGCGGTTCA |
| 1 6 2 | IS 10 | CTGATGAATCCCCTAATGATTTTGGTAAAAATCATTAAGTTAAGGTGGATACACATCTTGTCATATGA TCAAATGGTTTCGCGAAAAATCAATAATCAGACAACAAGATGTGCGAACTCGATATTTTACACGACTC TCTTTACCAATTCTGCCCCGAATTACACTTAAAACGACTCAACAGCTTAACGTTGGCTTGCCACGCAT TACTTGACTGTAAAACTCTCACTCTTACCGAACTTGGCCGTAACCTGCCAACCAAAGCGAGAACAAAA CATAACATCAAACGAATCGACCGATTGTTAGGTAATCGTCACCTCCACAAAGAGCGACTCGCTGTATA CCGTTGGCATGCTAGCTTTATCTGTTCGGGCAATACGATGCCCATTGTACTTGTTGACTGGTCTGATA TTCGTGAGCAAAAACGACTTATGGTATTGCGAGCTTCAGTCGCACTACACGGTCGTTCTGTTACTCTT TATGAGAAAGCGTTCCCGCTTTCAGAGCAATGTTCAAAGAAAGCTCATGACCAATTTCTAGCCGACCT TGCGAGCATTCTACCGAGTAACACCACACCGCTCATTGTCAGTGATGCTGGCTTTAAAGTGCCATGGT ATAAATCCGTTGAGAAGCTGGGTTGGTACTGGTTAAGTCGAGTAAGAGGAAAAGTACAATATGCAGAC CTAGGAGCGGAAAACTGGAAACCTATCAGCAACTTACATGATATGTCATCTAGTCACTCAAAGACTTT AGGCTATAAGAGGCTGACTAAAAGCAATCCAATCTCATGCCAAATTCTATTGTATAAATCTCGCTCTA AAGGCCGAAAAAATCAGCGCTCGACACGGACTCATTGTCACCACCCGTCACCTAAAATCTACTCAGCG TCGGCAAAGGAGCCATGGGTTCTAGCAACTAACTTACCTGTTGAAATTCGAACACCCAAACAACTTGT TAATATCTATTCGAAGCGAATGCAGATTGAAGAAACCTTCCGAGACTTGAAAAGTCCTGCCTACGGAC TAGGCCTACGCCATAGCCGAACGAGCAGCTCAGAGCGTTTTGATATCATGCTGCTAATCGCCCTGATG CTTCAACTAACATGTTGGCTTGCGGGCGTTCATGCTCAGAAACAAGGTTGGGACAAGCACTTCCAGGC TAACACAGTCAGAAATCGAAACGTACTCTCAACAGTTCGCTTAGGCATGGAAGTTTTGCGGCATTCTG GCTACACAATAACAAGGGAAGACTTACTCGTGGCTGCAACCCTACTAGCTCAAAATTTATTCACACAT GGTTACGCTTTGGGGAAATTATGAGGGGATCTCTCAG |
| 1 6 3 | IS 50 | CTGACTCTTATACACAAGTAGCGTCCTGAACGGAACCTTTCCCGTTTTCCAGGATCTGATCTTCCATG TGACCTCCTAACATGGTAACGTTCATGATAACTTCTGCTCTTCATCGTGCGGCCGACTGGGCTAAATC TGTGTTCTCTTCGGCGGCGCTGGGTGATCCTCGCCGTACTGCCCGCTTGGTTAACGTCGCCGCCCAAT TGGCAAAATATTCTGGTAAATCAATAACCATCTCATCAGAGGGTAGTGAAGCCATGCAGGAAGGCGCT TACCGATTTATCCGCAATCCCAACGTTTCTGCCGAGGCGATCAGAAAGGCTGGCGCCATGCAAACAGT CAAGTTGGCTCAGGAGTTTCCCGAACTGCTGGCCATTGAGGACACCACCTCTTTGAGTTATCGCCACC AGGTCGCCGAAGAGCTTGGCAAGCTGGGCTCTATTCAGGATAAATCCCGCGGATGGTGGGTTCACTCC GTTCTCTTGCTCGAGGCCACCACATTCCGCACCGTAGGATTACTGCATCAGGAGTGGTGGATGCGCCC GGATGACCCTGCCGATGCGGATGAAAAGGAGAGTGGCAAATGGCTGGCAGCGGCCGCAACTAGCCGGT TACGCATGGGCAGCATGATGAGCAACGTGATTGCGGTCTGTGACCGCGAAGCCGATATTCATGCTTAT CTGCAGGACAAACTGGCGCATAACGAGCGCTTCGTGGTGCGCTCCAAGCACCCACGCAAGGACGTAGA GTCTGGGTTGTATCTGTACGACCATCTGAAGAACCAACCGGAGTTGGGTGGCTATCAGATCAGCATTC CGCAAAAGGGCGTGGTGGATAAACGCGGTAAACGTAAAAATCGACCAGCCCGCAAGGCGAGCTTGAGC CTGCGCAGTGGGCGCATCACGCTAAAACAGGGGAATATCACGCTCAACGCGGTGCTGGCCGAGGAGAT TAACCCGCCCAAGGGTGAGACCCCGTTGAAATGGTTGTTGCTGACCAGCGAACCGGTCGAGTCGCTAG CCCAAGCCTTGCGCGTCATCGACATTTATACCCATCGCTGGCGGATCGAGGAGTTCCATAAGGCATGG AAAACCGGAGCAGGAGCCGAGAGGCAACGCATGGAGGAGCCGGATAATCTGGAGCGGATGGTCTCGAT CCTCTCGTTTGTTGCGGTCAGGCTGTTACAGCTCAGAGAAAGCTTCACGCTGCCGCAAGCACTCAGGG CGCAAGGGCTGCTAAAGGAAGCGGAACACGTAGAAAGCCAGTCCGCAGAAACGGTGCTGACCCCGGAT GAATGTCAGCTACTGGGCTATCTGGACAAGGGAAAACGCAAGCGCAAAGAGAAAGCAGGTAGCTTGCA GTGGGCTTACATGGCGATAGCTAGACTGGGCGGTTTTATGGACAGCAAGCGAACCGGAATTGCCAGCT GGGGCGCCCTCTGGGAAGGTTGGGAAGCCCTGCAAAGTAAACTGGATGGCTTTCTTGCCGCCAAGGAT CTGATGGCGCAGGGGATCAAGATCTGATCAAGAGACAG |
| 1 6 4 | IS 25 7 | GGTTCTGTTGCAAAGTTGAATTTATAGTATAATTTTAACAAAAAGGAGTCTTCTGTATGAACTATTTC AGATATAAACAATTTAACAAGGATGTTATCACTGTAGCCGTTGGCTACTATCTAAGATATGCATTGAG TTATCGTGATATATCTGAAATATTAAGGGAACGTGGTGTAAACGTTCATCATTCAACAGTCTACCGTT GGGTTCAAGAATATGCCCCAATTTTGTATCAAATTTGGAAGAAAAAGCATAAAAAAGCTTATTACAAA TGGCGTATTGATGAGACGTACATCAAAATAAAAGGAAAATGGAGCTATTTATATCGTGCCATTGATGC AGAGGGACATACATTAGATATTTGGTTGCGTAAGCAACGAGATAATCATTCAGCATATGCGTTTATCA AACGTCTCATTAAACAATTTGGTAAACCTCAAAAGGTAATTACAGATCAGGCACCTTCAACGAAAGTA GCAATAGCTAAAGTAATTAAAGCTTTTAAACTTAAACCTGACTGTCATTGTACATCGAAATATCTGAA TAACCTCATTGAGCAAGATCACCGTCATATTAAAGTAAGAAAGACAAGATATCAAAGTATCAATACGG CAAAAAATACACTCAAAGGCATTGAATGTATTTACGCTCTATATAAAAAGAACCGCAGATCTCTTCAG ATCTACGGATTTTCGCCATGCCACGAAATTAGGGATATGTTAGCCAGTTAAGGGAACAACGACATGAT AAAATGGTAGTTAGCTATATTTTTCTAACTTTGCAACAGAACC |
| 1 6 5 | IS 15 | GGCACTGTTGCAAATAGTCGGTGGTGATAAACTTATCATCCCCTTTTGCTGATGGAGCTGCACATGAA CCCATTCAAAGGCCGGCATTTTCAGCGTGACATCATTCTGTGGGCCGTACGCTGGTACTGCAAATACG GCATCAGTTACCGTGAGCTGCAGGAGATGCTGGCTGAACGCGGAGTGAATGTCGATCACTCCACGATT TACCGCTGGGTTCAGCGTTATGCGCCTGAAATGGAAAAACGGCTGCGCTGGTACTGGCGTAACCCTTC |
| | | CGATCTTTGCCCGTGGCACATGGATGAAACCTACGTGAAGGTCAATGGCCGCTGGGCGTATCTGTACC GGGCCGTCGACAGCCGGGGCCGCACTGTCGATTTTTATCTCTCCTCCCGTCGTAACAGCAAAGCTGCA TACCGGTTTCTGGGTAAAATCCTCAACAACGTGAAGAAGTGGCAGATCCCGCGATTCATCAACACGGA TAAAGCGCCCGCCTATGGTCGCGCGCTTGCTCTGCTCAAACGCGAAGGCCGGTGCCCGTCTGACGTTG AACACCGACAGATTAAGTACCGGAACAACGTGATTGAATGCGATCATGGCAAACTGAAACGGATAATC GGCGCCACGCTGGGATTTAAATCCATGAAGACGGCTTACGCGGCACTGTTGCAAATAGTCGGTGGTGA TAAACTTATCATCCCCTTTTGCTGATGGAGCTGCACATGAACCCATTCAAAGGCCGGCATTTTCAGCG TGACATCATTCTGTGGGCCGTACGCTGGTACTGCAAATACGGCATCAGTTACCGTGAGCTGCAGGAGA TGCTGGCTGAACGCGGAGTGAATGTCGATCACTCCACGATTTACCGCTGGGTTCAGCGTTATGCGCCT GAAATGGAAAAACGGCTGCGCTGGTACTGGCGTAACCCTTCCGATCTTTGCCCGTGGCACATGGATGA AACCTACGTGAAGGTCAATGGCCGCTGGGCGTATCTGTACCGGGCCGTCGACAGCCGGGGCCGCACTG TCGATTTTTATCTCTCCTCCCGTCGTAACAGCAAAGCTGCATACCGGTTTCTGGGTAAAATCCTCAAC AACGTGAAGAAGTGGCAGATCCCACGATTCATCAACACGGATAAAGCGCCCGCCTATGGTCGCGCGCT TGCTCTGCTCAAACGCGAAGGCCGGTGCCCGTCTGACGTTGAACACCGACAGATTAAGTACCGGAACA ACGTGATTGAATGCGATCATGGCAAACTGAAACGGATAATCAACGCCACGCTGGGATTTAAATCCATG AAGACGGCTTACGCCACCATCAAAGGTATTGAGGTGATGCGTGCACTACGCAAAGGCCAGGCCTCAGC ATTTTATTATGGTGATCCCCTGGGCGAAATGCGCCTGGTAAGCAGAGTTTTTGAAATGTAAGGCCTTT GAATAAGACAAAAGGCTGCCTCATCGCTAACTTTGCAACAGTGCCGCTTACGCCACCATCAAAGGTAT TGAGGTGATGCGTGCACTACGCAAAGGCCAGGCCTCAGCATTTTATTATGGTGATCCCCTGGGCGAAA TGCGCCTGGTAAGCAGAGTTTTTGAAACGTAAGGCCTTTGAATAAGACAAAAGGCTGCCTCATCGCTA ACTTTGCAACAGTGCC |
| 1 66 | IS 26 | GGCACTGTTGCAAATAGTCGGTGGTGATAAACTTATCATCCCCTTTTGCTGATGGAGCTGCACATGAA CCCATTCAAAGGCCGGCATTTTCAGCGTGACATCATTCTGTGGGCCGTACGCTGGTACTGCAAATACG GCATCAGTTACCGTGAGCTGCAGGAGATGCTGGCTGAACGCGGAGTGAATGTCGATCACTCCACGATT TACCGCTGGGTTCAGCGTTATGCGCCTGAAATGGAAAAACGGCTGCGCTGGTACTGGCGTAACCCTTC CGATCTTTGCCCGTGGCACATGGATGAAACCTACGTGAAGGTCAATGGCCGCTGGGCGTATCTGTACC GGGCCGTCGACAGCCGGGGCCGCACTGTCGATTTTTATCTCTCCTCCCGTCGTAACAGCAAAGCTGCA TACCGGTTTCTGGGTAAAATCCTCAACAACGTGAAGAAGTGGCAGATCCCGCGATTCATCAACACGGA TAAAGCGCCCGCCTATGGTCGCGCGCTTGCTCTGCTCAAACGCGAAGGCCGGTGCCCGTCTGACGTTG AACACCGACAGATTAAGTACCGGAACAACGTGATTGAATGCGATCATGGCAAACTGAAACGGATAATC GGCGCCACGCTGGGATTTAAATCCATGAAGACGGCTTACGCCACCATCAAAGGTATTGAGGTGATGCG TGCACTACGCAAAGGCCAGGCCTCAGCATTTTATTATGGTGATCCCCTGGGCGAAATGCGCCTGGTAA GCAGAGTTTTTGAAATGTAAGGCCTTTGAATAAGACAAAAGGCTGCCTCATCGCTAACTTTGCAACAG TGCC |
| 1 6 7 | Ac at sa c1 | Bold = binding site P1 for the Fp1 and Rp1 oligomers |
| | | NCBI Genbank MF124798.1 |
| | | GTGTAGGCTGGAGCTGCTTCGAAGAGACGTCAGGTGGCAAACGCAAAAAGCCCCGAGCGGTTAAACTC AGGGCTTTATTTTTATTAGGCGACCACAGGTTTGCGTGCAATGGAAATTTCACACTGCTCAACCGAAG |
| | | TGTAATCCTTGTTGTGATTGGTTACATCCAGTTTGCGGTCAACATAGTGATACCCTGGCATCTTCACA GGCTTCTTTGCCTTGTAAGTAGTTTTAAATTCACACAAATAGTGACCGCCTCCTTCTAACTTCAGAGC CATAAAGTTGTTTCCTAGCAGCATTCCATCTCGTGCAAAGAGACGCTCAGTGTTGGGTTCCCAGCCCT GTGTCTTCTTCTGCATGACAGGTCCATTGGGAGGAAAGTTCAAACCAGAGAACTTGACATGGTAGATG AAACAGTTGCCTTGGATGCTGGAATCATTGCTGACAGTACACACTGCACCATCTTCAAAGTTCATGAT CCTCTCCCATGTATAGCCCTCCGGGAATGACTGCTTTACATAGTCAGGGATGTCTTCAGGGTACTTGG TGAATGGTATGCTTCCGTACTGACACTGTGGTGATAAAATATCCCAAGCAAATGGCAGAGGTCCGCCC TTGGTGACAGTGAGCTTTACCGTCTGCTCCCCCTCGTAGGGCTTACCTTTTCCATCGCCTTCGACCTC AAAGTAGTGTCCATTGACCGTGCCTGACATATAAACCTTGTAGGTCATTTGTTTAGCGATCACACTCA TCTAGTATTTCTCCTCTTTCTCTAGTAACAGTACTATGTGATTATACCAGCCCCCTCACTACATGTCA AGAATAAACTGCCAAAGCTCTAGAAGCGGCCGCGAATTCGTTTACAGCTAGCTCAGTCCTAGGTATTA TGCTAGCGTTGATCGGGCACGTAAGAGGTTCCAACTTTCACCATAATGAAATAAGATCACTACCGGGC GTATTTTTTGAGTTATCGAGATTTTCAGGAGCTAAGGAAGCTAAAATGGAGAAAAAAATCACGGGATA TACCACCGTTGATATATCCCAATGGCATCGTAAAGAACATTTTGAGGCATTTCAGTCAGTTGCTCAAT GTACCTATAACCAGACCGTTCAGCTGGATATTACGGCCTTTTTAAAGACCGTAAAGAAAAATAAGCAC AAGTTTTATCCGGCCTTTATTCACATTCTTGCCCGCCTGATGAACGCTCACCCGGAGTTTCGTATGGC CATGAAAGACGGTGAGCTGGTGATCTGGGATAGTGTTCACCCTTGTTACACCGTTTTCCATGAGCAAA CTGAAACGTTTTCGTCCCTCTGGAGTGAATACCACGACGATTTCCGGCAGTTTCTCCACATATATTCG CAAGATGTGGCGTGTTACGGTGAAAACCTGGCCTATTTCCCTAAAGGGTTTATTGAGAATATGTTTTT TGTCTCAGCCAATCCCTGGGTGAGTTTCACCAGTTTTGATTTAAACGTGGCCAATATGGACAACTTCT TCGCCCCCGTTTTCACGATGGGCAAATATTATACGCAAGGCGACAAGGTGCTGATGCCGCTGGCGATC CAGGTTCATCATGCCGTTTGTGATGGCTTCCATGTCGGCCGCATGCTTAATGAATTACAACAGTACTG TGATGAGTGGCAGGGCGGGGCGTAATAATACTAGAGGAGACATGAACGATGAACATCAAAAAGTTTGC AAAACAAGCAACAGTATTAACCTTTACTACCGCACTGCTGGCAGGAGGCGCAACTCAAGCGTTTGCGA AAGAAACGAACCAAAAGCCATATAAGGAAACATACGGCATTTCCCATATTACACGCCATGATATGCTG CAAATCCCTGAACAGCAAAAAAATGAAAAATATCAAGTTCCTGAGTTCGATTCGTCCACAATTAAAAA TATCTCTTCTGCAAAAGGCCTGGACGTTTGGGACAGCTGGCCATTACAAAACGCTGACGGCACTGTCG CAAACTATCACGGCTACCACATCGTCTTTGCATTAGCCGGAGATCCTAAAAATGCGGATGACACATCG ATTTACATGTTCTATCAAAAAGTCGGCGAAACTTCTATTGACAGCTGGAAAAACGCTGGCCGCGTCTT TAAAGACAGCGACAAATTCGATGCAAATGATTCTATCCTAAAAGACCAAACACAAGAATGGTCAGGTT CAGCCACATTTACATCTGACGGAAAAATCCGTTTATTCTACACTGATTTCTCCGGTAAACATTACGGC AAACAAACACTGACAACTGCACAAGTTAACGTATCAGCATCAGACAGCTCTTTGAACATCAACGGTGT AGAGGATTATAAATCAATCTTTGACGGTGACGGAAAAACGTATCAAAATGTACAGCAGTTCATCGATG AAGGCAACTACAGCTCAGGCGACAACCATACGCTGAGAGATCCTCACTACGTAGAAGATAAAGGCCAC AAATACTTAGTATTTGAAGCAAACACTGGAACTGAAGATGGCTACCAAGGCGAAGAATCTTTATTTAA CAAAGCATACTATGGCAAAAGCACATCATTCTTCCGTCAAGAAAGTCAAAAACTTCTGCAAAGCGATA AAAAACGCACGGCTGAGTTAGCAAACGGCGCTCTCGGTATGATTGAGCTAAACGATGATTACACACTG AAAAAAGTGATGAAACCGCTGATTGCATCTAACACAGTAACAGATGAAATTGAACGCGCGAACGTCTT TAAAATGAACGGCAAATGGTACCTGTTCACTGACTCCCGCGGATCAAAAATGACGATTGACGGCATTA |
| | | CGTCTAACGATATTTACATGCTTGGTTATGTTTCTAATTCTTTAACTGGCCCATACAAGCCGCTGAAC AAAACTGGCCTTGTGTTAAAAATGGATCTTGATCCTAACGATGTAACCTTTACTTACTCACACTTCGC TGTACCTCAAGCGAAAGGAAACAATGTCGTGATTACAAGCTATATGACAAACAGAGGATTCTACGCAG ACAAACAATCAACGTTTGCGCCAAGCTTCCTGCTGAACATCAAAGGCAAGAAAACATCTGTTGTCAAA GACAGCATCCTTGAACAAGGACAATTAACAGTTAACAAATAATAAATTTGGCTTGGACTCCTGTTGAT AGATCCAGTAATGACCTCAGAACTCCATCTGGATTTGTTCAGAACGCTCGGTTGCCGCCGGGCGTTTT TTATTGGTGAGAATCCAAGCACTAGGAACTAAGGAGGATATTCATATG |
| 1 6 8 | Ac at sa c3 | **Bold = binding site P1 for the Fp1 and Rp1 oligomers** |
| | | NCBI Genbank MF124799.1 |
| | | GTGTAGGCTGGAGCTGCTTCGAAGAGACGTCAGGTGGCAAACGCAAAAAGCCCCGAGCGGTTAAACTC AGGGCTTTATTTTATTAGGCGACCACAGGTTTGCGTGCAATGGAAATTTCACACTGCTCAACCGAAG TGTAATCCTTGTTGTGATTGGTTACATCCAGTTTGCGGTCAACATAGTGATACCCTGGCATCTTCACA GGCTTCTTTGCCTTGTAAGTAGTTTTAAATTCACACAAATAGTGACCGCCTCCTTCTAACTTCAGAGC CATAAAGTTGTTTCCTAGCAGCATTCCATCTCGTGCAAAGAGACGCTCAGTGTTGGGTTCCCAGCCCT GTGTCTTCTTCTGCATGACAGGTCCATTGGGAGGAAAGTTCAAACCAGAGAACTTGACATGGTAGATG AAACAGTTGCCTTGGATGCTGGAATCATTGCTGACAGTACACACTGCACCATCTTCAAAGTTCATGAT CCTCTCCCATGTATAGCCCTCCGGGAATGACTGCTTTACATAGTCAGGGATGTCTTCAGGGTACTTGG TGAATGGTATGCTTCCGTACTGACACTGTGGTGATAAAATATCCCAAGCAAATGGCAGAGGTCCGCCC TTGGTGACAGTGAGCTTTACCGTCTGCTCCCCCTCGTAGGGCTTACCTTTTCCATCGCCTTCGACCTC AAAGTAGTGTCCATTGACCGTGCCTGACATATAAACCTTGTAGGTCATTTGTTTAGCGATCACACTCA TCTAGTATTTCTCCTCTTTCTCTAGTAACAGTACTATGTGATTATACCAGCCCCCTCACTACATGTCA AGAATAAACTGCCAAAGCTCTAGACATATGAATATCCTCCTTAGTTCCTAGTGCTTGGATTCTCACCA ATAAAAAACGCCCGGCGGCAACCGAGCGTTCTGAACAAATCCAGATGGAGTTCTGAGGTCATTACTGG ATCTATCAACAGGAGTCCAAGCCAAATTTATTATTTGTTAACTGTTAATTGTCCTTGTTCAAGGATGC TGTCTTTGACAACAGATGTTTTCTTGCCTTTGATGTTCAGCAGGAAGCTTGGCGCAAACGTTGATTGT TTGTCTGCGTAGAATCCTCTGTTTGTCATATAGCTTGTAATCACGACATTGTTTCCTTTCGCTTGAGG TACAGCGAAGTGTGAGTAAGTAAAGGTTACATCGTTAGGATCAAGATCCATTTTTAACACAAGGCCAG TTTTGTTCAGCGGCTTGTATGGGCCAGTTAAAGAATTAGAAACATAACCAAGCATGTAAATATCGTTA GACGTAATGCCGTCAATCGTCATTTTTGATCCGCGGGAGTCAGTGAACAGGTACCATTTGCCGTTCAT TTTAAAGACGTTCGCGCGTTCAATTTCATCTGTTACTGTGTTAGATGCAATCAGCGGTTTCATCACTT TTTTCAGTGTGTAATCATCGTTTAGCTCAATCATACCGAGAGCGCCGTTTGCTAACTCAGCCGTGCGT TTTTTATCGCTTTGCAGAAGTTTTTGACTTTCTTGACGGAAGAATGATGTGCTTTTGCCATAGTATGC TTTGTTAAATAAAGATTCTTCGCCTTGGTAGCCATCTTCAGTTCCAGTGTTTGCTTCAAATACTAAGT ATTTGTGGCCTTTATCTTCTACGTAGTGAGGATCTCTCAGCGTATGGTTGTCGCCTGAGCTGTAGTTG CCTTCATCGATGAACTGCTGTACATTTTGATACGTTTTCCGTCACCGTCAAAGATTGATTTATAATC CTCTACACCGTTGATGTTCAAAGAGCTGTCTGATGCTGATACGTTAACTTGTGCAGTTGTCAGTGTTT GTTTGCCGTAATGTTTACCGGAGAAATCAGTGTAGAATAAACGGATTTTTCCGTCAGATGTAAATGTG GCTGAACCTGACCATTCTTGTGTTTGGTCTTTTAGGATAGAATCATTTGCATCGAATTTGTCGCTGTC |
| | | TTTAAAGACGCGGCCAGCGTTTTTCCAGCTGTCAATAGAAGTTTCGCCGACTTTTTGATAGAACATGT AAATCGATGTGTCATCCGCATTTTTAGGATCTCCGGCTAATGCAAAGACGATGTGGTAGCCGTGATAG TTTGCGACAGTGCCGTCAGCGTTTTGTAATGGCCAGCTGTCCCAAACGTCCAGGCCTTTTGCAGAAGA GATATTTTTAATTGTGGACGAATCGAACTCAGGAACTTGATATTTTTCATTTTTTTGCTGTTCAGGGA TTTGCAGCATATCATGGCGTGTAATATGGGAAATGCCGTATGTTTCCTTATATGGCTTTTGGTTCGTT TCTTTCGCAAACGCTTGAGTTGCGCCTCCTGCCAGCAGTGCGGTAGTAAAGGTTAATACTGTTGCTTG TTTTGCAAACTTTTTGATGTTCATCGTTCATGTCTCCTCTAGTATTATTACGCCCCGCCCTGCCACTC ATCACAGTACTGTTGTAATTCATTAAGCATGCGGCCGACATGGAAGCCATCACAAACGGCATGATGAA CCTGGATCGCCAGCGGCATCAGCACCTTGTCGCCTTGCGTATAATATTTGCCCATCGTGAAAACGGGG GCGAAGAAGTTGTCCATATTGGCCACGTTTAAATCAAAACTGGTGAAACTCACCCAGGGATTGGCTGA GACAAAAAACATATTCTCAATAAACCCTTTAGGGAAATAGGCCAGGTTTTCACCGTAACACGCCACAT CTTGCGAATATATGTGGAGAAACTGCCGGAAATCGTCGTGGTATTCACTCCAGAGGGACGAAAACGTT TCAGTTTGCTCATGGAAAACGGTGTAACAAGGGTGAACACTATCCCAGATCACCAGCTCACCGTCTTT CATGGCCATACGAAACTCCGGGTGAGCGTTCATCAGGCGGGCAAGAATGTGAATAAAGGCCGGATAAA ACTTGTGCTTATTTTTCTTTACGGTCTTTAAAAAGGCCGTAATATCCAGCTGAACGGTCTGGTTATAG GTACATTGAGCAACTGACTGAAATGCCTCAAAATGTTCTTTACGATGCCATTGGGATATATCAACGGT GGTATATCCCGTGATTTTTTTCTCCATTTTAGCTTCCTTAGCTCCTGAAAATCTCGATAACTCAAAAA ATACGCCCGGTAGTGATCTTATTTCATTATGGTGAAAGTTGGAACCTCTTACGTGCCCGATCAACGCT AGCATAATACCTAGGACTGAGCTAGCTGTAAAC |
| 1 6 9 | Ak an sa c1 | **Bold = binding site P1 for the Fp1 and Rp1 oligomers** |
| | | GTGTAGGCTGGAGCTGCTTCGAAGAGACGTCAGGTGGCAAACGCAAAAAGCCCCGAGCGGTTAAACTC AGGGCTTTATTTTATTAGGCGACCACAGGTTTGCGTGCAATGGAAATTTCACACTGCTCAACCGAAG TGTAATCCTTGTTGTGATTGGTTACATCCAGTTTGCGGTCAACATAGTGATACCCTGGCATCTTCACA GGCTTCTTTGCCTTGTAAGTAGTTTTAAATTCACACAAATAGTGACCGCCTCCTTCTAACTTCAGAGC CATAAAGTTGTTTCCTAGCAGCATTCCATCTCGTGCAAAGAGACGCTCAGTGTTGGGTTCCCAGCCCT GTGTCTTCTTCTGCATGACAGGTCCATTGGGAGGAAAGTTCAAACCAGAGAACTTGACATGGTAGATG AAACAGTTGCCTTGGATGCTGGAATCATTGCTGACAGTACACACTGCACCATCTTCAAAGTTCATGAT CCTCTCCCATGTATAGCCCTCCGGGAATGACTGCTTTACATAGTCAGGGATGTCTTCAGGGTACTTGG TGAATGGTATGCTTCCGTACTGACACTGTGGTGATAAAATATCCCAAGCAAATGGCAGAGGTCCGCCC TTGGTGACAGTGAGCTTTACCGTCTGCTCCCCCTCGTAGGGCTTACCTTTTCCATCGCCTTCGACCTC AAAGTAGTGTCCATTGACCGTGCCTGACATATAAACCTTGTAGGTCATTTGTTTAGCGATCACACTCA TCTAGTATTTCTCCTCTTTCTCTAGTAACAGTACTATGTGATTATACCAGCCCCCTCACTACATGTCA AGAATAAACTGCCAAAGCTCTAGAAGCGGCCGCGAATTCGTTTACAGCTAGCTCAGTCCTAGGTATTA TGCTAGCGTTGATCGGGCACGTAAGAGGTTCCAACTTTCACCATAATGAAATAAGATCACTACCGGGC GTATTTTTTGAGTTATCGAGATTTTCAGGAGCTAAGGAAGCTAAAATGAGCCATATTCAACGGGAAAC GTCTTGCTCCCGTCCGCGCTTAAACTCCAACATGGACGCTGATTTATATGGGTATAAATGGGCTCGCG ATAATGTCGGGCAATCAGGTGCGACAATCTATCGCTTGTATGGGAAGCCCGATGCGCCAGAGTTGTTT CTGAAACATGGCAAAGGTAGCGTTGCCAATGATGTTACAGATGAGATGGTCCGTCTCAACTGGCTGAC |
| | | GGAGTTTATGCCTCTCCCGACCATCAAGCATTTTATCCGTACTCCTGATGATGCGTGGTTACTCACCA CCGCGATTCCTGGGAAAACAGCCTTCCAGGTATTAGAAGAATATCCTGATTCAGGTGAAAATATTGTT GATGCGCTGGCCGTGTTCCTGCGCCGGTTACATTCGATTCCTGTTTGTAATTGTCCTTTTAACAGCGA TCGTGTATTTCGTCTTGCTCAGGCGCAATCACGCATGAATAACGGTTTGGTTGATGCGAGTGATTTTG ATGACGAGCGTAATGGCTGGCCTGTTGAACAAGTCTGGAAAGAAATGCACAAGCTCTTGCCATTCTCA CCGGATTCAGTCGTCACTCATGGTGATTTCTCACTTGATAACCTTATTTTTGACGAGGGGAAATTAAT AGGTTGTATTGATGTTGGACGGGTCGGAATCGCAGACCGTTACCAGGACCTTGCCATTCTTTGGAACT GCCTCGGTGAGTTTTCTCCTTCATTACAGAAACGGCTTTTTCAAAAATATGGTATTGATAATCCTGAT ATGAATAAATTGCAGTTTCATTTGATGCTCGATGAGTTTTTCTAATAATACTAGAGGAGACATGAACG ATGAACATCAAAAAGTTTGCAAAACAAGCAACAGTATTAACCTTTACTACCGCACTGCTGGCAGGAGG CGCAACTCAAGCGTTTGCGAAAGAAACGAACCAAAAGCCATATAAGGAAACATACGGCATTTCCCATA TTACACGCCATGATATGCTGCAAATCCCTGAACAGCAAAAAAATGAAAAATATCAAGTTCCTGAGTTC GATTCGTCCACAATTAAAAATATCTCTTCTGCAAAAGGCCTGGACGTTTGGGACAGCTGGCCATTACA AAACGCTGACGGCACTGTCGCAAACTATCACGGCTACCACATCGTCTTTGCATTAGCCGGAGATCCTA AAAATGCGGATGACACATCGATTTACATGTTCTATCAAAAAGTCGGCGAAACTTCTATTGACAGCTGG AAAAACGCTGGCCGCGTCTTTAAAGACAGCGACAAATTCGATGCAAATGATTCTATCCTAAAAGACCA AACACAAGAATGGTCAGGTTCAGCCACATTTACATCTGACGGAAAAATCCGTTTATTCTACACTGATT TCTCCGGTAAACATTACGGCAAACAAACACTGACAACTGCACAAGTTAACGTATCAGCATCAGACAGC TCTTTGAACATCAACGGTGTAGAGGATTATAAATCAATCTTTGACGGTGACGGAAAAACGTATCAAAA TGTACAGCAGTTCATCGATGAAGGCAACTACAGCTCAGGCGACAACCATACGCTGAGAGATCCTCACT ACGTAGAAGATAAAGGCCACAAATACTTAGTATTTGAAGCAAACACTGGAACTGAAGATGGCTACCAA GGCGAAGAATCTTTATTTAACAAAGCATACTATGGCAAAAGCACATCATTCTTCCGTCAAGAAAGTCA AAAACTTCTGCAAAGCGATAAAAAACGCACGGCTGAGTTAGCAAACGGCGCTCTCGGTATGATTGAGC TAAACGATGATTACACACTGAAAAAAGTGATGAAACCGCTGATTGCATCTAACACAGTAACAGATGAA ATTGAACGCGCGAACGTCTTTAAAATGAACGGCAAATGGTACCTGTTCACTGACTCCCGCGGATCAAA AATGACGATTGACGGCATTACGTCTAACGATATTTACATGCTTGGTTATGTTTCTAATTCTTTAACTG GCCCATACAAGCCGCTGAACAAAACTGGCCTTGTGTTAAAAATGGATCTTGATCCTAACGATGTAACC TTTACTTACTCACACTTCGCTGTACCTCAAGCGAAAGGAAACAATGTCGTGATTACAAGCTATATGAC AAACAGAGGATTCTACGCAGACAAACAATCAACGTTTGCGCCAAGCTTCCTGCTGAACATCAAAGGCA AGAAAACATCTGTTGTCAAAGACAGCATCCTTGAACAAGGACAATTAACAGTTAACAAATAATAAATT TGGCTTGGACTCCTGTTGATAGATCCAGTAATGACCTCAGAACTCCATCTGGATTTGTTCAGAACGCT CGGTTGCCGCCGGGCGTTTTTTATTGGTGAGAATCCAAGCACTAGGAACTAAGGAGGATATTCATATG |
| 1 7 0 | Ak an sa c3 | **Bold = binding site P1 for the Fp1 and Rp1 oligomers** |
| | | GTGTAGGCTGGAGCTGCTTCGAAGAGACGTCAGGTGGCAAACGCAAAAAGCCCCGAGCGGTTAAACTC AGGGCTTTATTTTATTAGGCGACCACAGGTTTGCGTGCAATGGAAATTTCACACTGCTCAACCGAAG TGTAATCCTTGTTGTGATTGGTTACATCCAGTTTGCGGTCAACATAGTGATACCCTGGCATCTTCACA GGCTTCTTTGCCTTGTAAGTAGTTTTAAATTCACACAAATAGTGACCGCCTCCTTCTAACTTCAGAGC |
| | | CATAAAGTTGTTTCCTAGCAGCATTCCATCTCGTGCAAAGAGACGCTCAGTGTTGGGTTCCCAGCCCT GTGTCTTCTTCTGCATGACAGGTCCATTGGGAGGAAAGTTCAAACCAGAGAACTTGACATGGTAGATG AAACAGTTGCCTTGGATGCTGGAATCATTGCTGACAGTACACACTGCACCATCTTCAAAGTTCATGAT CCTCTCCCATGTATAGCCCTCCGGGAATGACTGCTTTACATAGTCAGGGATGTCTTCAGGGTACTTGG TGAATGGTATGCTTCCGTACTGACACTGTGGTGATAAAATATCCCAAGCAAATGGCAGAGGTCCGCCC TTGGTGACAGTGAGCTTTACCGTCTGCTCCCCCTCGTAGGGCTTACCTTTTCCATCGCCTTCGACCTC AAAGTAGTGTCCATTGACCGTGCCTGACATATAAACCTTGTAGGTCATTTGTTTAGCGATCACACTCA TCTAGTATTTCTCCTCTTTCTCTAGTAACAGTACTATGTGATTATACCAGCCCCCTCACTACATGTCA AGAATAAACTGCCAAAGCTCTAGACATATGAATATCCTCCTTAGTTCCTAGTGCTTGGATTCTCACCA ATAAAAAACGCCCGGCGGCAACCGAGCGTTCTGAACAAATCCAGATGGAGTTCTGAGGTCATTACTGG ATCTATCAACAGGAGTCCAAGCCAAATTTATTATTTGTTAACTGTTAATTGTCCTTGTTCAAGGATGC TGTCTTTGACAACAGATGTTTTCTTGCCTTTGATGTTCAGCAGGAAGCTTGGCGCAAACGTTGATTGT TTGTCTGCGTAGAATCCTCTGTTTGTCATATAGCTTGTAATCACGACATTGTTTCCTTTCGCTTGAGG TACAGCGAAGTGTGAGTAAGTAAAGGTTACATCGTTAGGATCAAGATCCATTTTTAACACAAGGCCAG TTTTGTTCAGCGGCTTGTATGGGCCAGTTAAAGAATTAGAAACATAACCAAGCATGTAAATATCGTTA GACGTAATGCCGTCAATCGTCATTTTTGATCCGCGGGAGTCAGTGAACAGGTACCATTTGCCGTTCAT TTTAAAGACGTTCGCGCGTTCAATTTCATCTGTTACTGTGTTAGATGCAATCAGCGGTTTCATCACTT TTTTCAGTGTGTAATCATCGTTTAGCTCAATCATACCGAGAGCGCCGTTTGCTAACTCAGCCGTGCGT TTTTTATCGCTTTGCAGAAGTTTTTGACTTTCTTGACGGAAGAATGATGTGCTTTTGCCATAGTATGC TTTGTTAAATAAAGATTCTTCGCCTTGGTAGCCATCTTCAGTTCCAGTGTTTGCTTCAAATACTAAGT ATTTGTGGCCTTTATCTTCTACGTAGTGAGGATCTCTCAGCGTATGGTTGTCGCCTGAGCTGTAGTTG CCTTCATCGATGAACTGCTGTACATTTTGATACGTTTTTCCGTCACCGTCAAAGATTGATTTATAATC CTCTACACCGTTGATGTTCAAAGAGCTGTCTGATGCTGATACGTTAACTTGTGCAGTTGTCAGTGTTT GTTTGCCGTAATGTTTACCGGAGAAATCAGTGTAGAATAAACGGATTTTTCCGTCAGATGTAAATGTG GCTGAACCTGACCATTCTTGTGTTTGGTCTTTTAGGATAGAATCATTTGCATCGAATTTGTCGCTGTC TTTAAAGACGCGGCCAGCGTTTTTCCAGCTGTCAATAGAAGTTTCGCCGACTTTTTGATAGAACATGT AAATCGATGTGTCATCCGCATTTTTAGGATCTCCGGCTAATGCAAAGACGATGTGGTAGCCGTGATAG TTTGCGACAGTGCCGTCAGCGTTTTGTAATGGCCAGCTGTCCCAAACGTCCAGGCCTTTTGCAGAAGA GATATTTTTAATTGTGGACGAATCGAACTCAGGAACTTGATATTTTTCATTTTTTTGCTGTTCAGGGA TTTGCAGCATATCATGGCGTGTAATATGGGAAATGCCGTATGTTTCCTTATATGGCTTTTGGTTCGTT TCTTTCGCAAACGCTTGAGTTGCGCCTCCTGCCAGCAGTGCGGTAGTAAAGGTTAATACTGTTGCTTG TTTTGCAAACTTTTTGATGTTCATCGTTCATGTCTCCTCTAGTATTATTAGAAAAACTCATCGAGCAT CAAATGAAACTGCAATTTATTCATATCAGGATTATCAATACCATATTTTTGAAAAAGCCGTTTCTGTA ATGAAGGAGAAAACTCACCGAGGCAGTTCCAAAGAATGGCAAGGTCCTGGTAACGGTCTGCGATTCCG ACCCGTCCAACATCAATACAACCTATTAATTTCCCCTCGTCAAAAATAAGGTTATCAAGTGAGAAATC ACCATGAGTGACGACTGAATCCGGTGAGAATGGCAAGAGCTTGTGCATTTCTTTCCAGACTTGTTCAA CAGGCCAGCCATTACGCTCGTCATCAAAATCACTCGCATCAACCAAACCGTTATTCATGCGTGATTGC GCCTGAGCAAGACGAAATACACGATCGCTGTTAAAAGGACAATTACAAACAGGAATCGAATGTAACCG GCGCAGGAACACGGCCAGCGCATCAACAATATTTTCACCTGAATCAGGATATTCTTCTAATACCTGGA |
| | | AGGCTGTTTTCCCAGGAATCGCGGTGGTGAGTAACCACGCATCATCAGGAGTACGGATAAAATGCTTG ATGGTCGGGAGAGGCATAAACTCCGTCAGCCAGTTGAGACGGACCATCTCATCTGTAACATCATTGGC AACGCTACCTTTGCCATGTTTCAGAAACAACTCTGGCGCATCGGGCTTCCCATACAAGCGATAGATTG TCGCACCTGATTGCCCGACATTATCGCGAGCCCATTTATACCCATATAAATCAGCGTCCATGTTGGAG TTTAAGCGCGGACGGGAGCAAGACGTTTCCCGTTGAATATGGCTCATTTTAGCTTCCTTAGCTCCTGA AAATCTCGATAACTCAAAAAATACGCCCGGTAGTGATCTTATTTCATTATGGTGAAAGTTGGAACCTC TTACGTGCCCGATCAACGCTAGCATAATACCTAGGACTGAGCTAGCTGTAAAC |
| 2 0 2 | IS 21 | TGTCAGCGCCAGTGATATAAGACGGTAATTCACCATTTGGATTGTCCGCTCCACCCAACATGTTGTTT CCTTAAGGTTCTCACACCAGAAAGGACATCAACATGCTGAGCAGAGAGGACTTTTACATGATAAAGCA AATGCGCCAGCAGGGCGCGTACATTGTCGATATTGCGACTCAGATTGGTTGCTCTGAACGGACGGTCA GACGCTACCTCAAATACCCTGAACCGCCAGCCAGAAAGACCCGCCACAAAATGGTTAAGCTGAAACCG TTTATGGATTACATCGACATGCGCCTGGCAGAGAATGTCTGGAATAGTGAGGTTATCTTTGCGGAGAT TAAGGCAATGGGTTATACGGGCGGACGTTCCATGCTGCGTTACTACATCCAGCCCAAACGTAAAATGC GTCCGTCAAAAAGAACAGTTCGCTTCGAAACTCAGCCTAGATACCAGCTCCAGCACGACTGGGGCGAA GTTGAGGTGGAGGTTGCCGGGCAACGGTGCAAAGTTAACTTTGCGGTTAATACGCTGGGGTTCTCCCG CCGCTTCCATGTCTTCGCCGCACCAAAACAGGATGCTGAGCATACCTACGAATCACTGGTTCGCGCCT TCCGCTACTTCGGTGGTTGTGTGAAAACGGTGCTGGTTGATAACCAGAAGGCTGCGGTGCTGAAGAAT AACAACGGGAAAGTCGTGTTCAACTCCGGATTCCTGTTGCTGGCCGACCACTATAACTTCCTGCCACG GGCATGCCGTCCACGCAGGGCCAGAACAAAAGGTAAGGTTGAGCGGATGGTGAAATACCTCAAGGAGA ACTTCTTCGTTCGGTACCGCAGGTTCGACAGCTTCACTCATGTTAATCAACAACTGGAGCAATGGATA GCCGATGTGGCTGACAAACGGGAACTTCGCCAGTTCAAAGAAACGCCGGAACAGCGCTTCGCGCGTGA GCAGGAACATCTGCAGCCGTTACCGGATACGGACTTCGATACCAGTTACTTCGACATCCGCCATGTGT CCTGGGACAGCTATATCGAGGTTGGTGGTAATCGTTACAGCGTTCCCGAAGCGCTGTGTGGTCAGCCG GTATCGATACGAATATCGCTGGATGACGAGTTGCGGATCTACAGTAATGAGAAACTGGTGGCCTCACA TCGCCTCTGTTCGGCATCGTCTGGCTGGCAGACAGTGCCGGAGCATCACGCCCCGCTCTGGCAGCAGG TCAGTCAGGTGGAACATCGACCACTGAGTGCCTATGAGGAGCTGTTGTGATGCATGAGCTGGAAGTCC TGCTGAGTCGCCTGAAAATGGAGCATCTGAGTTATCACGTTGAAAGCCTGCTGGAACAGGCAGCTAAA AAAGAGCTGAACTACCGGGAGTTCCTGTGCATGGCGCTACAGCAGGAATGGAACGGCAGGCATCAGCG CGGTATGGAGTCCAGGCTGAAGCAGGCTCGTCTGCCGTGGGTCAAAACGCTGGAGCAGTTCGACTTTA CCTTCCAGCCGGGCATCGACCGTAAGGTTGTCCGGGAACTGGCTGGTCTGGCGTTCGTGGAGCGCAGC GAAAACGTGATCCTGCTGGGACCTCCTGGTGTCGGAAAAACTCATCTGGCCATAGCTCTTGGCGTGAA AGCGGTGGATGCGGGACATCGGGTACTGTTTATGCCACTGGACAGACTGATCGCGACACTGATGAAAG CGAAAGAGGAAAACCGGCTGGAGCGTCAGCTGCAGCAACTGAGTTATGCCCGGGTGTTGATCCTGGAT GAAATAGGCTATCTGCCCATGAACAGAGAGGAAGCCAGCCTGTTCTTCCGGCTACTGAACCGTCGATA TGAAAAAGCGAGCATCATACTGACGTCAAACAAAGGGTTCGCAGACTGGGGAGAAATGTTCGGAGATC ACGTGCTGGCAACAGCGATACTGGATCGGTTGCTACATCACTCAACCACGCTGAATATCAAAGGAGAG AGTTACCGGTTAAAAGAGAAACGTAAAGCTGGAGTGCTGACCAAAAACACAACGCCAATCAGTGATGA TGAAATGGTGAAAAGCGGACAGCATCAGTAACGAAAGTATCTTAGCGGGCATGAAAATGGCAAATAAC GGTCAAACATCGTGGCGTTGACA |
| 2 0 3 | IS 20 0 | TGTCTATGGAAACCCCCAGCTAGGCTGGGGGTTCCGGAAAGCTTTCAGCTTTAAGCCAGTTATTAAAA CCCCTTTTGATTTGTTAAAACATCTTGCGGTCTGGCAACTGCAAAAGTTCAACAAGAAATCAAAAGGG GGTCCAATGGGGGACGAAAAGAGCTTAGCGCACACCCGATGGAACTGTAAATATCACATAGTTTTCGC GCCCAAATACCGAAGACAAGCGTTCTATGGAGAGAAGCGTAGGGCAGTAGGCAGCATATTAAGAAAAT TGTGTGAATGGAAAAACGTACGAATTCTGGAAGCAGAATGTTGTGCATATCATATTCACATGCTTCTG GAGATCCCGCCGAAGATGAGTGTGTCGAGTTTCATGGGATATCTGAAGGGTAAAAGTAGTCTGATGCT TTACGAGCAGTTTGGGGATCTAAAATTCAAATACAGGAACAGGGAGTTCTGGTGCAGAGGGTACTATG TCGATACGGTGGGTAAGAACACGGCGAAGATACAGGACTACATAAAGCACCAGCTTGAAGAGGATAAA ATGGGTGAGCAATTATCCATCCCCTATCCGGGCAGCCCGATTTACGGGCCGTAAGTAACGAAGTTTGA TGCAAATGTCAGATCGTATGCGCCTGTTAGGGCGCGGCTGGTAAGAGAGCCTTATAGGCGCATCTGAA AAACCTCGGCTATGCCGGAGGATATTTAT |
| 2 0 4 | IS 25 6 | GATAAAGTCCGTATAATTGTGTAAAAGTAAAAAGGCCATATAACAGTCCTTTTACGGTACAATGTTTT TAACGACAAAAACATACCCAGGAGGACTTTTACATGACCCAAGTACATTTTACACTGAAAAGCGAAGA GATTCAAAGCATTATTGAATATTCTGTAAAGGATGACGTTTCTAAAAATATTTTAACAACGGTATTTA ATCAACTAATGGAAAATCAACGAACAGAATATATTCAAGCAAAAGAATATGAACGAACAGAAAACCGA CAAAGTCAACGAAATGGCTATTATGAGCGCAGCTTTACGACACGTGTAGGCACGCTAGAATTAAAAGT ACCCAGAACACGTGATGGCCATTTTTCACCCACAGTGTTTGAACGTTATCAACGAAACGAAAAAGCCC TCATGGCTTCAATGTTGGAAATGTATGTATCAGGCGTTTCAACTCGTAAAGTATCAAAAATTGTGGAA GAACTTTGTGGTAAATCCGTCTCTAAGTCCTTCGTTTCTAGCTTAACAGAACAGCTAGAACCTATGGT TAACGAGTGGCAGAATCGTTTATTATCAGAAAAAAATTATCCTTACTTAATGACCGATGTACTCTATA TAAAAGTACGAGAAGAAAATCGAGTACTCTCAAAAAGCTGTCATATAGCGATTGGAATAACCAAAGAT GGCGACCGTGAAATTATCGGCTTCATGATTCAAAGTGGCGAAAGCGAAGAGACCTGGACAACATTTTT TGAATACCTAAAAGAACGCGGTTTACAAGGTACGGAACTCGTTATTTCTGATGCGCACAAAGGATTAG TCTCTGCCATTAGAAAATCCTTCACCAACGTAAGTTGGCAAAGATGCCAAGTTCACTTCCTAAGAAAT ATCTTTACCACCATTCCTAAAAAAAATTCAAAATCTTTCAGAGAAGCTGTTAAAGGAATTTTTAAGTT CACAGATATTAACTTAGCGCGTGAGGCTAAAAATCGATTGATTCATGATTATATCGATCAACCAAAAT ATTCAAAAGCTTGCGCATCATTGGATGATGGATTCGAAGACGCCTTTCAATATACCGTACAAGGAAAT TCCCACAATCGACTAAAGAGTACCAATCTAATTGAACGACTGAATCAAGAAGTACGCAGAAGAGAAAA GATTATTCGCATCTTCCCCAATCAAACATCAGCCAATCGCTTAATTGGAGCCGTTCTTATGGACCTAC ATGATGAATGGATTTATTCTTCAAGAAAATACATCAATTTTGATAAGTAGAAATGGTAAAAACATTGT ATAGCATTTTACACAGGAGTCTGGACTTGACT |
| 2 0 5 | IS 62 1 | CTGATGACAAACGCAACATTGCCTGATGCGCTACGCTTATCAGGCCTTGTATTATCCCTCCAGTGCAG AGAAAATCGGCCAGTTTTCTCTGCCTGCAGTCCGCATGCCGTATCGGGCCTTGGGTTCTAACCTGTTG CGTAGATTTATGCAGCGGACTGCCTTTCTCCCAAAGTGATAAACCGGACAGTATCATGGACCGGTTTT CCCGGTAATCCGTATTTGCAAGGTTGGTTTCACTATGGAACATGAACTTCATTATATCGGTATCGACA CCGCTAAAGAGAAACTGGATGTCGATGTGTTGCGTCCTGATGGTCGTCATCGCACCAAAAAATTCGCT AACACCACTAAAGGGCACGATGAGCTGGTGAGCTGGCTGAAAGGTCACAAGATTGACCATGCGCATAT CTGCATCGAAGCGACCGGCACCTATATGGAACCTGTCGCTGAGTGCCTTTACGATGCTGGCTACATAG TGTCAGTCATTAATCCTGCGCTGGGTAAAGCTTTCGCTCAGAGTGAAGGACTGCGTAACAAGACTGAT |
| | | ACCGTGGATGCGCGCATGCTGGCAGAGTTCTGTCGTCAGAAGCGCCCTGCAGCCTGGGAAGCGCCTCA CCCGCTTGAACGCGCGTTGCGTGCCCTGGTAGTCCGCCACCAGGCGCTGACAGATATGCACACGCAGG AACTGAATCGCACTGAAACGGCGCGGGAAGTCCAGAGACCGAGCATTGATGCTCACCTTCTGTGGCTT GAAGCAGAGCTGAAGCGTCTTGAGAAGCAGATAAAAGACCTGACAGACGATGATCCGGATATGAAACA CCGCAGGAAACTGCTGGAAAGCATCCCGGGTATCGGAGAGAAAACATCTGCGGTATTGCTGGCTTATA TCGGTCTGAAGGACCGCTTCGCCCATGCCAGACAGTTCGCCGCTTTTGCGGGTCTGACACCACGGCGT TATGAATCAGGTAGCAGTGTGAGAGGGGCGAGCCGGATGAGTAAGGCCGGACATGTGTCGCTTCGCAG GGCGTTGTATATGCCCGCAATGGTAGCCACCAGTAAGACTGAGTGGGACGGGCGTTCCGCGACCGTC TGGCGGCTAATGGCAAGAAAGGAAAGGTGATTCTCGGCGCGATGATGCGCAAGCTGGCACAGGTGGCG TATGGAGTGCTGAAGTCAGGCGTGCCGTTCGATGCGTCACGGCATAATCCGGTAGCGGCGTAAAAATC GCGGAAGGGATGAAAAAAACAGCGCCTGACGGCGCTGTGTCTGGCATGCCTGCAATCCGGGAAACCGG ACCAGGAAAAAACTTGCAGGCCATAACAGTATCTACGCGTCCCCTGCAATATTTTGAATTTGCACGAT TTTGTAGGCCGGATAAGGCGCTCGTGCCGCATCCGGCATAAACAAAGCGCACTTTGCCAGAAATC |
| 2 0 6 | IS 63 0 | AATAGCTGCGCGGAATAGTAGATCACTTTGAGGGAACTTAGCCCGGATTGTGCGATCTGATCAATCGC CAAATCAAAACAAATCACCAACCGGACTGAGCAATGCCGATCATAGCACCAATTTCCCGTGACGAACG ACGCCTGATGCAGAAAGCCATCCATAAAACACACGATAAAAATTATGCCCGCAGACTGACTGCCATGC TGATGCTGCACCGGGGCGACCGTGTCAGCGACGTTGCCAGAACGCTCTGCTGCGCCCGTTCCTCTGTT GGACGCTGGATTAACTGGTTCACGCAGTCGGGTGTTGAGGGACTGAAATCATTACCTGCCGGGCGTGC CCGTCGCTGGCCGTTTGAGCATATCTGCACACTGTTACGTGAGCTGGTAAAACATTCTCCCGGCGACT TTGGCTACCAGCGTTCACGCTGGAGTACAGAACTGCTGGCAATAAAAATCAATGAGATAACCGGTTGC CAGTTAAATGCCGGAACCGTTCGCCGCTGGTTGCCGTCTGCGGGGATTGTGTGGCGAAGGGCTGCGCC AACTCTGCGTATCCGTGACCCGCATAAAGATGAAAAGATGGCAGCAATCCATAAAGCACTGGACGAAT GCAGCGCAGAGCATCCGGTCTTTTATGAAGATGAAGTGGATATCCATCTTAATCCCAAAATCGGTGCG GACTGGCAACTGCGCGGACAGCAAAAACGGGTGGTCACGCCGGGACAGAATGAAAAATATTATCTGGC CGGAGCGCTGCACAGCGGACAGGTAAAGTCAGCTGTGTGGGCGGCAACAGCAAAAGTTCGGCGCTGT TCATCAGCCTGCTGAAGCGGCTTAAAGCGACATACCGTCGGGCGAAAACCATCACGCTGATCGTGGAC AACTACATTATCCACAAAAGCCGGGAAACACAGAGCTGGCTGAAGGAGAACCCGAAGTTCAGGGTCAT TTATCAGCCGGTTTACTCGCCATGGGTGAATCATGTTGAACGGCTATGGCAGGCACTTCACGACACAA TAACGCGTAATCATCAGTGCAGCTCAATGTGGCAACTGTTGAAAAAAGTTCGCCATTTTATGGAAACC GTCAGCCCATTCCCCGGAGGCAAACATGGGCTGGCAAAAGTGTAGCGGTATTAAGCGCAGCTATT |

## Claims

1. A modified bacterial strain lacking an insertion sequence (IS), wherein the modified bacterial strain (i) includes a specific deletion of an IS present in an unmodified bacterial strain, and (ii) retains non-IS sequences flanking the IS in the unmodified bacterial strain.

2. A modified bacterial strain according to claim 1, wherein the bacterial strain is an *Escherichia coli* (*E. coli*) strain.

3. A modified bacterial strain according to claim 1 or claim 2, wherein the modified bacterial strain includes specific deletions of at least 2, 3, 5, 10, 15, 20, 30, 40 or 45 IS present in the unmodified bacterial strain, preferably wherein the modified bacterial strain includes a specific deletion of all IS present in the unmodified bacterial strain, more preferably wherein the modified bacterial strain includes a specific deletion of all IS present in the unmodified bacterial strain and retains a genome size greater than 98% as compared to the genome size of the unmodified bacterial strain.

4. A modified bacterial strain according to any preceding claim, wherein the modified bacterial strain includes a specific deletion of IS nucleotide residues between and including two inverted repeats each defining an end of the IS, wherein the IS nucleotide residues do not include non-IS bacterial genome sequence.

5. A modified bacterial strain according to any preceding claim, wherein the modified bacterial strain further includes a specific deletion of a target site duplication sequence present in the unmodified bacterial strain adjacent to the IS.

6. A modified bacterial strain according to any preceding claim, wherein the modified bacterial strain retains at least 3, 10, 20, 30, 50, 100 or 500 non-IS nucleotide residues that immediately flank either end of the IS in the unmodified bacterial strain, preferably wherein the modified bacterial strain retains all non-IS sequences present in the unmodified bacterial strain.

7. A modified bacterial strain according to any preceding claim, wherein the modified bacterial strain lacks additional exogenous sequence elements compared to the unmodified bacterial strain.

8. A modified bacterial strain according to any of claims 1 to 6, wherein the modified bacterial strain comprises a stop codon and/or a frame-shift mutation between the retained non-IS flanking sequences, preferably wherein the stop codon and/or frame-shift mutation is in a *wbbL* gene, more preferably wherein the stop codon and/or frameshift mutation is in a *wbbL* gene at a position corresponding to an IS present in the unmodified bacterial strain, even more preferably wherein the stop codon and/or a frame-shift mutation is in a *wbbL* gene at a position corresponding to an IS5I present in the unmodified bacterial strain.

9. A modified bacterial strain according to any preceding claim, wherein the IS is a member of one of the following families or subfamilies: insL-2, IS1, IS2, IS3, IS4, IS5, IS6, IS629, IS50, IS21, IS200, IS256, IS621, IS630, IS257, IS15, IS26, IS10, IS30, IS150, IS186, IS600, IS609, IS911, ISX and/or ISZ, and/or a homologue thereof, preferably wherein the IS has at least 95% identity to: IS1A (SEQ ID NO: 2), IS1B (SEQ ID NO: 3), IS1C (SEQ ID NO:4), IS1D (SEQ ID NOs: 5 or 47), IS1E (SEQ ID NOs: 6 or48), IS1F (SEQ ID NO: 7), IS1H (SEQ ID NOs: 8 or 49), IS5A (SEQ ID NO: 9), IS5B (SEQ ID NOs: 10 or 50), IS5D (SEQ ID NOs: 11 or 51), IS5F (SEQ ID NOs: 12 or 52), IS5H (SEQ ID NOs: 13 or 53), IS5I (SEQ ID NOs: 14 or 54), IS5K (SEQ ID NOs: 15 or 55), IS5LO (SEQ ID NO: 16), IS5R (SEQ ID NOs: 17 or 56), IS5T (SEQ ID NOs: 18 or 57), IS5Y (SEQ ID NOs: 19 or 58), IS4 (SEQ ID NOs: 20 or 62), IS2A (SEQ ID NOs: 21 or 65), IS2D (SEQ ID NOs: 22 or 67), IS2E (SEQ ID NO: 23), IS2F (SEQ ID NOs: 24 or 69), IS2H (SEQ ID NO: 25), IS2I (SEQ ID NOs: 26 or 70), IS2K (SEQ ID NOs: 27 or 71), IS3A (SEQ ID NO: 28), IS3B (SEQ ID NOs: 29 or 66), IS3C (SEQ ID NO: 30), IS3D (SEQ ID NO: 31), IS3E (SEQ ID NOs: 32 or 68), IS30A (SEQ ID NO: 33), IS30B (SEQ ID NO: 34), IS30C (SEQ ID NOs: 35 or 63), IS30D (SEQ ID NO: 36), IS150 (SEQ ID NO: 37), IS186A (SEQ ID NOs: 38 or 59), IS186B (SEQ ID NOs: 39 or 60), IS186C (SEQ ID NOs: 40 or 61), IS600 (SEQ ID NO: 41), IS609 (SEQ ID NOs: 42 or 64), IS911A (SEQ ID NO: 43), IS911B (SEQ ID NO: 44), ISX (SEQ ID NO: 45), ISZ (SEQ ID NO: 46), IS629 (SEQ ID NO: 161), IS10 (SEQ ID NO: 162), IS50 (SEQ ID NO: 163), IS257 (SEQ ID NO: 164), IS15 (SEQ ID NO: 165), IS26 (SEQ ID NO: 166), IS21 (SEQ ID NO: 202), IS200 (SEQ ID NO: 203), IS256 (SEQ ID NO: 204), IS621 (SEQ ID NO: 205), IS630 (SEQ ID NO: 206), and/or insL-2 (SEQ ID NOs: 1 or 218), and/or a homologue thereof.

10. A method for producing a modified bacterial strain lacking an insertion sequence (IS) according to any preceding claim, the method comprising:
(a) Providing a bacterial strain comprising an IS flanked by non-IS sequences; and
(b) Specifically deleting the IS in the bacterial strain without deleting non-IS sequences flanking the IS.

11. A method according to claim 10, wherein (b) is repeated to delete more than one IS, preferably wherein (b) is repeated to delete at least 2, 3, 5, 10, 15, 20, 30, 40 or 45 IS present in the bacterial strain (a), more preferably wherein (b) is repeated to delete all IS present in the bacterial strain (a).

12. A method according to claims 10 or 11, wherein the deletion of the IS in (b) comprises transforming the bacterial strain with a first and second insertion cassette, wherein the first insertion cassette comprises:
i. A polynucleotide sequence having at least 25 nucleotide residues, wherein the polynucleotide sequence is identical to the flanking non-IS sequences 5' to the IS in the bacterial strain;
ii. A polynucleotide sequence having 5-35 nucleotide residues, wherein the polynucleotide sequence is identical to the flanking non-IS sequences 3' to the IS in the bacterial strain; and
iii. A truncated polynucleotide sequence encoding part of a selection marker; and
the second insertion cassette comprises:
iv. A truncated polynucleotide sequence encoding a part of the same selection marker (iii);
v. A polynucleotide sequence having 5-35 nucleotide residues, wherein the polynucleotide sequence is identical to the flanking non-IS sequences 5' to the IS in the bacterial strain; and
vi. A polynucleotide sequence having at least 25 nucleotide residues, wherein the polynucleotide sequence is identical to the flanking non-IS sequences positioned 3' to the IS in the bacterial strain;
wherein the truncated polynucleotide sequences (iii) and (iv) each comprise at least 50 identical consecutive nucleotide residues, and
wherein the truncated polynucleotide sequences (iii) and (iv) are configured such that, when the first and second insertion cassette undergo recombination, the selection marker is expressed.

13. A method according to any of claims 10 to 12, wherein the first and/or second cassette further comprise a stop codon and/or frame-shift mutation, such that the deleted IS is replaced by a stop codon and/or frame-shift mutation, preferably wherein the first and/or second cassette further comprise SEQ ID NOs: 72 or 73.

14. A method according to claim 13, wherein the stop codon and/or frame-shift mutation replaces an IS positioned in a *wbbL* gene, preferably wherein the IS is IS5I (SEQ ID NO: 14) or a homologue thereof.

15. A method according to claim 13 or 14, wherein the method further comprises:
(c) modifying one or more genes of interest in the bacterial strain; and
(d) deleting the stop codon and/or frame-shift mutation.

16. A method according to any of claims 10 to 15, wherein the IS is a member of one of the following IS families or subfamilies: insL-2, IS1, IS2, IS3, IS4, IS5, IS6, IS629, IS50, IS21, IS200, IS256, IS621, IS630, IS257, IS15, IS26, IS10, IS30, IS150, IS186, IS600, IS609, IS911, ISX and/or ISZ, and/or a homologue thereof, preferably wherein the IS has at least 95% identity to: IS1A (SEQ ID NO: 2), IS1B (SEQ ID NO: 3), IS1C (SEQ ID NO:4), IS1D (SEQ ID NO: 5), IS1E (SEQ ID NO: 6), IS1F (SEQ ID NO: 7), IS1H (SEQ ID NO: 8), IS5A (SEQ ID NO: 9), IS5B (SEQ ID NO: 10), IS5D (SEQ ID NO: 11), IS5F (SEQ ID NO: 12), IS5H (SEQ ID NO: 13), IS5I (SEQ ID NO: 14), IS5K (SEQ ID NO: 15), IS5LO (SEQ ID NO: 16), IS5R (SEQ ID NO: 17), IS5T (SEQ ID NO: 18), IS5Y (SEQ ID NO: 19), IS4 (SEQ ID NO: 20), IS2A (SEQ ID NO: 21), IS2D (SEQ ID NO: 22), IS2E (SEQ ID NO: 23), IS2F (SEQ ID NO: 24), IS2H (SEQ ID NO: 25), IS2I (SEQ ID NO: 26), IS2K (SEQ ID NO: 27), IS3A (SEQ ID NO: 28), IS3B (SEQ ID NO: 29), IS3C (SEQ ID NO: 30), IS3D (SEQ ID NO: 31), IS3E (SEQ ID NO: 32), IS30A (SEQ ID NO: 33), IS30B (SEQ ID NO: 34), IS30C (SEQ ID NO: 35), IS30D (SEQ ID NO: 36), IS150 (SEQ ID NO: 37), IS186A (SEQ ID NO: 38), IS186B (SEQ ID NO: 39), IS186C (SEQ ID NO: 40), IS600 (SEQ ID NO: 41), IS609 (SEQ ID NO: 42), IS911A (SEQ ID NO: 43), IS911B (SEQ ID NO: 44), ISX (SEQ ID NO: 45), ISZ (SEQ ID NO: 46), IS629 (SEQ ID NO: 161), IS10 (SEQ ID NO: 162), IS50 (SEQ ID NO: 163), IS257 (SEQ ID NO: 164), IS15 (SEQ ID NO: 165), IS26 (SEQ ID NO: 166), IS21 (SEQ ID NO: 202), IS200 (SEQ ID NO: 203), IS256 (SEQ ID NO: 204), IS621 (SEQ ID NO: 205), IS630 (SEQ ID NO: 206), and/or insL-2 (SEQ ID NO: 1), and/or a homologue thereof.

17. A method according to any of claims 10 to 16, wherein the IS is an IS present in a promoter of *Ion* protease in the bacterial strain (a), an IS present in a coding sequence of *Ion* protease and/or an IS present in a *wbbL* gene in the bacterial strain (a).
